# EUROPEAN PATENT APPLICATION

(11) **EP 2 604 611 A1**
(43) Date of publication of application: **19.06.2013**
(21) Application number: 12194563.8
(22) Date of filing: 20.11.2008
(51) Int. Cl.: C07D 487/04, A61K 31/407, A61P 25/00

(54) **N-Biaryl substituted diazabicycloalkane derivatives as agonists of the alpha7 and alpha4beta2 nACh Receptor**

(30) Priority: 21.11.2007 US 989607 P
(62) Divisional of application: 08853111.6
(71) Applicant: AbbVie Inc., North Chicago, IL 60064 (US)
(72) Inventor: JI, Jianguo, Libertyville, IL Illinois 60048 (US); LI, Tao, Grayslake, IL Illinois 60030 (US); Sippy, Kevin B, Antioch, IL Illinois 60002 (US); Lee, Chih-hung, Vernon Hills, IL Illinois 60061 (US); Gopalakrishnan, Murali, Libertyville, IL Illinois 60048 (US)
(74) Representative: Modiano, Micaela Nadia

(57) **Abstract**

The invention relates to biaryl substituted diazabicycloalkanes of formula (I), and more particularly bicycloheteroaryl substituted fused diazabicycloalkane derivatives, compositions comprising such compounds, and such compounds for use for treating or preventing conditions and disorders related to both α7 and α4β2 nAChR activity Formula: (I).

## Description

### CROSS-REFERENCE TO RELATED APPLICATIONS

This application claims priority to provisional application Serial No. 60/989,607, filed on November 21, 2007.

### FIELD OF THE INVENTION

The invention relates to biaryl substituted diazabicycloalkanes, and more particularly to bicycloheteroaryl substituted fused diazabicycloalkane derivatives, compositions comprising such compounds, methods of preventing or treating conditions and disorders using such compounds and compositions, process for preparing such compounds, and intermediates obtained during preparation of the compounds.

### DESCRIPTION OF RELATED TECHNOLOGY

Nicotinic acetylcholine receptors (nAChRs) are widely distributed throughout the central (CNS) and peripheral (PNS) nervous systems. Such receptors play an important role in regulating CNS function, particularly by modulating release of a wide range of neurotransmitters, such as acetylcholine, norepinephrine, dopamine, serotonin and GABA. Consequently, nicotinic receptors mediate a very wide range of physiological effects and have been targeted for therapeutic treatment of disorders relating to cognitive function, learning and memory, neurodegeneration, pain and inflammation, psychosis and sensory gating, mood and emotion, among others.

The plant alkaloid nicotine interacts with all subtypes of the nAChRs. While nicotine has been demonstrated to have many biological activities, not all of the effects mediated by nicotine are desirable. For example, nicotine exerts gastrointestinal and cardiovascular side effects at therapeutic doses, and it is addictive and acutelyoxic. Ligands that are selective for interacting with only certain subtypes of the nAChRs offer potential for achieving beneficial therapeutic effects with an improved margin of safety.

Many subtypes of the nAChR have been observed in the CNS and periphery. Each subtype has a different effect on regulating overall physiological function. Typically, nAChRs are ion channels that are constructed from pentamers. At least 12 subunit proteins, α2-α10 and β2-β4, have been identified in neuronal tissue. These subunits provide for a great variety of homomeric and heteromeric combinations that account for the diverse receptor subtypes. For example, the predominant receptor that is responsible for high affinity binding of nicotine in brain tissue is (α4)₂(β2)₃ (the α4β2 subtype), while another major population of receptors is the homopentamer (α7)₅ (the α7 subtype).

### The α7 and α4β2 nAChRs: Receptors with many roles

The α7 and α4β2 nAChRs play roles in multifarious processes, including cognitive function, protection against neuron degeneration, pain relief and schizophrenia; as well as other functions that appear less related to neuronal activity, such as angiogenesis and the sperm acrosome reaction during egg fertilization.

Alpha-7 nAChRs are implicated in aspects of neurodevelopment, for example neurogenesis of the brain. (Falk, L, et al., Developmental Brain Research 142:151-160, 2003; Tsuneki, H., et al., J. Physiol. (London) 547:169-179, 2003; Adams, C.E., et al., Developmental Brain Research 139:175-187, 2002). As such, modulating α7 nAChRs can be useful in preventing or treating conditions or disorders associated with impaired neurodevelopment, for example schizophrenia. (Sawa A., Mol. Med. 9:3-9, 2003).

The α7 and α4β2 nAChRs play significant roles in enhancing cognitive function, including aspects of learning, memory and attention (Levin, E.D., J. Neurobiol. 53: 633-640, 2002). For example, α7 nAChRs arc linked to conditions and disorders related to attention deficit disorder (ADD), attention deficit hyperactivity disorder (ADHD), Alzheimer's disease (AD), mild cognitive impairment, senile dementia, dementia associated with Lewy bodies, dementia associated with Down's syndrome, AIDS dementia, Pick's Disease, as well as cognitive deficits associated with schizophrenia, among other systemic activities. The α4β2 receptor subtype is implicated in attention, cognition, schizophrenia, epilepsy, and pain control (Paterson and Norberg, Progress in Neurobiology 61 75-111, 2000).

In addition to their roles in enhancing cognitive function, α7-containing nAChRs are involved in the neuroprotective effects of nicotine both *in vitro* (Jonnala, R. B. and Buccafusco, J. J., J. Neurosci. Res. 66: 565-572, 2001) and *in vivo* (Shimohama, S. et al., Brain Res. 779: 359-363, 1998). More particularly, neurodegeneration underlies several progressive CNS disorders, such as Alzheimer's disease, Parkinson's disease, amyotrophic lateral sclerosis, Huntington's disease, dementia with Lewy bodies, as well as diminished CNS function resulting from traumatic brain injury. For example, the impaired function of α7 nAChRs by β-amyloid peptides linked to Alzheimer's disease is implicated as a key factor in development of the cognitive deficits associated with the disease (Liu, Q.-S., Kawai, H., Berg, D. K., PNAS 98: 4734-4739, 2001). The activation of α7 nAChRs can block this neurotoxicity (Kihara, T. et al., J. Biol. Chem. 276: 13541-13546, 2001). As such, selective ligands that enhance α7 activity can counter the deficits of Alzheimer's and other neurodegenerative diseases.

Schizophrenia is a complex disease that is characterized by abnormalities in perception, cognition, and emotions. Significant evidence implicates the involvement of α7 nAChRs in this disease, including a measured deficit of these receptors in post-mortem patients (Leonard, S. Eur. J. Pharmacol. 393: 237-242, 2000). Deficits in sensory processing (gating) are one of the hallmarks of schizophrenia. These deficits can be normalized by nicotinic ligands that operate at the α7 nAChR (Adler L. E. et al., Schizophrenia Bull. 24: 189-202, 1998; Stevens, K. E. et al., Psychopharmacology 136: 320-327, 1998).

Cognitive impairment associated with schizophrenia often limits the ability of patients to function normally, a symptom not adequately treated by commonly available treatments, for example, treatment with an atypical antipsychotic. (Rowley, M. et al., J. Med. Chem. 44: 477-501, 2001). Such cognitive deficit is linked to dysfunction of the nicotinic cholinergic system, in particular with decreased activity receptors. (Friedman, J. I. et al., Biol Psychiatry, 51: 349-357, 2002).

Angiogenesis, a process involved in the growth of new blood vessels, is important in beneficial systemic functions, such as wound healing, vascularization of skin grafts, and enhancement of circulation, for example, increased circulation around a vascular occlusion. Non-selective nAChR agonists like nicotine can stimulate angiogenesis (Heeschen, C. et al., Nature Medicine 7: 833-839, 2001). Improved angiogenesis involves α7 nAChR activation (Heeschen, C. et al., J. Clin. Invest. 110: 527-536, 2002).

A population of α7 nAChRs in the spinal cord modulate serotonergic transmission that are associated with the pain-relieving effects of nicotinic compounds (Cordero-Erausquin, M. and Changeux, J.-P. PNAS 98:2803-2807, 2001). The α7 nAChR ligands are therapeutic targets for the treatment of pain states, including acute pain and post-surgical pain; as well as chronic pain states, including inflammatory pain and neuropathic pain. Moreover, α7 nAChRs are expressed on the surface of primary macrophages that are involved in the inflammation response. Activation of the α7 receptor inhibits TNF release and other cytokines that trigger the inflammation response (Wang, H. et al., Nature 421: 384-388, 2003). TNF-mediated diseases include, for example, rheumatoid arthritis, Crohn's disease, ulcerative colitis, inflammatory bowel disease, organ transplant rejection, acute immune disease associated with organ transplantation, chronic immune disease associated with organ transplantation, septic shock, toxic shock syndrome, sepsis syndrome, depression, and rheumatoid spondylitis.

The mammalian sperm acrosome reaction is an exocytosis process important in fertilization of the ovum by sperm. Activation of an α7 nAChR on sperm is essential for the acrosome reaction (Son, J.-H. and Meizel, S. Biol. Reproduct. 68: 1348-1353 2003).

The activity at both α7 and α4β2 nAChRs can be modified or regulated by the administration of subtype-selective nAChR ligands. The ligands can exhibit antagonist, agonist, or partial agonist properties. Compounds that function as positive allosteric modulators are also known.

In fact, several compounds with high affinity for α4β2 NNRs have been shown to improve attentive and cognitive performance in preclinical models that are relevant to attention-deficit/hyperactivity disorder (ADHD), a disease characterized by core symptoms of hyperactivity, inattentiveness, and impulsivity. For example, ABT-418, a full agonist at α4β2 NNRs, is efficacious in a variety of preclinical cognition models. ABT-418 administered transdermally, was shown in a controlled clinical trial in 32 adults to be effective in treating ADHD in general, and attentional/cognitive deficits in particular (Wilens TE, Biederman J, Spencer TJ, Bostic J, Prince J, Monuteaux MC, Soriano J, Fine C, Abrams A, Rater M, Polisner D. 1999. A pilot controlled clinical trial of ABT-418, a cholinergic agonist, in the treatment of adults with attention deficit hyperactivity disorder. Am J Psychiatry. 1999 Dec;156(12):1931-7). Likewise, ABT-418 showed a signal of efficacy in a pilot Alzheimer's disease trial. ABT-089, a α4β2 selective partial agonist, has been shown in rodent and primate animal models to improve attention, learning, and memory deficits. ABT-089 and another α4β2 agonist, ispronicline has shown efficacy in a pilot clinical trials. In addition to cognition, compounds that interact with α4β2 nAChRs such as ABT-594 and others are also efficacious in preclinical and clinical models of pain. As such, ligands that modulate both α7 and α4β2 activity can have broader spectrum of therapeutic efficacy in disease states such as those involving cognitive and attentive deficits, pain, neurodegenerative diseases and others.

Although compounds, such as nicotine, that nonselectively modulate nicotinic receptor subtypes including the α4β2 and α7 nAChRs are known, compounds that interact selectively with the α7-containing neuronal nAChRs, α4β2 nAChRs, or both α7 and α4β2 nAChRs arc desirable because of these receptors' many roles in pain, cognition, disorders and diseases.

### SUMMARY OF THE INVENTION

The invention is directed to biaryl substituted diazabicycloalkancs, compositions comprising such compounds, processes for preparing such compounds, and intermediates obtained during such processes. More particularly, the invention relates particularly to bicycloheteroaryl substituted fused diazabicycloalkane compounds and related methods of use and processes thereof.

One aspect of the invention relates to a compound of formula (1) 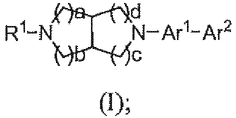 or a pharmaceutically acceptable salt or prodrug thereof, wherein
R¹ is selected from group consisting of hydrogen, alkyl, cyclic alkyl, haloalkyl, aryl, heteroaryl;
a and c are each independently selected from 0, 1, or 2; b and d are each independently selected from 1, 2, or 3, provided that when both b and d are 1, a and c cannot be 1 simultaneously;
Ar¹ is from a 5- or 6-membered aromatic group of formula 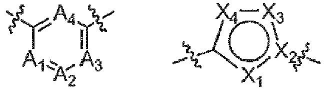 A₁, A₂, A₃ and A₄ are each independently -N-, or -CR^{a};
X₁, X₃, X₄ are independently selected from group consisting of -CR^{a},-NR^{a}, -O-, and -S-;
X₂ is -C- or -N-, provided that when X₂ is -C-, at least one of X₁, X₃, and X₄ is other than -C-;
R^{a} is selected from group consisting of hydrogen, or alkyl, cyclic alkyl, haloalkyl, alkyl, heteroaryl, halogen, -CO₂R¹, -COR¹, -CONR¹, -OR¹, and -NR¹, wherein R¹ is selected from group consisting of hydrogen, alkyl, cyclic alkyl, haloalkyl, aryl, and heteroaryl;
Ar² is selected from a fused bicyclic aromatic group of formula 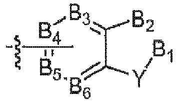 B₁, B₂, B₃, B₄, B₅, B₆ are each independently -N-, or -CR^{a}-;
Y is selected from group the consisting of -NR^{d}-, -O-, and -S-;
R^{a} is selected from the group consisting of hydrogen, alkyl, cyclic alkyl, haloalkyl, aryl, heteroaryl, halogen, -CO₂R¹, -COR¹, -CONR¹, -OR¹, and -NR¹; and
R^{d} is selected from group consisting of hydrogen, alkyl, and cyclic alkyl.

Another aspect of the invention relates to pharmaceutical compositions comprising compounds of the invention. Such compositions can be administered in accordance with a method of the invention, typically as part of a therapeutic regimen for treatment or prevention of conditions and disorders related to nAChR activity, and more particularly α7 nAChR activity, α4β2 nAChR activity, or both α7 nAChR activity and α4β2 nAChR activity.

Yet another aspect of the invention relates to a method of modulating both α7 and α4β2 nAChR activity. The method is useful for treating, preventing or both treating and preventing conditions and disorders related to both α7 and α4β2 nAChR activity, particularly in mammals.

A further aspect of the invention relates to a method of selectively modulating nAChR activity, for example α7 nAChR activity. The method is useful for treating, preventing or both treating and preventing conditions and disorders related to α7 nAChR activity in mammals. More particularly, the method is useful for conditions and disorders related to attention deficit disorder, attention deficit hyperactivity disorder (ADHD), Alzheimer's disease (AD), schizophrenia, mild cognitive impairment, age-associated memory impairment (AAMI), senile dementia, AIDS dementia, Pick's Disease, dementia associated with Lewy bodies, dementia associated with Down's syndrome, schizophrenia, amyotrophic lateral sclerosis, Huntington's disease, diminished CNS function associated with traumatic brain injury, acute pain, post-surgical pain, chronic pain, inflammatory pain, neuropathic pain, infertility, lack of circulation, need for new blood vessel growth associated with wound healing, more particularly circulation around a vascular occlusion, need for new blood vessel growth associated with vascularization of skin grafts, ischemia, inflammation, sepsis, wound healing, and other complications associated with diabetes, among other systemic and neuroimmunomodulatory activities.

A method of selectively modulating nAChR activity, for example a4p2 nAChR activity, also is contemplated.

The compounds, compositions comprising the compounds, methods for using the compounds, and processes for preparing the compounds, as well as intermediates obtained in such processes, are further described herein.

### DETAILED DESCRIPTION

### Definitions of Terms

As used throughout this specification and in the appended claims, the following terms have the following meanings:

The term "alkenyl" means a straight or branched chain hydrocarbon containing from 2 to 10 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl.

The term "alkenylene" means a divalent group derived from a straight or branched chain hydrocarbon of from 2 to 10 carbon atoms containing at least one double bond. Representative examples of alkenylene include, but are not limited to, -CH=CH-, - CH=CH₂CH₂-, and -CH=C(CH₃)CH₂-.

The term "alkenyloxy" means an alkenyl group, as defined herein, appended to the parent molecular moiety through an oxygen atom. Representative examples of alkenyloxy include, but are not limited to, allyloxy, 2-butenyloxy and 3-butenyloxy.

The term "alkoxy" means an alkyl group, as defined herein, appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

The term "alkoxyalkoxy" means an alkoxy group, as defined herein, appended to the parent molecular moiety through another alkoxy group, as defined herein. Representative examples of alkoxyalkoxy include, but are not limited to, tert-butoxymethoxy, 2-ethoxyethoxy, 2-methoxyethoxy, and methoxymethoxy.

The term "alkoxyalkoxyalkyl" means an alkoxyalkoxy group, as defined herein, appended to the parent molecular moiety through an alkyl group, as defined herein. Representative examples of alkoxyalkoxyalkyl include, but are not limited to, tert-butoxymethoxymethyl, ethoxymethoxymethyl, (2-methoxyethoxy)methyl, and 2-(2-methoxyethoxy)ethyl.

The term "alkoxyalkyl" means an alkoxy group, as defined herein, appended to the parent molecular moiety through an alkyl group, as defined herein. Representative examples of alkoxyalkyl include, but are not limited to, tert-butoxymethyl, 2-ethoxyethyl, 2-methoxyethyl, and methoxymethyl.

The term "alkoxycarbonyl" means an alkoxy group, as defined herein, appended to the parent molecular moiety through a carbonyl group, as defined herein. Representative examples of alkoxycarbonyl include, but are not limited to, methoxycarbonyl, ethoxycarbonyl, and tert-butoxycarbonyl.

The term "alkoxycarbonylalkyl" means an alkoxycarbonyl group, as defined herein, appended to the parent molecular moiety through an alkyl group, as defined herein. Representative examples of alkoxycarbonylalkyl include, but are not limited to, 3-methoxycarbonylpropyl, 4-ethoxycarbonylbutyl, and 2-tert-butoxycarbonylethyl.

The term "alkoxysulfonyl" means an alkoxy group, as defined herein, appended to the parent molecular moiety through a sulfonyl group, as defined herein. Representative examples of alkoxysulfonyl include, but are not limited to, methoxysulfonyl, ethoxysulfonyl and propoxysulfonyl.

The term "alkyl" means a straight or branched chain hydrocarbon containing from 1 to 10 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl.

The term "alkylcarbonyl" means an alkyl group, as defined herein, appended to the parent molecular moiety through a carbonyl group, as defined herein. Representative examples of alkylcarbonyl include, but are not limited to, acetyl, 1-oxopropyl, 2,2-dimethyl-1-oxopropyl, 1-oxobutyl, and 1-oxopentyl.

The term "alkylcarbonylalkyl" means an alkylcarbonyl group, as defined herein, appended to the parent molecular moiety through an alkyl group, as defined herein. Representative examples of alkylcarbonylalkyl include, but are not limited to, 2-oxopropyl, 3,3-dimethyl-2-oxopropyl, 3-oxobutyl, and 3-oxopentyl.

The term "alkylcarbonyloxy" means an alkylcarbonyl group, as defined herein, appended to the parent molecular moiety through an oxygen atom. Representative examples of alkylcarbonyloxy include, but are not limited to, acetyloxy, ethylcarbonyloxy, and tert-butylcarbonyloxy.

The term "alkylene" means a divalent group derived from a straight or branched chain hydrocarbon of from 1 to 10 carbon atoms. Representative examples of alkylene include, but are not limited to, -CH₂-, -CH(CH₃)-, -C(CH₃)₂-, -CH₂CH₂-, -CH₂CH₂CH₂-, -CH₂CH₂CH₂CH₂-, and -CH₂CH(CH₃)CH₂-.

The term "alkylsulfinyl" means an alkyl group, as defined herein, appended to the parent molecular moiety through a sulfinyl group, as defined herein. Representative examples of alkylsulfinyl include, but are not limited to, methylsulfinyl and ethylsulfinyl.

The term "alkylsulfinylalkyl" means an alkylsulfinyl group, as defined herein, appended to the parent molecular moiety through an alkyl group, as defined herein. Representative examples of alkylsulfinylalkyl include, but are not limited to, methylsulfinylmethyl and ethylsulfinylmethyl.

The term "alkylsulfonyl" means an alkyl group, as defined herein, appended to the parent molecular moiety through a sulfonyl group, as defined herein. Representative examples of alkylsulfonyl include, but are not limited to, methylsulfonyl and ethylsulfonyl.

The term "alkylsulfonylalkyl" means an alkylsulfonyl group, as defined herein, appended to the parent molecular moiety through an alkyl group, as defined herein. Representative examples of alkylsulfonylalkyl include, but are not limited to, methylsulfonylmethyl and ethylsulfonylmethyl.

The term "alkylthio" means an alkyl group, as defined herein, appended to the parent molecular moiety through a sulfur atom. Representative examples of alkylthio include, but are not limited, methylthio, ethylthio, tert-butylthio, and hexylthio.

The term "alkylthioalkyl" means an alkylthio group, as defined herein, appended to the parent molecular moiety through an alkyl group, as defined herein. Representative examples of alkylthioalkyl include, but are not limited, methylthiomethyl and 2-(ethylthio)ethyl.

The term "alkynyl" means a straight or branched chain hydrocarbon group containing from 2 to 10 carbon atoms and containing at least one carbon-carbon triple bond. Representative examples of alkynyl include, but are not limited, to acetylenyl, 1-propynyl, 2-propynyl, 3-butynyl, 2-pentynyl, and 1-butynyl.

The term "alkynylene" means a divalent group derived from a straight or branched chain hydrocarbon of from 2 to 10 carbon atoms containing at least one triple bond. Representative examples of alkynylene include, but are not limited to, -C≡C-, -CH₂C≡C-, - CH(CH₃)CH₂C≡C-, -C≡CCH₂-, and -C≡CCH(CH₃)CH₂-.

The term "alkynyloxy" means an alkynyl group, as defined herein, appended to the parent molecular moiety through an oxygen atom. Representative examples of alkynyloxy include, but are not limited to, 2-propynyloxy and 2-butynyloxy.

The term "aryl," means phenyl, a bicyclic aryl or a tricyclic aryl. The bicyclic aryl is naphthyl, a phenyl fused to a cycloalkyl, or a phenyl fused to a cycloalkenyl. Representative examples of the bicyclic aryl include, but are not limited to, dihydroindenyl, indenyl, naphthyl, dihydronaphthalenyl, and tetrahydronaphthalenyl. The tricyclic aryl is anthracene or phenanthrene, or a bicyclic aryl fused to a cycloalkyl, or a bicyclic aryl fused to a cycloalkenyl, or a bicyclic aryl fused to a phenyl. Representative examples of tricyclic aryl ring include, but are not limited to, azulenyl, dihydroanthracenyl, fluorenyl, and tetrahydrophenanthrenyl.

The aryl groups of this invention can be substituted with 1, 2, 3, 4 or 5 substituents independently selected from alkenyl, alkoxy, alkoxyalkoxy, alkoxyalkoxyalkyl, alkoxyalkyl, alkoxycarbonyl, alkoxycarbonylalkyl, alkyl, alkylcarbonyl, alkylcarbonylalkyl, alkylcarbonyloxy, alkylsulfinyl, alkylsulfinylalkyl, alkylsulfonyl, alkylsulfonylalkyl, alkylthio, alkylthioalkyl, alkynyl, carboxy, carboxyalkyl, cyano, cyanoalkyl, formyl, formylalkyl, halogen, haloalkyl, hydroxy, hydroxyalkyl, mercapto, nitro, -NZ₁Z₂, and (NZ₃Z₄)carbonyl.

The term "arylalkoxy" means an aryl group, as defined herein, appended to the parent molecular moiety through an alkoxy group, as defined herein. Representative examples of arylalkoxy include, but are not limited to, 2-phenylethoxy, 3-naphth-2-ylpropoxy, and 5-phenylpentyloxy.

The term "arylalkoxycarbonyl" means an arylalkoxy group, as defined herein, appended to the parent molecular moiety through a carbonyl group, as defined herein. Representative examples of arylalkoxycarbonyl include, but are not limited to, benzyloxycarbonyl and naphth-2-ylmethoxycarbonyl.

The term "arylalkyl" means an aryl group, as defined herein, appended to the parent molecular moiety through an alkyl group, as defined herein. Representative examples of arylalkyl include, but are not limited to, benzyl, 2-phenylethyl, 3-phenylpropyl, and 2-naphth-2-ylethyl.

The term "arylalkylthio" means an arylalkyl group, as defined herein, appended to the parent molecular moiety through a sulfur atom. Representative examples of arylalkylthio include, but are not limited to, 2-phenylethylthio, 3-naphth-2-ylpropylthio, and 5-phenylpentylthio.

The term "arylcarbonyl" means an aryl group, as defined herein, appended to the parent molecular moiety through a carbonyl group, as defined herein. Representative examples of arylcarbonyl include, but are not limited to, benzoyl and naphthoyl.

The term "aryloxy" means an aryl group, as defined herein, appended to the parent molecular moiety through an oxygen atom. Representative examples of aryloxy include, but are not limited to, phenoxy, naphthyloxy, 3-bromophenoxy, 4-chlorophenoxy, 4-mcthylphenoxy, and 3,5-dimethoxyphenoxy.

The term "aryloxyalkyl" means an aryloxy group, as defined herein, appended to the parent molecular moiety through an alkyl group, as defined herein. Representative examples of aryloxyalkyl include, but are not limited to, 2-phenoxyethyl, 3-naphth-2-yloxypropyl and 3-bromophenoxy methyl.

The term "arylthio" means an aryl group, as defined herein, appended to the parent molecular moiety through a sulfur atom. Representative examples of arylthio include, but are not limited to, phenylthio and 2-naphthylthio.

The term "arylthioalkyl" means an arylthio group, as defined herein, appended to the parent molecular moiety through an alkyl group, as defined herein. Representative examples of arylthioalkyl include, but are not limited to, phenylthiomethyl, 2-naphth-2-ylthioethyl, and 5-phenylthiomethyl.

The term "azido" means a -N₃ group.

The term "carbonyl' means a -C(O)- group.

The term "carboxy" means a -CO₂H group.

The term "carboxyalkyl" means a carboxy group, as defined herein, appended to the parent molecular moiety through an alkyl group, as defined herein. Representative examples of carboxyalkyl include, but are not limited to, carboxymethyl, 2-carboxyethyl, and 3-carboxypropyl.

The term "cyano" means a -CN group.

The term "cyanoalkyl" means a cyano group, as defined herein, appended to the parent molecular moiety through an alkyl group, as defined herein. Representative examples of cyanoalkyl include, but are not limited to, cyanomethyl, 2-cyanoethyl, and 3-cyanopropyl.

The term "cycloalkenyl" means a cyclic hydrocarbon containing from 3 to 8 carbons and containing at least one carbon-carbon double bond formed by the removal of two hydrogens. Representative examples of cycloalkenyl include, but are not limited to, 2-cyclohexen-1-yl, 3-cyclohexen-1-yl, 2,4-cyclohexadien-1-yl and 3-cyclopenten-1-yl.

The term "cycloalkyl" as used herein, means a monocyclic, bicyclic, or tricyclic ring system. Monocyclic ring systems are exemplified by a saturated cyclic hydrocarbon group containing from 3 to 8 carbon atoms. Examples of monocyclic ring systems include cyclopropyl, cyclobutyl, cyclopentyl, cyclohexyl, cycloheptyl, and cyclooctyl. Bicyclic ring systems are exemplified by a bridged monocyclic ring system in which two adjacent or non-adjacent carbon atoms of the monocyclic ring are linked by an alkylene bridge of between one and three additional carbon atoms. Representative examples of bicyclic ring systems include, but arc not limited to, bicyclo[3.2.0]heptane, bicyclo[3.3.0]octane, bicyclo[4.3.0]nonane, bicyclo[4.2.0]octane, bicyclo[5.2.0]nonane, bicyclo[5.3.0]decane and bicyclo[4.4.0]decane.

The cycloalkyl groups of the invention are optionally substituted with 1, 2, 3, 4 or 5 substituents selected from the group consisting of alkenyl, alkoxy, alkoxyalkoxy, alkoxyalkyl, alkoxycarbonyl, alkoxysulfonyl, alkyl, alkylcarbonyl, alkylcarbonyloxy, alkylsulfonyl, alkylthio, alkylthioalkyl, alkynyl, carboxy, cyano, formyl, haloalkoxy, haloalkyl, halogen, hydroxy, hydroxyalkyl, mercapto, oxo, -NZ₁Z₂, and (NZ₃Z₄)carbonyl.

The term "cycloalkylalkyl" means a cycloalkyl group, as defined herein, appended to the parent molecular moiety through an alkyl group, as defined herein. Representative examples of cycloalkylalkyl include, but are not limited to, cyclopropylmethyl, 2-cyclobutylethyl, cyclopentylmethyl, cyclohexylmethyl, and 4-cycloheptylbutyl.

The term "cycloalkylcarbonyl" means cycloalkyl group, as defined herein, appended to the parent molecular moiety through a carbonyl group, as defined herein. Representative examples of cycloalkylcarbonyl include, but are not limited to, cyclopropylcarbonyl, 2-cyclobutylcarbonyl, and cyclohexylcarbonyl.

The term "cycloalkyloxy" means cycloalkyl group, as defined herein, appended to the parent molecular moiety through an oxygen atom, as defined herein. Representative examples of cycloalkyloxy include, but are not limited to, cyclopropyloxy, cyclobutyloxy, cyclopentyloxy, cyclohexyloxy, cycloheptyloxy, and cyclooctyloxy.

The term "cycloalkylthio" means cycloalkyl group, as defined herein, appended to the parent molecular moiety through a sulfur atom, as defined herein. Representative examples of cycloalkylthio include, but are not limited to, cyclopropylthio, cyclobutylthio, cyclopentylthio, cyclohexylthio, cycloheptylthio, and cyclooctylthio.

The term "ethylenedioxy" means a -O(CH₂)₂O- group wherein the oxygen atoms of the ethylenedioxy group arc attached to the parent molecular moiety through one carbon atom forming a 5-membered ring or the oxygen atoms of the ethylenedioxy group are attached to the parent molecular moiety through two adjacent carbon atoms forming a 6-membered ring.

The term "formyl" means a -C(O)H group.

The term "formylalkyl" means a formyl group, as defined herein, appended to the parent molecular moiety through an alkyl group, as defined herein. Representative examples of formylalkyl include, but are not limited to, formylmethyl and 2-formylethyl.

The term "halo" or "halogen" means -Cl, -Br, -I or -F.

The term "haloalkoxy" means at least one halogen, as defined herein, appended to the parent molecular moiety through an alkoxy group, as defined herein. Representative examples of haloalkoxy include, but are not limited to, chloromethoxy, 2-fluoroethoxy, trifluoromethoxy, and pentafluoroethoxy.

The term "haloalkyl" means at least one halogen, as defined herein, appended to the parent molecular moiety through an alkyl group, as defined herein. Representative examples of haloalkyl include, but are not limited to, chloromethyl, 2-fluoroethyl, trifluoromethyl, pentafluoroethyl, and 2-chloro-3-fluoropentyl.

The term "heteroaryl," means a monocyclic heteroaryl or a bicyclic heteroaryl. The monocyclic heteroaryl is a 5- or 6-membered ring that contains at least one heteroatom selected from the group consisting of nitrogen, oxygen and sulfur. The 5-membered ring contains two double bonds and the 6-membered ring contains three double bonds. The 5- or 6-membered heteroaryl is connected to the parent molecular moiety through any carbon atom or any substitutable nitrogen atom contained within the heteroaryl, provided that proper valance is maintained. Representative examples of monocyclic heteroaryl include, but are not limited to, furyl, imidazolyl, isoxazolyl, isothiazolyl, oxadiazolyl, oxazolyl, pyridinyl, pyridazinyl, pyrimidinyl, pyrazinyl, pyrazolyl, pyrrolyl, tetrazolyl, thiadiazolyl, thiazolyl, thienyl, triazolyl, and triazinyl. The bicyclic heteroaryl consists of a monocyclic heteroaryl fused to a phenyl, or a monocyclic heteroaryl fused to a cycloalkyl, or a monocyclic heteroaryl fused to a cycloalkenyl, or a monocyclic heteroaryl fused to a monocyclic heteroaryl. The bicyclic heteroaryl is connected to the parent molecular moiety through any carbon atom or any substitutable nitrogen atom contained within the bicyclic heteroaryl, provided that proper valance is maintained. Representative examples of bicyclic heteroaryl include, but are not limited to, azaindolyl, benzimidazolyl, benzofuranyl, benzoxadiazolyl, benzoisoxazole, benzoisothiazole, benzooxazole, 1,3-benzothiazolyl, benzothiophenyl, cinnolinyl, furopyridine, indolyl, indazolyl, isobenzofuran, isoindolyl, isoquinolinyl, naphthyridinyl, oxazolopyridine, quinolinyl, quinoxalinyl and thienopyridinyl.

The heteroaryl groups of the invention are optionally substituted with 1, 2, 3 or 4 substituents independently selected from the group consisting of alkenyl, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylthio, alkylthioalkyl, alkynyl, carboxy, carboxyalkyl, cyano, cyanoalkyl, formyl, haloalkoxy, haloalkyl, halogen, hydroxy, hydroxyalkyl, mercapto, nitro, -NZ₁Z₂ and (NZ₃Z₄)carbonyl. Heteroaryl groups of the invention that are substituted with a hydroxyl group may be present as tautomers. The heteroaryl groups of the invention encompass all tautomers including non-aromatic tautomers.

The term "heteroarylalkoxy" means a heteroaryl group, as defined herein, appended to the parent molecular moiety through an alkoxy group, as defined herein. Representative examples of heteroarylalkoxy include, but are not limited to, fur-3-ylmethoxy, 1H-imidazol-2-ylmethoxy, 1H-imidazol-4-ylmethoxy, 1-(pyridin-4-yl)ethoxy, pyridin-3-ylmethoxy, 6-chloropyridin-3-ylmethoxy, pyridin-4-ylmethoxy, (6-(trifluoromethyl)pyridin-3-yl)methoxy, (6-(cyano)pyridin-3-yl)methoxy, (2-(cyano)pyridin-4-yl)methoxy, (5-(cyano)pyridin-2-yl)methoxy, (2-(chloro)pyridin-4-yl)methoxy, pyrimidin-5-ylmethoxy, 2-(pyrimidin-2-yl)propoxy, thien-2-ylmethoxy, and thien-3-ylmethoxy.

The term "heteroarylalkyl" means a heteroaryl, as defined herein, appended to the parent molecular moiety through an alkyl group, as defined herein. Representative examples of heteroarylalkyl include, but are not limited to, fur-3-ylmethyl, 1H-imidazol-2-ylmethyl, 1H-imidazol-4-ylmethyl, 1-(pyridin-4-yl)ethyl, pyridin-3-ylmethyl, 6-chloropyridin-3-ylmethyl, pyridin-4-ylmethyl, (6-(trifluoromethyl)pyridin-3-yl)methyl, (6-(cyano)pyridin-3-yl)methyl, (2-(cyano)pyridin-4-yl)methyl, (5-(cyano)pyridin-2-yl)methyl, (2-(chloro)pyridin-4-yl)methyl, pyrimidin-5-ylmethyl, 2-(pyrimidin-2-yl)propyl, thien-2-ylmethyl, and thien-3-ylmethyl.

The term "heteroarylalkylcarbonyl" means a heteroarylalkyl, as defined herein, appended to the parent molecular moiety through a carbonyl group, as defined herein.

The term "heteroarylalkylthio" means a heteroarylalkyl group, as defined herein, appended to the parent molecular moiety through a sulfur atom. Representative examples of heteroarylalkylthio include, but are not limited to, fur-3-ylmcthylthio, 1H-imidazol-2-ylmethylthio, 1H-imidazol-4-ylmethylthio, pyridin-3-ylmethylthio, 6-chloropyridin-3-ylmethylthio, pyridin-4-ylmethylthio, (6-(trifluoromethyl)pyridin-3-yl)methylthio, (6-(cyano)pyridin-3-yl)methylthio, (2-(cyano)pyridin-4-yl)methylthio, (5-(cyano)pyridin-2-yl)methylthio, (2-(chloro)pyridin-4-yl)methylthio, pyrimidin-5-ylmethylthio, 2-(pyrimidin-2-yl)propylthio, thien-2-ylmethylthio, and thien-3-ylmethylthio.

The term "heteroarylcarbonyl" means a heteroaryl group, as defined herein, appended to the parent molecular moiety through a carbonyl group, as defined herein. Representative examples of heteroarylcarbonyl include, but are not limited to, fur-3-ylcarbonyl, 1H-imidazol-2-ylcarbonyl, 1H-imidazol-4-ylcarbonyl, pyridin-3-ylcarbonyl, 6-chloropyridin-3-ylcarbonyl, pyridin-4-ylcarbonyl, (6-(trifluoromethyl)pyridin-3-yl)carbonyl, (6-(cyano)pyridin-3-yl)carbonyl, (2-(cyano)pyridin-4-yl)carbonyl, (5-(cyano)pyridin-2-yl)carbonyl, (2-(chloro)pyridin-4-yl)carbonyl, pyrimidin-5-ylcarbonyl, pyrimidin-2-ylcarbonyl, thien-2-ylcarbonyl, and thien-3-ylcarbonyl.

The term "heteroaryloxy" means a heteroaryl group, as defined herein, appended to the parent molecular moiety through an oxygen atom. Representative examples of heteroaryloxy include, but are not limited to, fur-3-yloxy, 1H-imidazol-2-yloxy, 1H-imidazol-4-yloxy, pyridin-3-yloxy, 6-chloropyridin-3-yloxy, pyridin-4-yloxy, (6-(trifluoromethyl)pyridin-3-yl) oxy, (6-(cyano)pyridin-3-yl) oxy, (2-(cyano)pyridin-4-yl)oxy, (5-(cyano)pyridin-2-yl)oxy, (2-(chloro)pyridin-4-yl)oxy, pyrimidin-5-yloxy, pyrimidin-2-yloxy, thien-2-yloxy, and thien-3-yloxy.

The term "heteroaryloxyalkyl" means a heteroaryloxy group, as defined herein, appended to the parent molecular moiety through an alkyl group, as defined herein. Representative examples of heteroaryloxyalkyl include, but are not limited to, pyridin-3-yloxymethyl and 2-quinolin-3-yloxyethyl.

The term "heteroarylthio" means a heteroaryl group, as defined herein, appended to the parent molecular moiety through a sulfur atom. Representative examples of heteroarylthio include, but are not limited to, pyridin-3-ylthio and quinolin-3-ylthio.

The term "heteroarylthioalkyl" means a heteroarylthio group, as defined herein, appended to the parent molecular moiety through an alkyl group, as defined herein. Representative examples of heteroarylthioalkyl include, but are not limited to, pyridin-3-ylthiomethyl, and 2-quinolin-3-ylthioethyl.

The term "heterocycle" or "heterocyclic" means a monocyclic heterocycle, a bicyclic heterocycle or a tricyclic heterocycle. The monocyclic heterocycle is a 3-, 4-, 5-, 6- or 7-membered ring containing at least one heteroatom independently selected from the group consisting of O, N, and S. The 3- or 4-membered ring contains 1 heteroatom selected from the group consisting of O, N and S. The 5-membered ring contains zero or one double bond and one, two or three heteroatoms selected from the group consisting of O, N and S. The 6-or 7-membered ring contains zero, one or two double bonds and one, two or three heteroatoms selected from the group consisting of O, N and S. The monocyclic heterocycle is connected to the parent molecular moiety through any carbon atom or any nitrogen atom contained within the monocyclic heterocycle. Representative examples of monocyclic heterocycle include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, piperazinyl, piperidinyl, pyranyl, pyrazolinyl, pyrazolidinyl, pyrrolinyl, pyrrolidinyl, tetrahydrofuranyl, tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, and trithianyl, The bicyclic heterocycle is a 5- or 6-membered monocyclic heterocycle fused to a phenyl group, or a 5- or 6-membered monocyclic heterocycle fused to a cycloalkyl, or a 5- or 6-membered monocyclic heterocycle fused to a cycloalkenyl, or a 5- or 6-membered monocyclic heterocycle fused to a monocyclic heterocycle. The bicyclic heterocycle is connected to the parent molecular moiety through any carbon atom or any nitrogen atom contained within the bicyclic heterocycle. Representative examples of bicyclic heterocycle include, but are not limited to, 1,3-benzodioxolyl, 1,3-benzodithiolyl, 2,3-dihydro-1,4-benzodioxnyl, benzodioxolyl, 2,3-dihydro-1-benzofuratiyl, 2,3-dihydro-1-benzothienyl, chromenyl and 1,2,3,4-tetrahydroquinolinyl.

The heterocycles of this invention are optionally substituted with 1, 2, 3 or 4 substituents independently selected from the group consisting of alkenyl, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylthio, alkylthioalkyl, alkynyl, carboxy, carboxyalkyl, cyano, cyanoalkyl, formyl, haloalkoxy, haloalkyl, halogen, hydroxy, hydroxyalkyl, mercapto, and oxo.

The term "heterocyclealkoxy" means a heterocycle group, as defined herein, appended to the parent molecular moiety through an alkoxy group, as defined herein. Representative examples of heterocyclealkoxy include, but are not limited to, 2-pyridin-3-ylethoxy, 3-quinolin-3-ylpropoxy, and 5-pyridin-4-ylpcntyloxy.

The term "heterocyclealkyl" means a heterocycle, as defined herein, appended to the parent molecular moiety through an alkyl group, as defined herein.

The term "heterocyclealkylcarbonyl" means a heterocyclealkyl, as defined herein, appended to the parent molecular moiety through a carbonyl group, as defined herein. Representative examples of heterocyclealkylcarbonyl include, but are not limited to, piperidin-4-ylmethylcarbonyl, piperazin-1-ylmethylcarbonyl, 3-methyl-1-pyrrolidin-1-ylbutylcarbonyl, (*1R*)-3-methyl-1-pyrrolidin-1-ylbutylcarbonyl, (*1S*)-3-methyl-1-pyrrolidin-1-ylbutylcarbonyl.

The term "heterocyclealkylthio" means a heterocyclealkyl group, as defined herein, appended to the parent molecular moiety through a sulfur atom. Representative examples of heterocyclealkylthio include, but are not limited to, 2-pyridin-3-ylethythio, 3-quinolin-3-ylpropythio, and 5-pyridin-4-ylpentylthio.

The term "heterocyclecarbonyl" means a heterocycle, as defined herein, appended to the parent molecular moiety through a carbonyl group, as defined herein.

The term "heterocyclecarbonylalkyl" means a heterocyclecarbonyl, as defined herein, appended to the parent molecular moiety through an alkyl group, as defined herein.

The term "heterocycleoxy" means a heterocycle group, as defined herein, appended to the parent molecular moiety through an oxygen atom. Representative examples of heterocycleoxy include, but are not limited to, pyridin-3-yloxy and quinolin-3-yloxy.

The term "heterocycleoxyalkyl" means a heterocycleoxy group, as defined herein, appended to the parent molecular moiety through an alkyl group, as defined herein. Representative examples of heterocycleoxyalkyl include, but are not limited to, pyridin-3-yloxymethyl and 2-quinolin-3-yloxyethyl.

The term "heterocyclethio" means a heterocycle group, as defined herein, appended to the parent molecular moiety through a sulfur atom. Representative examples of heterocyclethio include, but are not limited to, pyridin-3-ylthio and quinolin-3-ylthio.

The term "heterocyclethioalkyl" means a heterocyclethio group, as defined herein, appended to the parent molecular moiety through an alkyl group, as defined herein. Representative examples of heterocyclethioalkyl include, but are not limited to, pyridin-3-ylthiomethyl, and 2-quinolin-3-ylthioethyl.

The term "hydroxy" means an -OH group.

The term "hydroxyalkyl" means at least one hydroxy group, as defined herein, is appended to the parent molecular moiety through an alkyl group, as defined herein. Representative examples of hydroxyalkyl include, but are not limited to, hydroxymethyl, 2-hydroxyethyl, 3-hydroxypropyl, 2,3-dihydroxypentyl, and 2-ethyl-4-hydroxyheptyl.

The term "hydroxy-protecting group" or "O-protecting group" means a substituent which protects hydroxyl groups against undesirable reactions during synthetic procedures. Examples of hydroxy-protecting groups include, but are not limited to, substituted methyl ethers, for example, methoxymethyl, benzyloxymethyl, 2-methoxyethoxymethyl, 2-(trimethylsilyl)-ethoxymethyl, benzyl, and triphenylmethyl; tetrahydropyranyl ethers; substituted ethyl ethers, for example, 2,2,2-trichloroethyl and t-butyl; silyl ethers, for example, trimethylsilyl, t-butyldimethylsilyl and t-butyldiphenylsilyl; cyclic acetals and kctals, for example, methylene acetal, acetonide and benzylidene acetal; cyclic ortho esters, for example, methoxymethylene; cyclic carbonates; and cyclic boronates. Commonly used hydroxy-protecting groups are disclosed in T.W. Greene and P.G.M. Wuts, Protective Groups in Organic Synthesis, 3rd edition, John Wiley & Sons, New York (1999).

The term "lower alkenyl" is a subset of alkenyl, as defined herein, and means an alkenyl group containing from 2 to 4 carbon atoms. Examples of lower alkenyl are ethetiyl, propenyl, and butenyl.

The term " lower alkoxy" is a subset of alkoxy, as defined herein, and means a lower alkyl group, as defined herein, appended to the parent molecular moiety through an oxygen atom, as defined herein. Representative examples of lower alkoxy include, but are not limited to, methoxy, ethoxy, propoxy, 2-propoxy, butoxy, and tert-butoxy.

The term "lower alkyl" is a subset of alkyl as defined herein and means a straight or branched chain hydrocarbon group containing from 1 to 4 carbon atoms. Examples of lower alkyl are methyl, ethyl, n-propyl, iso-propyl, n-butyl, iso-butyl, sec-butyl, and tert-butyl.

The term "lower alkylthio" is a subset of alkylthio, means a lower alkyl group, as defined herein, appended to the parent molecular moiety through a sulfur atom. Representative examples of lower alkylthio include, but are not limited, methylthio, ethylthio, and tert-butylthio.

The term "lower alkynyl" is a subset of alkynyl, as defined herein, and means an alkynyl group containing from 2 to 4 carbon atoms. Examples of lower alkynyl are ethynyl, propynyl, and butynyl.

The term "lower haloalkoxy" is a subset of haloalkoxy, as defined herein, and means a straight or branched chain haloalkoxy group containing from 1 to 4 carbon atoms. Representative examples of lower haloalkoxy include, but are not limited to, trifluoromethoxy, trichloromethoxy, dichloromethoxy, fluoromethoxy, and pentafluoroethoxy.

The term "lower haloalkyl" is a subset of haloalkyl, as defined herein, and means a straight or branched chain haloalkyl group containing from 1 to 4 carbon atoms. Representative examples of lower haloalkyl include, but are not limited to, trifluoromethyl, trichloromethyl, dichloromethyl, fluoromethyl, and pentafluoroethyl.

The term "mercapto" means a -SH group.

The term "mercaptoalkyl" means a mercapto group, as defined herein, appended to the parent molecular moiety through an alkyl group, as defined herein. Representative examples of mercaptoalkyl include, but are not limited to, 2-mercaptoethyl and 3-mercaptopropyl.

The term "methylenedioxy" means an -OCH₂O- group wherein the oxygen atoms of the methylenedioxy are attached to the parent molecular moiety through two adjacent carbon atoms.

The term "nitrogen protecting group" means those groups intended to protect an amino group against undesirable reactions during synthetic procedures. Preferred nitrogen protecting groups are acetyl, benzoyl, benzyl, benzyloxycarbonyl (Cbz), formyl, phenylsulfonyl, tert-butoxycarbonyl (Boc), tert-butylacetyl, trifluoroacetyl, and triphenylmethyl (trityl).

The term "nitro" means a -NO₂ group.

The term "NZ₁Z₂" means two groups, Z₁ and Z₂, which are appended to the parent molecular moiety through a nitrogen atom. Z₁ and Z₂ are each independently selected from the group consisting of hydrogen, alkyl, alkylcarbonyl, alkoxycarbonyl, aryl, arylalkyl, formyl and (NZ₅Z₆)carbonyl. In certain instances within the invention, Z₁ and Z₂ taken together with the nitrogen atom to which they are attached form a heterocyclic ring. Representative examples of NZ₁Z₂ include, but are not limited to, amino, methylamino, acetylamino, acetylmethylamino, phenylamino, benzylamino, azetidinyl, pyrrolidinyl and piperidinyl.

The term "NZ₃Z₄" means two groups, Z₃ and Z₄, which are appended to the parent molecular moiety through a nitrogen atom. Z₃ and Z₄ are each independently selected from the group consisting of hydrogen, alkyl, aryl and arylalkyl. Representative examples of NZ₃Z₄ include, but are not limited to, amino, methylamino, phenylamino and benzylamino.

The term "NZ₅Z₆" means two groups, Z₅ and Z₆, which are appended to the parent molecular moiety through a nitrogen atom. Z₅ and Z₆ are each independently selected from the group consisting of hydrogen, alkyl, aryl and arylalkyl. Representative examples of NZ₅Z₆ include, but are not limited to, amino, methylamino, phenylamino and benzylamino,

The term "(NZ₃Z₄)carbonyl" means a NZ₃Z₄ group, as defined herein, appended to the parent molecular moiety through a carbonyl group, as defined herein. Representative examples of (NZ₃Z₄)carbonyl include, but are not limited to, aminocarbonyl, (methylamino)carbonyl, (dimethylamino)carbonyl, and (ethylmethylamino)carbonyl.

The term "oxo" means a =O moiety.

The term "sulfinyl" means a -S(O)- group.

The term "sulfonyl" means a -SO₂- group.

The term "tautomer" means a proton shift from one atom of a compound to another atom of the same compound wherein two or more structurally distinct compounds are in equilibrium with each other.

Although typically it may be recognized that an asterisk is used to indicate that the exact subunit composition of a receptor is uncertain, for example αb4* indicates a receptor that contains the α and β4 proteins in combination with other subunits, the term α7 as used herein is intended to include receptors wherein the exact subunit composition is both certain and uncertain. For example, as used herein α7 includes homomeric (α7)₅ receptors and α7* receptors, which denote a nAChR containing at least one α7 subunit.

### Compounds of the Invention

Compounds of the invention have the formula (I) 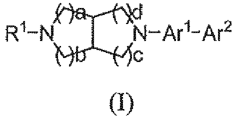 or a pharmaceutically acceptable salt or prodrug thereof, wherein
R¹ is selected from group consisting of hydrogen, alkyl, cyclic alkyl, haloalkyl, aryl, and heteroaryl;
a and c are each independently selected from 0, 1, or 2; b and d are each independently selected from 1, 2, or 3, provided that when both b and d are 1, a and c can not be 1 simultaneously;
Ar¹ is selected from 5- or 6-membered aromatic group of formula 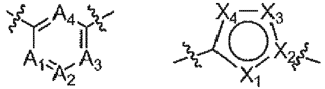 A₁, A₂, A₃ and A₄ are each independently selected from group consisting of-N- and -CR^{a};
X₁, X₃, X₄ are independently selected from group consisting of -CR^{a}, -NR^{a}, -O- and -S-;
X₂ is -C- or -N-, provided that when X₂ is -C-, at least one of X₁, X₃, X₄ is other than -C-;
R^{a} is selected from group consisting of hydrogen, or alkyl, cyclic alkyl, haloalkyl, aryl, heteroaryl, halogen, -CO₂R¹, -COR¹, -CONR¹, -OR¹, and -NR¹, wherein R¹ is selected from group consisting of hydrogen, alkyl, cyclic alkyl, haloalkyl, aryl, and heteroaryl;
Ar² is selected from a fused bicyclic aromatic group of formula 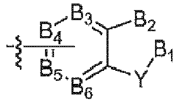 B₁, B₂, B₃, B₄, B₅, B₆ are each independently-N-, or -CR^{a}-;
Y is selected from the group consisting of-NR^{d}-, -O-, and -S-;
R^{a} is selected from the group consisting of hydrogen, alkyl, cyclic alkyl, haloalkyl, aryl, heteroaiyl, halogen, -CO₂R¹, -COR¹, -CONR¹, -OR¹, and -NR¹; and
R^{d} is selected from group consisting of hydrogen, alkyl, and cyclic alkyl.

More particularly, compounds of formular (I) are those having fused diazabicycloalkanes of formula (II)

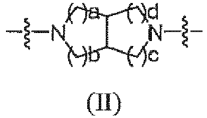

The variables a and c are each independently selected from 0, 1, 2; b and d are each independently selected from 1, 2, 3. Examples of fused diazabicycloalkanes at least include:

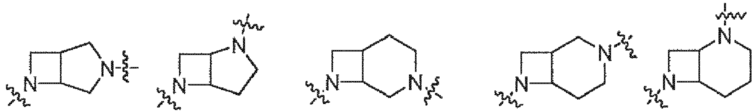

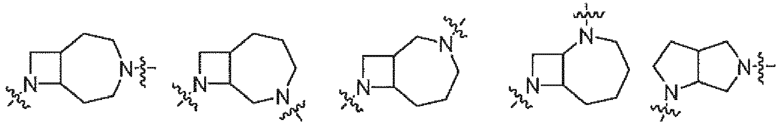

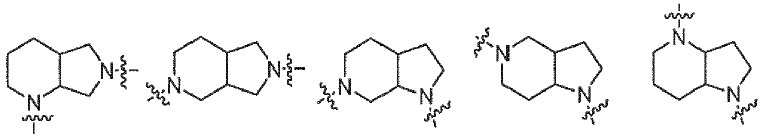

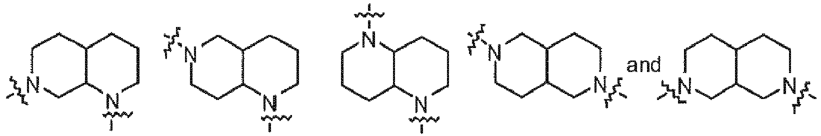

The Ar¹ moiety is independently selected from 5- or 6-membered aromatic group of formula (III) and (IV)

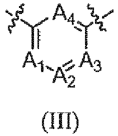

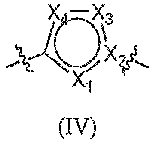

In Formula (III), A₁, A₂, A₃, A₄, are each independently selected from N or CR^{a}, wherein R^{a} is independently selected from group consisting of hydrogen, alkyl, cyclic alkyl, haloalkyl, aryl, heteroaryl, halogen, -CO₂R¹, -COR¹, -CONR¹, -OR¹, and -NR¹. The moiety is attached to fused diazabicycloalkanes and an Ar² group by 1,3-substitution or meta-attachment. Preferable, the moiety represented by Formula (III) contains at least one nitrogen atom.

Formula (IV) represents a 5-membered ring heteroaryl, wherein X₁, X₃, X₄ are independently selected from group consisting of -CR^{a}, -NR^{a}, -O-, and -S-; X² is -C- and - N-, provided that when X₂ is -C-, at least one of X₁, X₃, X₄ is other than -CR^{a}. W^{a} is independently selected from group consisting of hydrogen, alkyl, cyclic alkyl, haloalkyl, aryl, heteroaryl, halogen, -CO₂R¹, -COR¹, -CONR¹, -OR¹, and -NR¹. The moiety is generally attached to fused diazabicycloalkanes and Ar² group by 1,3-substitution.

Examples of specific 6- or 5-membered aromatic rings suitable for Ar₁ include imidazolyl, isoxazolyl, isothiazolyl, furyl, oxazolyl, 1,2,4-oxadiazolyl, 1,3,4-oxadiazolyl, phenyl, pyrazolyl, pyridinyl, pyrimidinyl, pyridazinyl, pyrazinyl, thiophenyl, thiazolyl, 1,2,4-thiadiazolyl, and 1,3,4-thiadiazolyl. Preferred Ar₁ groups are pyddazinyl, pyridinyl, thiazolyl, 1,3,4-thiadiazolyl, and 1,3,4-oxadiazolyl. Examples of aromatic rings suitable for formula (III) or (IV) at least include:

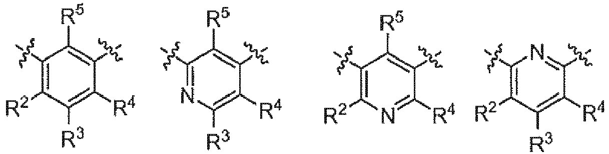

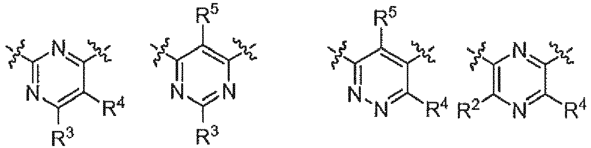

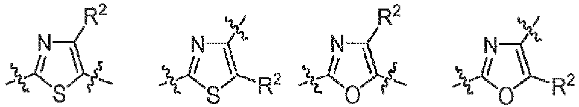

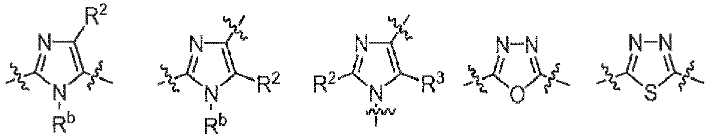

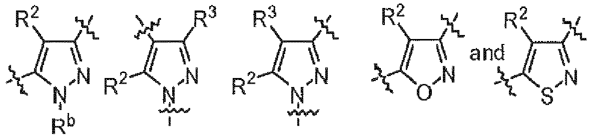

R² R³ R⁴, and R⁵ are each independently selected from group consisting of hydrogen, alkyl, alkoxy, alkoxycarbonyl, cyano, halo, nitro and -NR^{b}R^{c}; R^{b}, and R^{c} are each independently hydrogen, alkyl, alkoxycarbonyl, or alkylcarbonyl.

The Ar² moiety is independently selected from a fused bicyclicheteroaryl of formula (V)

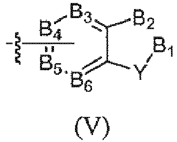

B₁, B₂, B₃, B₄, B₅, B₆ are each independently-N- or -CR^{a}-.

Y is selected from group consisting of -NR^{d}-, -O-, and -S-.

R^{a} is selected from group consisting of hydrogen, or alkyl, cyclic alkyl, haloalkyl, aryl, heteroaryl, halogen, -CO₂R¹, -COR¹, -CONR¹, -OR, and -NR¹.

R^{d} is independently selected from group consisting of hydrogen, alkyl, and cyclic alkyl.

The moiety Ar² is attatched to Ar¹ group through the C of B₁, B₂, B₃, B₄, B₅ and B₆. Preferably, the moiety represented by Formula (V) contains at least one heteroatom selected from N, O, and S. Examples of specific fused bicyclicheteroaromatic ring suitable for Ar² include benzofuranyl, benzo[*d*]imidazolyl, benzo[*d*]isoxazolyl, benzo[*d*]isothiazolyl, benzo[*d*]oxazolyl, benzo[*d*]thiazolyl, benzo[*b*]thiophenyl, furo[3,2-*b*]pyridinyl, furo[3,2-*c*]pyridinyl, imidazo[4,5-*b*]pyridinyl, imidazo[4,5-*c*]pyridine, indolyl, indazolyl, isoxazolo[4,5-*b*]pyridinyl, isoxazolo[4,5-*c*]pyridinyl, isoxazolo[5,4-*b*]pyridinyl, isoxazolo[5,4-*c*]pyridinyl, isothiazolo[4,5-*c*]pyridinyl, isothiazolo[4,5-*c*]pyridinyl, isothiazolo[5,4-*b*]pyridinyl, isothiazolo[5,4-*c*]pyridinyl, oxazolo[4,5-*b*]pyridinyl, oxazolo[4,5-*c*]pyridinyl, oxazolo[5,4-*b*]pyridinyl, oxazolo[5,4-*c*]pyridinyl, pyrazolo[3,4-*b*]pyridinyl, pyrazolo[3,4-*c*]pyridinyl, pyrazolo[4,3-*b*]pyridinyl, pyrazolo[4,3-*c*]pyridinyl, pyrrolo[2,3-*b*]pyridinyl, pyrrolo[2,3-*c*]pyridinyl, pyrrolo[3,2-*b*]pyridinyl, pyrrolo[3,2-*c*]pyridinyl, thiazolo[4,5-*b*]pyridinyl, thiazolo[4,5-*c*]pyridinyl, thiazolo[5,4-*b*]pyridinyl, thiazolo[5,4-*c*]pyridinyl, thieno[2,3-*b*]pyridinyl, thieno[2,3-*c*]pyridinyl, thieno[3,2-*b*]pyridinyl, and thieno[3,2-*c*]pyridinyl. Examples of suitable Ar² moieties at least include:

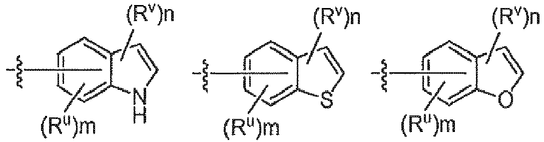

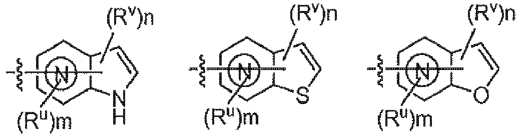

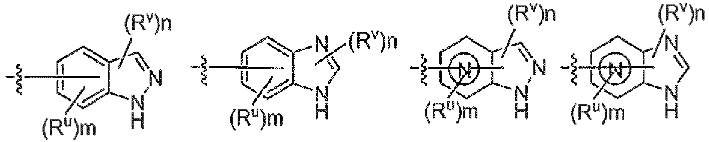

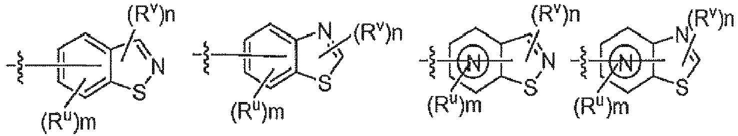

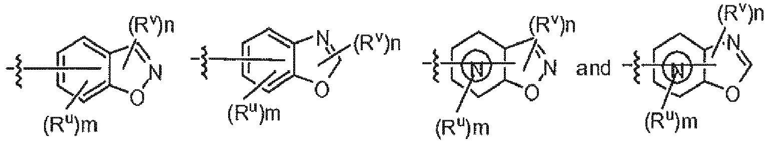

R^{u} and R^{v} are each independently selected from alkoxy, alkyl, cyano, haloalkyl, hydroxy, halogen and NR¹.

The variables m and n are are each independently selected from 0, 1 and 2.

Specific example of a particular embodiment of the compounds of formula (I) for invention is where the diazabicycloalkane is 3,6-diazabicyclo[3.2.0]heptane, for example:

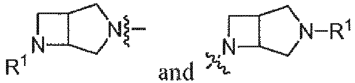

R¹ is hydrogen, alkyl, cyclic alkyl, or haloalkyl; more preferably hydrogen, alkyl, or cyclic alkyl. Ar¹ is oxazolyl, oxadiazolyl, pyridazinyl, pyrazinyl, pyridinyl, thiadiazolyl, or thiazolyl; more preferably pyridinyl, thiadiazolyl or thiazolyl. Ar² is benzofuranyl, benzo[*d*]imidazolyl, indolyl, indazolyl or pyrrolopyridinyl, pyrazolopyridinyl, imidazopyridinyl, more preferable indolyl or pyrrolopyridinyl.

Another example of a particular embodiment of the compounds of formula (I) for invention is where the diazabicycloalkane is 3,8-diazabicyclo[4.2.0]octane, for example:

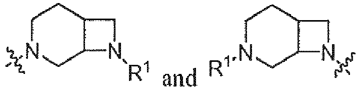

R¹ is hydrogen, alkyl, cyclic alkyl, haloalkyl; more preferably hydrogen, alkyl, or cyclic alkyl. Ar¹ is oxazoleyl, oxadiazolyl, pyridazinyl, pyrazinyl, pyndinyl, thiadiazolyl, or thiazolyl; more preferably pyridinyl, thiadiazolyl or thiazolyl. Ar² is benzofuranyl, benzo[*d*]imidazolyl, indolyl, indazolyl pyrrolopyridinyl, pyrazolopyridinyl, or imidazopyridinyl, more preferably indolyl or pyrrolopyridinyl.

Specific embodiments contemplated as part of the invention include compounds of Formula (I), or salts or prodrugs thereof, for example:
5-{5-[(*1S,5S*)-3,6-diazabicyclo[3.2.0]heptan-3-yl]pyridin-3-yl}-1H-indole;
5-{5-[(*1S,5S*)-6-methyl-3,6-diazabicyclo[3.2.0]heptan-3-yl]pyridin-3-yl}-*1H*-indole;
4-{5-[(*1S,5S*)-3,6-diazabicyclo[3.2.0]heptan-3-yl]pyridin-3-yl}-*1H*-indole;
4-{5-[(*1S,5S*)-6-methyl-3,6-diazabicyclo[3.2.0]heptan-3-yl]pyridin-3-yl}-*1H*-indole;
6-{5-[(*1S,5S*)-3,6-diazabicyclo[3.2.0]heptan-3-yl]pyridin-3-yl}-*1H*-indole;
6-{5-[(*1S,5S*)-6-methyl-3,6-diazabicyclo[3.2.0]heptan-3-yl]pyridin-3-yl}-*1H*-indole;
5-{5-[(*1S,5S*)-6-methyl-3,6-diazabicyclo[3.2.0]heptan-3-yl]pyridin-3-yl}-2-(trifluoromethyl)-*1H*-indole;
(*1S,5S*)-3-(5-(benzofuran-5-yl)pyridin-3-yl)-6-methyl-3,6-diazabicyclo[3.2.0]heptane;
5-{5-[(*1S,5S*)-6-methyl-3,6-diazabicyclo[3.2.0]heptan-3-yl]pyridin-3-yl}-1*H-*indazole;
(*1S,5S*)-3-[5-(benzo[b]thiophen-5-yl)pyridin-3-yl]-3,6-diazabicyclo[3.2.0]heptane;
(*1S,5S*)-3-[5-(benzo[b]thiophen-5-yl)pyridin-3-yl]-3,6-diazabicyclo[3.2.0]heptane;
7-{5-[(*1S,5S*)-6-methyl-3,6-diazabicyclo[3.2.0]heptan-3-yl]pyridin-3-yl}-1H-indole;
5-{5-[(*1S,5S*)-6-methyl-3,6-diazabicyclo[3.2.0]heptan-3-yl]pyridin-3-yl}-1H-benzo[d]imidazolc;
3-methyl-5-{5-[(*1S,5S*)-6-methyl-3,6-diazabicyclo[3.2.0]heptan-3-yl]pyridin-3-yl}-1*H*-indole;
3-{5-[(*1S,5S*)-3,6-diazabicyclo[3.2.0]heptan-3-yl]pyridin-3-yl}-9*H*-carbazole;
5-{5-[(*1S,5S*)-3,6-diazabicyclo[3.2.0]heptan-3-yl]pyridin-3-yl}-3-methyl-1*H*-indole;
3-(5-{(*1S,5S*)-6-methyl-3,6-diazabicyclo[3.2.0]heptan-3-yl)pyridin-3-yl)-9H-carbazole;
7-{5-[(*1S,5S*)-6-methyl-3,6-diazabicyclo[3.2.0]heptan-3-yl]pyridin-3-yl}-*1H* pyrrolo[2,3-c]pyridine;
5-{5-[(*1S,5S*)-6-methyl-3,6-diazabicyclo[3.2.0]heptan-3-yl]pyridin-3-yl}-1H-pyrrolo[2,3-b]pyridine;
3-{5-[(*1S,5S*)-6-methyl-3,6-diazabicyclo[3.2.0]heptan-3-yl]pyridin-3-yl}-1-(phenylsulfonyl)-1*H*-indole;
3-(5-((*1S,5S*)-6-methyl-3,6-diazabicyclo[3.2.0]heptan-3-yl)pyridin-3-yl)-1*H*-indole;
d-{6-[(*1S,5S*)-3,6-diazabicyclo[3.2.0]heptan-3-yl)pyrazin-2-yl}-1*H*-indole;
4-{6-[(*1S,5S*)-6-methyl-3,6-diazabicyclo[3.2.0]heptan-3-yl]pyrazin-2-yl}-1*H*-indole;
5-{6-[(*1S,5S*)-6-methyl-3,6-diazabicyclo[3.2.0]heptan-3-yl]pyrazin-2-yl}-1*H*-indole;
6-{6-[(*1S,5S*)-6-methyl-3,6-diazabicyclo[3.2.0]heptan-3-yl)pyrazin-2-yl}-1*H*-indole;
5-(6-((*1S,5S*)-6-methyl-3,6-diazabicyclo[3.2.0]heptan-3-yl)pyrazin-2-yl)-2-(trifluoromethyl)-1*H*-indole
5-{5-[(*1R,5R*)-6-methyl-3,6-diazabicyclo[3.2.0]heptan-3-yl]pyridin-3-yl}-1H-indole;
4-{5-[(*1R*,*5R*)-6-methyl-3,6-diazabicyclo[3.2.0]heptan-3-yl]pyridin-3-yl}-1H-indole;
6-{5-[(*1R,5R*)-6-methyl-3,6-diazabicyclo[3.2.0]heptan-3-yl]pyridin-3-yl}-1H-indole;
5-{5-[(*1R,5S*)-3,6-diazabicyclo[3.2.0]heptan-6-yl]pyridin-3-yl}-1*H*-indole;
5-{5-[(*1R,5S*)-3-methyl-3,6-diazabicyclo[3.2.0]heptan-6-yl]pyridin-3-yl}-1*H*-indole;
6-{5-[(*1R,5S*)-3,6-diazabicyclo[3.2.0]heptan-6-yl]pyridin-3-yl}-1*H*-indole;
6-{5-[(*1R,5S*)-3-methyl-3,6-diazabicyclo[3.2.0]heptan-6-yl)pyridin-3-yl}-1*H-*indole;
4-(5-((*1R,5S*)-3-methyl-3,6-diazabicyclo[3.2.0]heptan-6-yl)pyridin-3-yl)-1*H-*indole;
6-{5-[(*3aS,6aS*)-5-methylhexahydropyrrolo[3,4-b]pyrrol-1(2H)-yl]pyridin-3-yl}-1H-indole; and
5-{5-[(*3aS,6aS*)-5-methylhexahydropyrrolo[3,4-b]pyrrol-1(2H)-yl]pyridin-3-yl}-1*H-*indole,

Compounds of the invention can exist as stereoisomers wherein asymmetric or chiral centers are present. These stereoisomers are "R" or "S" depending on the configuration of substituents around the chiral element. The terms "R" and "S" used herein are configurations as defined in IUPAC 1974 Recommendations for Section E, Fundamental Stereochemistry, Pure Appl. Chem., 1976, 45: 13-30. The invention contemplates various stereoisomers and mixtures thereof and is specifically included within the scope of this invention. Stereoisomers include enantiomers, diastereomers, and mixtures of enantiomers or diastereomers. Individual stereoisomers of the invention can be prepared synthetically from commercially available starting materials that contain asymmetric or chiral centers or by preparation of racemic mixtures followed by resolution. These methods of resolution are exemplified by (1) attachment of a mixture of enantiomers to a chiral auxiliary, separation of the resulting mixture of diastereomers by recrystallization or chromatography and optional liberation of the optically pure product from the auxiliary as described in Furniss, Hannaford, Smith, and Tatchell, "Vogel's Textbook of Practical Organic Chemistry", 5th edition (1989), Longman Scientific & Technical, Essex CM20 2JE, England, or (2) direct separation of the mixture of optical enantiomers on chiral chromatographic columns or (3) fractional recrystallization methods.

### Methods for preparing compounds of the invention

As used in the descriptions of the schemes and the examples, certain abbreviations are intended to have the following meanings: Bu for butyl; DMAP for 4-dimethylaminopyridine; DMF for dimethyl formamide; DME for 1,2-dimethoxyethane; Et for ethyl; EtOAc for ethyl acetate; HPLC for high pressure liquid chromatography; Me for methyl; MeOH for methanol; OAc for acctoxy; Pd/C for palladium on carbon; Ph for phenyl; and THF for tetrahydrofuran.

The reactions exemplified in the schemes are performed in a solvent appropriate to the reagents and materials employed and suitable for the transformations being effected. The described transformations may require modifying the order of the synthetic steps or selecting one particular process scheme over another in order to obtain a desired compound of the invention, depending on the functionality present on the molecule.

Nitrogen protecting groups can be used for protecting amine groups in the described compounds. Such methods and some suitable nitrogen protecting groups are described in Greene and Wuts (Protective Groups In Organic Synthesis, Wiley and Sons, 1999). For example, suitable nitrogen protecting groups include tert-butoxycarbonyl (Boc), benzyloxycarbonyl (Cbz), benzyl (Bn), acetyl, and trifluoroacetyl. More particularly, the BOC protecting group can be removed by treatment with an acid such as trifluoroacetic acid or hydrochloric acid. The Cbz and Bn protecting groups can be removed by catalytic hydrogenation. The acetyl and trifluoroacetyl protecting groups can be removed by a hydroxide ion.

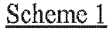

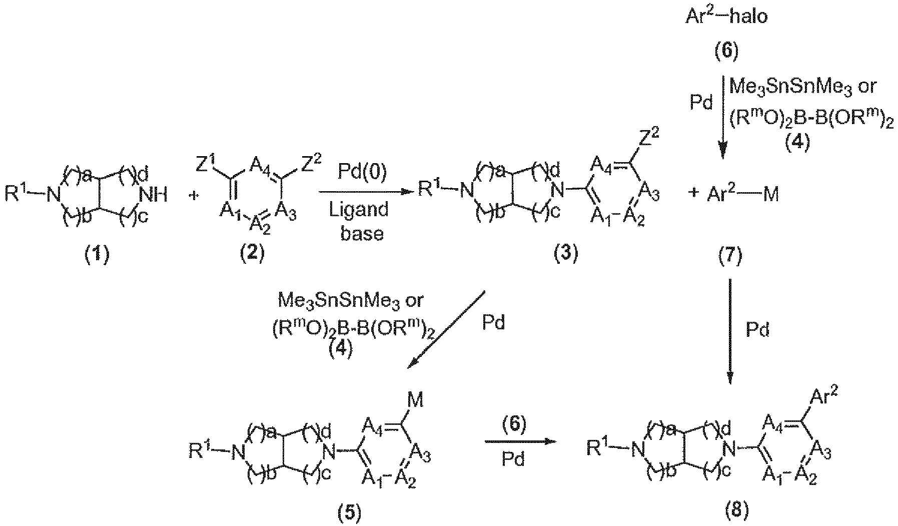

Compounds of formula (8), wherein R¹, a, b, c, d, A₁, A₂, A₃, A₄ and Ar² are as defined in formula (I), can be prepared as described in Scheme 1. Compounds of formula (1), (either commercially available, or prepared by well-known methods) when treated with a compound of formula (2), wherein Z¹ is bromide, chloride, or iodide, and Z², is bromide, chloride, iodide or Ar², in the presence of a ligand, such as BINAP, Xantphos, dicyclohexyl(2',4',6'-triisopropylbiphenyl-2-yl)phosphine, dicyclohexyl(2',6'-diisopropoxybiphenyl-2-yl)phosphine and 2'-(dicyclohexylphosphino)-N,N-dimethylbiphenyl-2-amine, and a palladium catalyst, such as Pd(OAc)₂, PdCl₂(PPh₃)₂, Pd(PPh₃)₄, PdCl₂(dppf), Pd₂(dba)₃, (with a base, such as *^{t}*BuONa and Cs₂CO₃), in a solvent, such as toluene, at 110 °C as described in Org. Lett., 2005, 7, 3965, provide compounds of formula (3). When Z² is Ar², compounds of formula (3) are representative of the invention. When Z² is a halogen, compounds of formula (3), when treated with hexametlylditin or an organo-borane compound of formula (4), such as bis(pinacolato)diboron or bis(catecholato)diboron, wherein R^{m} is hydrogen, alkyl or aryl, in the presence of a palladium catalyst, such as PdCl₂(PPh₃)₂, PdCl₂(dppf) provide the corresponding tin or boronic acid/esters of formula (5), wherein M is -SnMe₃ or -B(OR^{m})₂. Compounds of formula (5) when treated with compounds of formula (6), wherein Ar² is as defined in formula (I) and halo is bromide, chloride, or iodide, in the presence of a palladium catalyst, such as Pd(OAc)₂, PdCl₂(PPh₃)₂, Pd(PPh₃)₄, PdCl₂(dppf), Pd₂(dba)₃, will provide compounds of formula (8). Alternatively, compounds of formula (6) when treated with hexamethylditin or a di-borane containing compound of formula (4), such as bis(pinacolato)diboron and bis(catecholato)diboron, in the presence of a palladium catalyst, such as PdCl₂(PPh₃)₂, PdCl₂(dppf), provide a organotin or organoboronic acid/esters containing compounds of formula (7), wherein Ar² is as defined in formaul (I), and M is -SnMe₃ or -B(OR^{m})₂. Compounds of formula (7) when treated with a compound of formula (3) in the presence of a palladium catalyst, such as Pd(OAc)₂, PdCl₂(PPh₃)₂, Pd(PPh₃)₄, PdCl₂(dppf), Pd₂(dba)₃, provide a compound of formula (8).

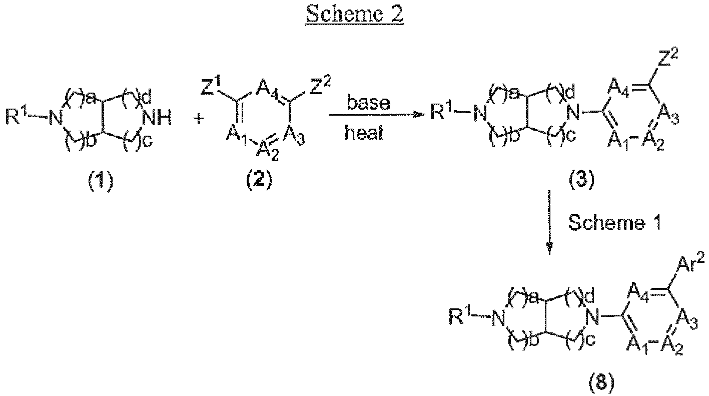

Alternatively, compounds of formula (8), wherein at least one of A₁ and A₄ is N, R¹, a, b, c, d, A₂, A₃ and Ar² are as defined in formula (1) can be prepared as described in Scheme 2. Compounds of formula (1), when treated with a compound of formula (2), wherein Z¹ is bromide, chloride, or iodide and Z² is bromide, chloride, iodide or Ar², in the presence of a base, such as but not limited to, Na₂CO₃, K₂CO₃, Cs₂CO₃ and *N, N*-diisopropyl ethylamine, in a solvent such as DMSO or NMP at 110 °C provide compounds of formula (3). Compounds of formula (3) can be transformed to compounds of formula (8) as described in Scheme 1.

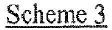

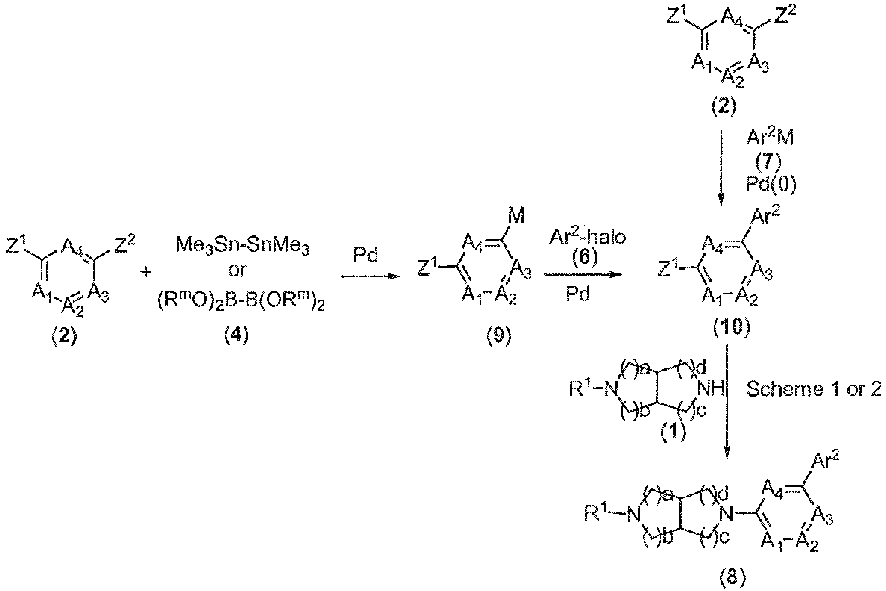

Another method of generating compounds of formula (8), wherein at least one of A₁ and A₄, is N, R¹, a, b, c, d, A₂, A₃ and Ar² are as defined in formula (I) is described in Scheme 3. Compounds of formula (2), wherein Z¹ and Z² are each independently bromide, chloride, or iodide, when treated with hexamethylditin or an organo-borane compound of formula (4), such as bis(pinacolato)diboron or bis(catecholato)diboron, wherein R^{m} is hydrogen, alkyl or aryl, in the presence of a palladium catalyst, such as PdCl₂(PPh₃)₂, PdCl₂(dppf) provide the corresponding tin or boronic acid/esters of formula (9), wherein M is -SnMe₃ of -B(OR^{m})₂. Compounds of formula (9) when treated with compounds of formula (6), wherein Ar² is as defined and halo is bromide, chloride, or iodide, in the presence of a palladium catalyst, such as Pd(OAc)₂, PdCl₂(PPh₃)₂, Pd(PPh₃)₄, PdCl₂(ddpf), Pd₂(dba)₃, provide compounds of formula (10). Alternatively, compounds of formula (2) when treated with compounds of formula (7) in the presence of a palladium catalyst such as Pd(OAc)₂, PdCl₂(PPh₃)₂, Pd(PPh₃)₄, PdCl₂(ddpf), Pd₂(dba)₃, provide compounds of formula (10). Compounds of formula (10) can be transformed to compounds of formula (8) as described in Schemes 1 and 2.

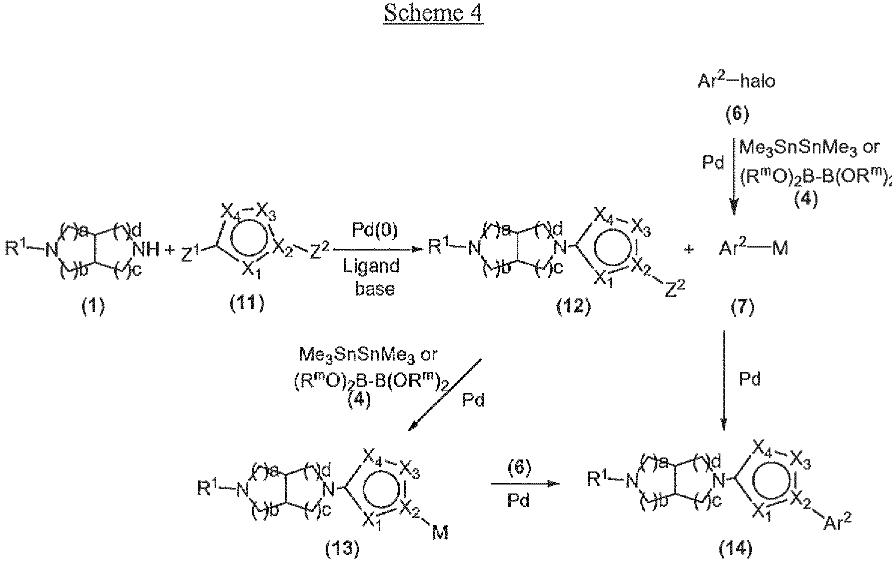

Compounds of formula (14), wherein R¹, a, b, c, d, X₁, X₂, X₃, X₄ and Ar² are as defined in formula (I), can be prepared as described in Scheme 4. Compounds of formula (1) when treated with a compound of formula (11), wherein Z¹ is bromide, chloride, or iodide and Z² is bromide, chloride, iodide or Ar², provided that when Z² is bromide, chloride or iodide, X₂ is C, in the presence of a ligand, such as BINAP, Xantphos, dicyclohexyl(2',4',6'-triisopropylbiphenyl-2-yl)phosphine, dicyclohexyl(2',6'-diisopropoxybiphenyl-2-yl)phosphine and 2'-(dicyclohexylphosphino)-N,N-dimethylbiphenyl-2-amine, and a palladium catalyst, such as Pd(OAc)₂, PdCl₂(PPh₃)₂, Pd(PPh₃)₄, PdCl₂(dppf), Pd₂(dba)₃, with a base, such as BuONa and Cs₂CO₃, in a solvent such as toluene at 110 °C as described in Org. Lett., 2005, 7, 3965, provide compounds of formula (12). When Z² is Ar², compounds of formula (12) are representative of the present invention. When Z² is a halogen, compounds of formula (12) when treated with hexamethylditin or an organo-borane compound of formula (4), such as bis(pinacolato)diboron or bis(catecholato)diboron, wherein R^{m} is hydrogen, alkyl or aryl, in the presence of a palladium catalyst, such as PdCl₂(PPh₃)₂, PdCl₂(dppf) provide the corresponding tin or boronic acid/esters of formula (13), wherein M is -SnMe₃ or -B(OR^{m})₂. Compounds of formula (13) when treated with compounds of formula (6), wherein Ar² is as defined and halo is bromide, chloride, or iodide, in the presence of a palladium catalyst, such as Pd(OAc)₂, PdCl₂(PPh₃)₂, Pd(PPh₃)₄, PdCl₂(dppf), Pd₂(dba)₃, provide compounds of formula (14). Alternatively, compounds of formula (6) when treated with hexamethylditin or a di-borane containing compound of formula (4), such as bis(pinacolato)diboron and bis(catecholato)diboron, in the presence of a palladium catalyst, such as PdCl₂(PPh₃)₂, PdCl₂(dppf), will provide an organotin or organoboronic acid/esters containing compounds of formula (7), wherein Ar² is as defined and M is -SnMe₃ or -B(OR^{m})₂. Compounds of formula (7) when treated with a compound of formula (13) in the presence of a palladium catalyst, such as Pd(OAc)₂, PdCl₂(PPh₃)₂, Pd(PPh₃)₄, PdCl₂(dppf), Pd₂(dba)₃, can provide a compound of formula (14).

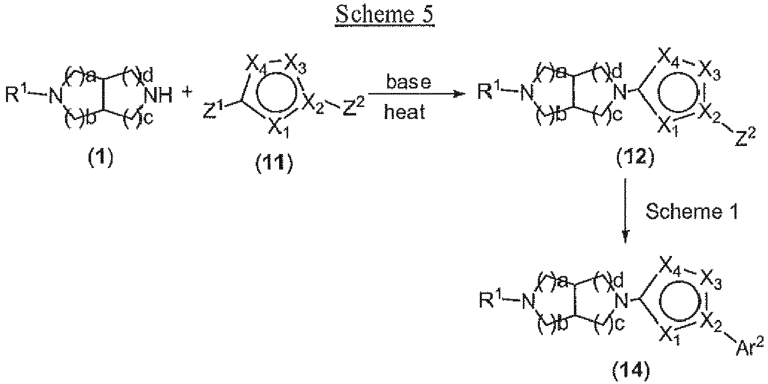

Alternatively, compounds of formula (14), wherein at least one of X₁ and X₄ is N and R¹, a, b, c, d, X₂, X₃, and Ar² are as previously defined in formula (I), can be prepared as described in Scheme 5. Compounds of formula (1) when treated with a compound of formula (11), wherein Z¹ is bromide, chloride, or iodide and Z² is bromide, chloride, iodide or Ar², provided that when Z² is bromide, chloride or iodide, X₂ is C, in the presence of a base, such as Na₂CO₃, K₂CO₃, Cs₂CO₃ and N,N-diisopropyl ethylamine, in a solvent such as DMSO or NMP at 110 °C as described provide compounds of formula (12). Compounds of formula (12) can be transformed to compounds of formula (14) as described in Scheme 4.

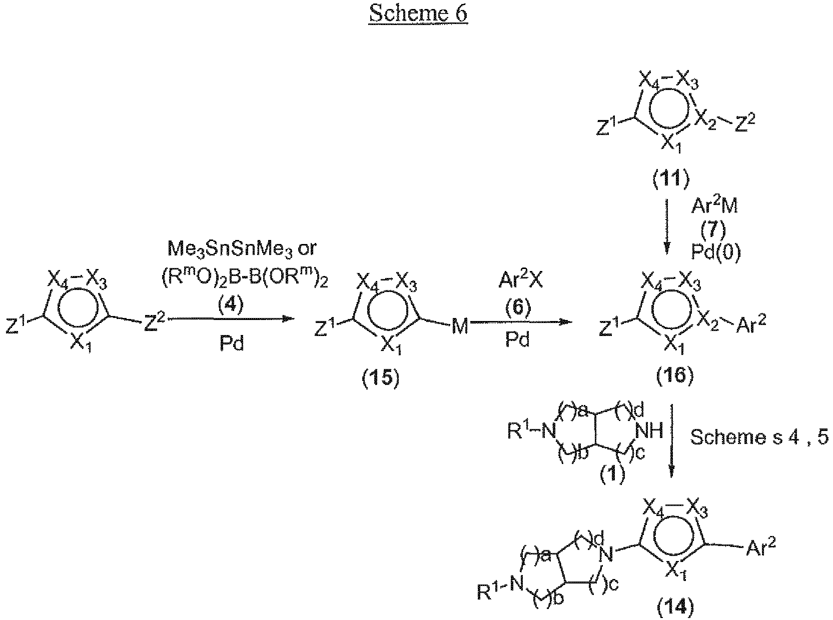

Another method of generating compounds of formula (14) wherein at least one of X₁ and X₄ is N and R¹, a, b, c, d, X₂, X₃, and Ar² are as previously defined in formula (1) is described in Scheme 6. Compounds of formula (11), wherein Z¹ and Z² are each bromide, chloride, or iodide, and X₁, X₃ and X₄ are as previously defined, when treated with hexamethylditin or an organo-borane compound of formula (4), such as bis(pinacolato)diboron or bis(catecholato)diboron, wherein R^{m} is hydrogen, alkyl or aryl, in the presence of a palladium catalyst, such as PdCl₂(PPh₃)₂, PdCl₂(dppf) provide the corresponding tin or boronic acid/esters of formula (15), wherein M is -SnMe₃ or -B(OR^{m})₂. Compounds of formula (15) when treated with compounds of formula (6), wherein Ar² is as defined if formula (I) and halo is bromide, chloride, or iodide, in the presence of a palladium catalyst, such as Pd(OAc)₂, PdCl₂(PPh₃)₂ Pd(PPh₃)₄, PdCl₂(dppf), Pd₂(dba)₃, provide compounds of formula (16). Altenatively, compounds of formula (11) when treated with compounds of formula (7) in the presence of a palladium catalyst such as Pd(OAc)₂, PdCl₂(PPh₃)₂, Pd(PPh₃)₄, PdCl₂(dppf), Pd₂(dba)₃, provide compounds of formula (16). Compounds of formula (16) can be transformed to compounds of formula (14) as described in Schemes 4 and 5.

The compounds and intermediates of the invention can be isolated and purified by methods well-known to those skilled in the art of organic synthesis. Examples of conventional methods for isolating and purifying compounds include chromatography on solid supports, such as silica gel, alumina, or silica derivatized with alkylsilane groups; by recrystallization at high or low temperature with an optional pretreatment with activated carbon, thin-layer chromatography, distillation at various pressures; sublimation under vacuum; and trituration, as described for instance in "Vogel's Textbook of Practical Organic Chemistry", 5th edition (1989), by Furniss, Hannaford, Smith, and Tatchell, pub. Longman Scientific & Technical, Essex CM20 2JE, England.

The compounds of the invention have at least one basic nitrogen whereby the compound can be treated with an acid to form a desired salt. For example, a compound can be reacted with an acid at or above room temperature to provide the desired salt, which is deposited and collected by filtration after cooling. Examples of acids suitable for the reaction include toluenesulfonic, fumaric, trifluoroacetic and the like.

The compounds of the invention and processes for making these compounds can be better understood by reference to the following Examples, which are intended as an illustration of, and not a limitation upon, the scope of the invention.

### Example 1

### 5-{5-[(1S,5S)-3,6-diazabicylo[3.2.0]heptan-3-yl]pyridin-3-yl}-1H-indole bistosylate

### Example 1A

### (1R,5S)-tert-butyl 3-(5-bromopyridin-3-yl)-3,6-diazabicyclo[3.2.0]heptane-6-carboxylate

3,5-dibromopyridine (Aldrich, 2.60 g, 15 mmol) was coupled with (*1R,5S*)*-tert-*butyl 3,6-diazabicyclo[3.2.0]heptane-6-carboxylate (US 2006035936, 1.98 g, 10 mmol) under the catalysis of Pd₂(dba)₃ (Aldrich, 183.6 mg, 0.2 mmol) and 2,2'-bis(diphenylphosphino)-1,1'-binaphthyl (Aldrich, 373 mg, 0.6 mmol) in the presence of Cs₂CO₃ (Aldrich, 6.50 g, 20.0 mmol) in toluene (anhydrous, Aldrich, 50 mL) at 110 °C for 48 h. After the completion of the reaction, the reaction mixture was cooled to ambient temperature and diluted with EtOAc (100 mL). The inorganic solid was filtered off. The organic solution was washed with brine (2 x 20 mL) and concentrated under reduced pressure. The residue was purified with chromatography (SiO₂, EtOAc/hexane, v. 50/50, R_{f}=0.40) to give the title compound (3.05 g, yield, 86%). ¹H NMR (300 MHz, CD₃OD) δ ppm 1.45 (s, 9 H), 2.95 (dd, *J*=11.02, 4.24 Hz, 1 H), 3.05 (dd, *J*=10.51, 6.44 Hz, 1 H), 3.16 - 3.28 (m, 1 H), 3.51 - 3.67 (m, 1 H), 3.79 (d, *J*=10.51 Hz, 1 H), 3.87 - 3,98 (m, 1 H), 4.03 - 4.19 (m, 1 H), 4.83 - 4.91 (m, Hz, 1 H), 7.40 (t, *J*=2.20 Hz, 1 H), 7.97 (d, *J*=1.70 Hz, 1 H) 8.05 (d, *J*=2.37 Hz, 1 H); MS (DCI/NH₃) m/z 354 (M+1)⁺, 356 (M+1)⁺.

### Example 1B

### (1R,5S)-tert-butyl 3-[5-(1H-indol-5-yl)pyridin-3-yl]-3,6-diazabicyclo[3.2.0]heptane-6-carboxylate

The product of Example 1A (1.0 g, 2.82 mmol) was coupled with *1H*-indol-5-ylboronic acid (Frontier, 677 mg, 4.23 mmol) under the catalysis of Pd₂(dba)₃ (Aldrich, 18.4 mg, 0.02 mmol) and Pd('Bu₃P)₂ (Strem Chemicals, 20.5 mg, 0.04 mmol) with CsF (Aldrich, 2.55 g, 17 mmol) in dioxane (50 mL) at 80°C for 15 h. After the reaction was completed, it was cooled to ambient temperature and diluted with EtOAc (100 mL). The mixture was then washed with brine (2 x 20 mL) and concentrated. The residue was purified with chromatography (SiO₂ EtOAc/hexane, v. 50/50, R_{f}=0.40) to give the title compound (1.05 g, yield, 95%). ¹H NMR (300 MHz, CD₃OD) δ ppm 1.45 [s (br.), 9 H], 2.87 - 2.99 (m, 1 H), 3.05 (dd, *J*=10.22, 6.56 Hz, 1 H), 3.15-3.30 (m, 2 H), 3.55 - 3.75 (m, 1 H), 3.82 - 3.93 (m, 1 H), 3.96 - 4.26 (m, 3 H), 6.53 (d, *J*=2.75 Hz, 1 H), 7.28 (d, *J*=3.05 Hz, 1 H) 7.39 (dd, 1 H) 7.43 (s, 1 H) 7.49 (d, *J*=8.54 Hz, 1 H) 7.83 (s, 1 H) 8.01 (d, *J*=2.44 Hz, 1 H) 8.21 (s, 1 H); MS (DCI/NH₃) m/z 391(M+1)⁺.

### Example 1C

### 5-{5-[(1S,5S)-3,6-diazabicyclo[3.2.0]heptan-3-yl]pyridin-3-yl}-1H-indole

The product of Example 1B (500 mg, 1.28 mmol) was treated with 2,2,2-trifluoroacetic acid (Aldrich, 2 mL) in dichloromethane (5.0 mL) at ambient temperature for 10 h. The solution was then concentrated under reduced pressure. The residue was diluted with CHCl₃ (50 mL) and washed with saturated Na₂CO₃ (2 x 5 mL). The organic solution was concentrated, and the residue was purified using chromatography (SiO₂, EtOAc/MeOH (with 2 v.% NH₃·H₂O), 50/50, R_{f}=0.20) to give the title compound (360 mg, yield, 97.0%). ¹H NMR (300 MHz, CD₃OD) δ ppm 2.91 - 3.09 (m, 2 H), 3.36 - 3.51 (m, 2 H), 3.69 - 3.83 (m, 2 H), 3.85 - 3.97 (m, 1 H), 4.49 - 4.69 (m, 1 H), 6.55 (t, *J*=7,36 Hz, 1 H), 6.77 - 6.93 (m, 2 H), 7.00 (t, *J*=7.21 Hz, 1 H), 7.15 (s, 1 H), 7.25 - 7.40 (m, 2 H), 8.00 (s, 1 H), MS (DCI/NH₃) m/z 291(M+1)⁺.

### Example 1D

### 5-{5-[(1S,5S)-3,6-diazabicyclo[3.2.0]heptan-3-yl]pyridin-3-yl}-1H-indole bistosylate

The product of Example 1C (360 mg, 1.24 mmol) was treated with *p*-TsOH·H₂O (570 mg, 3.0 mmol) in EtOAc (20 mL) at 80°C for 1 h, then ambient temperature overnight to give the title compound (400 mg, yield, 69.8%). ¹H NMR (300 MHz, CD₃OD) δ ppm 3.33 (dd, *J*=12.88, 6.40 Hz, 1 H), 3.44 (dd, *J*=12.88, 5.09 Hz, 1 H), 3.57-3.64 (m, 1 H), 3.82 (dd, *J*=11.30, 5.40 Hz, , 1 H), 4.12 (d, *J*=10.85 Hz, 1 H), 4.28 -4,38 (m, 2H), 5.15 (dd, *J*=6.78, 5,42 Hz, 1 H), 6.60 (d, *J*=3.05 Hz, 1 H), 7.19 (d, *J*=8.14 Hz, 4 H), 7.37 (d, *J*=3.05 Hz, 1 H), 7.48 - 7.61 (m, 2 H), 7,67 (d, *J*=8.14 Hz, 4 H), 8.02 (s, 1 H), 8.11 - 8.17 (m, 1 H), 8.22 (d, *J*=2.71 Hz, 1 H), 8.48 (s, 1 H); MS (DCI/NH₃) m/z 291 (M+1)⁺. Anal. calcd. for C₁₈H₁₈N₄·2.00TsOH·0.90H₂O: C, 59.60; H, 5.73; N, 8.42. Found: C, 59.26; H, 5.34; N, 8.50.

### Example 2

### 5-{5-[(1S,5S)-6-methyl-3,6-diazabicyclo[3.2.0]heptan-3-yl]pyridin-3-yl}-1H-indole bisfumarate

### Example 2A

### 5-{5-[(1S,5S)-6-methyl-3,6-diazabicyclo[3.2.0]heptan-3-yl]pyridin-3-yl}-1H-indole

The product of Example 1D (200 mg, 0.32 mmol) was treated with formaldehyde (Aldrich, 37%, 33 L, 0.40 mmol) in the presence of NaBH(OAc)₃ (Aldrich, 212 mg, 1.0 mmol) in MeCN (3.0 mL) and water (1.0 mL) at ambient temperature for 10 h, The solid was filtered off and the filtrate was dirrectly purified with chromatography (SiO₂ EtOAc/MeOH (with 2 v.% NH_{3·}H₂O), 50/50, R_{f}=0.20) to give the title compound (80 mg, yield, 82.0%). ¹H NMR (300 MHz, CD₃OD) δ ppm 2.62 (s, 3 H), 3.08 (dd, *J*=11.66, 4.60 Hz, 1 H), 3.19 (dd, *J*=10.13, 7.06 Hz, 1 H), 3.32 - 3.41 (m, 1 H), 3.59 (dd, *J*=9.36, 4.14 Hz, 1 H), 3.71 (t, *J*=8.75 Hz, 1 H), 3.89 (d, *J*=10.13 Hz, 1 H), 4.01 (d, *J*=11.66 Hz, 1 H), 4.40 (dd, *J*=6.75, 4,60 Hz, 1 H), 6.52 (d, *J*=3.07 Hz, 1 H), 7.29 (d, *J*=3.07 Hz, 1 H), 7.39 (dd, *J*=8.29, 1.53 Hz, 1 H), 7.43 - 7.54 (m, 2 H), 7.83 (s, 1 H), 8.03 (d, *J*=2.45 Hz, 1 H), 8.23 (s, 1 H); MS (DCI/NH₃) m/z 305(M+1)⁺.

### Example 2B

### 5-{5-[(1S,5S)-6-methyl-3,6-diazabicyclo[3.2.0]heptan-3-yl]pyridin-3-yl}-1H-indole bisfumarate

The product of Example 2A (80 mg, 0.26 mmol) was treated with fumaric acid (Aldrich, 58 mg, 0.5 mmol) in EtOAc/EtOH (v. 10/1, 5 mL) at ambient temperature overnight to give the title compound (110 mg, yield, 78.9%). ¹H NMR (300 MHz, CD₃OD δ ppm 2.97 (s, 3 H) 3.11 (dd, *J*=8.50, 6.20 Hz1 H), 3.19 (dd, *J*=12.90, 4.70 Hz, 1 H) 3.46 - 3.64 (m, 1 H), 3.97 - 4.09 (m, 2 H), 4.19 - 4.37 (m, 2 H), 4.93 - 5.02 (m, 1 H), 6.53 (d, *J*=3.05 Hz, 1 H), 6.76 (s, 5 H), 7.30 (d, *J*=3.05 Hz, 1 H), 7.41 (dd, *J*=8.40, 1.70 Hz1 H) 7.51 (d, *J*=8.20 Hz, 1 H) 7.62 (t, *J*=2.03 Hz, 1 H), 7.86 (d, *J*=1.70 Hz, 1 H) 8.15 (d, *J*=2.37 Hz, 1 H) 8.34 (d, *J*=1.70 Hz, 1 H); MS (DCI/NH₃) m/z 305 (M+1)⁺. Anal. calcd. for C₁₉H₂₀N₄·2.41C₄H₄O₄·2.40H₂O: C, 54.83; H, 5.53; N, 8.93. Found: C, 54.41; H, 5.13; N, 9.33.

### Example 3

### 4-{5-[(1S,5S)-3,6-diazabicyclo[3.2.0]heptan-3-yl]pyridin-3-yl}-1H-indole bistosylate

### Example 3A

### (1R,5S)-tert-butyl 3-[5-(1H-indol-4-yl)pyridin-3-yl]-3,6-diazabicyclo[3.2.0]heptane-6-carrtjaxylate

The product of Example 1A (240 mg, 0.68 mmol) was coupled with *1H*-indol-4-ylboronic acid (Frontier, 164 mg, 1.02 mmol) under the catalysis of Pd(PPh₃)₄ (Aldrich, 7.8 mg, 0.007 mmol) dioxane (4.0 mL) and K₂CO₃ aqueous solution (2M, 1.0 mL) at 80 °C for 12 h. After the reaction was completed, it was cooled to ambient temperature and diluted with EtOAc (40 mL). The mixture was then washed with brine (2 x 10 mL) and concentrated. The residue was purified using chromatography (SiO₂ EtOAc/hexane, v. 75/25, R_{f}=0.30) to give the title compound (220 mg, yield, 83%). ¹H NMR (300 MHz, CD₃OD) δ ppm 1.45 [s (br.), 9 H], 2.98 (dd, *J*=10.85, 4.41 Hz, 1 H), 3.08 (dd, *J*=10.51, 6.44 Hz, 1 H), 3.21 - 3.28 (m, 1 H), 3.59 - 3.72 (m, 1 H), 3.87 (d, *J*=10.17 Hz, 1 H), 3.98 - 4.19 (m, 2 H), 4.78 - 4.93 (m, 1H), 6.59 (d, *J*=2.71 Hz, 1 H), 7.13 (dd, *J*=7.40, 2.00 Hz, 1 H), 7.22 (t, *J*=7.63 Hz, 1 H), 7.32 (d, *J*=3.39 Hz, 1 H), 7.44 (d, *J*=8.14 Hz, 1 H), 7.47 - 7.50 (m, 1 H), 8.09 (d, *J*=2.71 Hz, 1 H), 8.23 (d, *J*=1.70 Hz, 1 H); MS (DCI/NH₃) m/z 391(M+1)⁺.

### Example 3B

### 4-{5-[(1S,5S)-3,6-diazabicyclo[3.2.0]heptan-3-yl]pyridin-3-yl}-1H-indole bistosylate

The product of Example 3A (220 mg, 0.56 mmol) was treated with *p*-TsOH·H₂O (235 mg, 1.24 mmol) in EtOAc (20 mL) at 80 °C for 4 h, then ambient temperature overnight to give the title compound (210 mg, yield, 59.1%). ¹H NMR (300 MHz, CD₃OD) δ ppm 3.33 (dd, *J*=12.88, 6.40 Hz, 1 H), 3.44 (dd, *J*=12.88, 5.09 Hz, 1 H), 3.57-3.64 (m, 1 H), 3.82 (dd, *J*=11.30, 5.40 Hz, , 1 H), 4.12 (d, *J*=10.85 Hz, 1 H), 4.28 -4.38 (m, 2H), 5.15 (dd, *J*=6.78, 5.42 Hz, 1 H), 6.60 (d, *J*=3.05 Hz, 1 H), 7.19 (d, *J*=8.14 Hz, 4 H), 7.37 (d, *J*=3.05 Hz, 1 H), 7.48 - 7.61 (m, 2 H), 7.67 (d, *J*=8.14 Hz, 4 H), 8.02 (s, 1 H), 8.11- 8.17 (m, 1 H), 8.22 (d, *J*=2.71 Hz, 1 H), 8.48 (s, 1 H); MS (DCI/NH₃) m/z 291 (M+1)⁺, Anal. calcd. for C₁₈H₁₈N₄·2.40TsOH·0.80H₂O: C, 58.21; H, 5.45; N, 7.80. Found: C, 58.06; H, 5.44; N, 7.67.

### Example 4

### 4-{5-[(1S,5S)-6-methyl-3,6-diazabicyclo[3.2.0]heptan-3-yl]pyridin-3-yl}-1H-indole bistosylate

### Example 4A

### 4-{5-[(1S,5S)-6-methyl-3,6-diazabicyclo[3.2.0]heptan-3-yl]pyridin-3-yl}-1H-indole

The product of Example 3B (200 mg, 0.32 mmol) was treated with formaldehyde (Aldrich, 37%, 33 µL, 0.40 mmol) according to the procedure of Example 2A. The title compound was purified by preparative HPLC (Gilson, Xterra® column, 7 µm, 40 x 100 mm, eluting solvent, MeCN /H₂O (with 0.1 M NH₄HCO₃/NH₄OH, PH=10) (v. 90/10 to 10/90 over 25 minutes), flow rate, 40 mL/min., uv, 254 nm) (60 mg, yield, 62.0%). ¹H NMR (300 MHz, CD₃OD) δ ppm 2.78 (s, 3 H), 3.10 - 3.22 (m, 2 H), 3.38 - 3.51 (m, 1 H), 3.79 (dd, *J*=10.17, 4.41 Hz, 1 H), 3.90 - 4.01 (m, 2 H), 4.12 (dd, *J*=12.50, 7.80 Hz, 1 H), 4.66 (dd, *J*=7.12, 4.75 Hz, 1 H), 6.59 (d, *J*=3.05 Hz, 1 H), 7.14 (d, *J=*7.12 Hz, 1 H), 7.22 (t, *J*=7.50 Hz,1 H), 7.33 (d, *J*=3.05 Hz, 1 H), 7.46 (d, *J*=8.14 Hz, 1 H), 7.54 - 7.61 (m, 1 H), 8.16 (d, *J*=2.71 Hz, 1 H), 8.30 (d, *J*=1.70 Hz, 1 H); MS (DCI/NH₃) m/z 305(M+1)⁺.

### Example 4B

### 4-{5-[1S,5S]-6-methyl-3,6-diazabicyclo[3.2.0]heptan-3-yl]pyridin-3-yl}-1H-indole bistosylate

The product of Example 4A (50 mg, 0.16 mmol) was treated with TsOH·H₂O (Aldrich, 62 mg, 0.33 mmol) in EtOAc/EtOH (v. 10/1, 5 mL) at ambient temperature overnight to give the title compound (45 mg, yield, 43.4%). ¹H NMR (300 MHz, Pyridine-D₅) δ ppm 2.16 (s, 6 H), 3.04 (s, 3 H), 3.08 (dd, *J*=12.73, 5.06 Hz, 1 H), 3.09 (dd, *J*=12.50, 4.90 Hz, 1H), 3.43 - 3.56 (m, 1 H), 3.80 (d, *J*=10.13 Hz, 1 H), 3.97 (dd, *J*=10.59, 5.37 Hz, 1 H), 4.45 (t, *J*=9.67 Hz, 1 H), 4.53 (d, *J*=12.89 Hz, 1 H),5.23 (t, *J*=5.40 Hz, 1 H), 6.98 (s, 1 H), 7.15 (d, *J*=7.80 Hz, 4H), 7.34 - 7.44 (m, 2 H), 7.63 (dt, *J*=8.90, 2.30 Hz, 1H), 7.72 (dd, *J*=6.40, 2.8 Hz, 1 H), 8.34 (d, *J*=8.0 Hz, 4H), 8.52 (d, *J*=2.50 Hz, 1H), 8.91 (d, *J*=1.50 Hz, 1 H),12.38 (s, 1 H); MS (DCI/NH₃) m/z 305 (M+1)⁺. Anal. calcd. for C₁₉H₂₀N₄·2.25TsOH·1.20H₂O: C, 57.96; H, 5.53; N, 8.01. Found: C, 58.14; H, 5.20; N, 7.63.

### Example 5

### 6-{5-[(1S,5S)-3,6-diazabicyclo[3.2.0]heptan-3-yl]pyridin-3-yl}-1H-indole bistosylate

### Example_5A

### (1R,5S)-tert-butyl 3-[5-(1H-indol-6-yl)pyridin-3-yl]-3,6-diazabicyclo[3.2.0]heptane-6-carhoxylate

The product of Example 1A (240 mg, 0.68 mmol) was coupled with *1H*-indol-4-ylboronic acid (Frontier, 164 mg, 1.02 mmol) according to the procedure of Example 3A. The title compound was purified using chromatography (SiO₂, EtOAc/hexane, v. 50/50, R_{f}=0.40) (240 mg, yield, 91%). ¹H NMR (300 MHz, CD₃OD) δ ppm 1.46 [s (br.), 9 H], 2.96 (dd, *J*=10.85, 4.07 Hz, 1 H), 3.06 (dd, *J=1*0.17, 6.44 Hz, 1 H), 3.21 - 3.30 (m, 1 H), 3.54 - 3.75 (m, 1 H), 3.88 (d, *J*=10.17 Hz, 1 H), 3.96 - 4.20 (m, 2 H), 4.85 - 4.90 (m, 1 H), 6.48 (d, *J*=3.05 Hz, 1 H), 7.26 - 7.36 (m, 2 H), 7.39 - 7.47 (m, 1 H), 7.61 - 7.69 (m, 2 H), 8.03 (d, *J*=2.37 Hz, 1 H), 8.22 (d, *J*=1.70 Hz, 1 H); MS (DCI/NH₃) m/z 391(M+1)⁺.

### Example_5B

### 6-{5-[(1S,5S)-3,6-diazabicyclo[3.2.0]heptan-3-yl]pyridin-3-yl}-1H-indole bistosylate

The product of Example 5A (240 mg, 0.61 mmol) was treated with *p*-TsOH·H₂O (257 mg, 1.35 mmol) in EtOAc (20 mL) at 80°C for 4h, then ambient temperature overnight to give the title compound (230 mg, yield, 59.4%). ¹H NMR (400 MHz, Pyridine-D₅) δ ppm 2.17 (s, 6 H), 2.91 (dd, *J*=10.40, 6.20 Hz, 1 H), 2.98 (dd, *J*=12.20, 4.90 Hz, 1 H), 3.35 - 3.51 (m, 1 H), 3.82 - 3.92 (m, 2H), 4.40 (d, *J*=12.27 Hz, 1 H), 4.58 (dd, *J*=10.74, 8.59 Hz, 1 H), 5.47 (dd, *J*=7.06, 4.91 Hz, 1 H), 6.75 - 6.83 (m, 1 H), 7.17 (d, *J*=7.67 Hz, 4 H), 7.46 - 7.51 (m, 1 H), 7.56 (dd, *J*=8.13, 1.69 Hz, 1 H), 7.60 - 7.62 (m, 1 H), 7.92 (d, *J*=8.29 Hz, 1 H), 8.03 (s, 1 H), 8.36 (d, *J*=7.98 Hz, 4 H), 8.41 (d, *J*=2.76 Hz, 1 H), 8.79 (d, *J*=1.84 Hz, 1 H), 12.26 (s, 1 H); MS (DCI/NH₃) m/z 291 (M+1)⁷⁺. Anal. calcd. for C₁₈H₁₈N₄·2.25TsOH·1.20H₂O: C, 57.96; H, 5.53; N, 8.01. Found: C, 58.14; H, 5.20; N, 7.63.

### Example 6

### 6-{5-[(1S,5S)-6-methyl-3,6-diazabicyclo[3.2.0]heptan-3-yl]pyridin-3-yl}-1H-indole tosylate

### Example 6A

### 6-{5-[(1S,5S)-6-methyl-3,6-diazabicyclo[3.2.0]heptan-3-yl]pyridin-3-yl}-1H-indole

The product of Example 5B (200 mg, 0.32 mmol) was treated with formaldehyde (Aldrich, 37%, 33 µL, 0.40 mmol) according to the procedure of Example 2A. The title compound was purified by preparative HPLC (Gilson, Xterra® column, 7 µm, 40 x 100 mm, eluting solvent, MeCN /H₂O (with 0.1 M NH₄HCO₃/NH₄OH, PH=10) (v. 90/10 to 10/90 over 25 minutes), flow rate, 40 mL/min., uv, 254 nm) (50 mg, yield, 51.8%). ¹H NMR (300 MHz, CD₃OD) δ ppm 2.39 (s, 3 H) 3.01 (dd, *J*=11.02, 4.58 Hz, 1 H), 3.17 - 3.29 (m, 3 H), 3.30-3.37 (m, 1H), 3.73 - 3.87 (m, 2 H), 4.01 (dd, *J*=6.10, 4.41 Hz, 1 H), 4.26 (s, 1H), 7.08 (s, 1 H), 7.22 - 7.29 (m, 1 H), 7.34 - 7.39 (m, 1 H), 7.57 - 7.67 (m, 2 H), 7.96 (d, *J*=2.37 Hz, 1 H), 8.16 (s, 1 H); MS (DCI/NH₃) m/z 305(M+1)⁺.

### Examples 6B

### 6-{5-[(1S,5S)-6-methyl-3,6-diazabicyclo[3.2.0]heptan-3-yl]pyridin-3-yl}-1H-indole tosylate

The product of Example 6A (50 mg, 0.16 mmol) was treated with p-TsOH·H₂O (Aldrich, 62 mg, 0.33 mmol) in EtOAc/EtOH (v. 10/1, 5 mL) at ambient temperature overnight to give the title compound (55 mg, yield, 74.4%). ¹H NMR (300 MHz, CD₃OD) δ ppm ¹H NMR (400 MHz, Pyridine-D₅) δ ppm 2.14 (s, 4.2 H) 2.87 (s, 3 H), 2.91 (dd, *J*=10.28, 6.60 Hz, 1 H), 2.98 (dd, *J*=12.43, 4.76 Hz, 1 H), 3.26 - 3.39 (m, 1 H), 3.73 (d, *J*=10.13 Hz, 1 H), 3.78 (dd, *J*=10.13, 4.60 Hz, 1 H), 4.11 - 4.21 (m, 1 H), 4.36 (d, *J*=12.27 Hz, 1 H), 4.61 (s, 1H), 4.84 - 4.97 (m, *J*=6.14 Hz, 1 H), 7.13 (d, *J*=7.90 Hz, 2.8H), 7.50 (t, *J*=2.30 Hz, 1H), 7.54-7.58 (m, 2H), 8.03 - 8.09 (m, 3 H), 8.34 (d, *J*=7.98 Hz, 2.8 H), 8.83 (d, *J*=1.90 Hz, 1H), 12.05 (s, 1 H); MS (DCI/NH₃) m/z 305 (M+1)⁺. Anal. calcd. for C₁₉H₂₀N₄·1.42TsOH·1.00H₂O: C, 61.31; H, 5.93; N, 9.88. Found; C, 61.29; H, 6.11; N, 9.53.

### Example 7

### 5-{5-[(1S,5S)-6-methyl-3,6-diazabicyclo[3.2.0]heptan-3-yl]pyridin-3-yl}-2-(trifluoromethyl)-1H-indole bistrifluoroacetate

### Example 7A

### (1S,5S)-3-(5-bromopyridin-3-yl)-6-methyl-3,6-diazabicyclo[3.2.0]heptane

The product of Example 1A (4.50 g, 12.7 mmol) was treated with HCHO (Aldrich, 37%, 5 mL) in HCO₂ (Aldrich, 88%, 30 mL) at 90 °C for 1 h. The solution was then concentrated under reduced pressure. The residue was carefully basified with saturated Na₂CO₃ to pH9. The mixture was extracted with CHCl₃ (3 x 100 mL). The combined extracts were washed with brine (2 x 20 mL) and concentrated. The residue was purified with with chromatography (SiO₂ CH₂Cl₂/MeOH (with 2 v.% NH₃·H₂O), 90/10, R_{f}=0.10) to give the title compound (3.36 g, yield, 99%). ¹H NMR (300 MHz, CD₃OD) δ ppm 2.89 (s, 3 H), 3.09 (dd, *J*=10.51, 6.44 Hz, 1 H), 3.15 (dd, *J*=12.89, 4.75 Hz, 1 H), 3.41 - 3.60 (m, 1 H), 3.85 - 3.98 (m, 2 H), 4.09 - 4.25 (m, 2 H), 4.87 - 4.97 (m, 1 H), 7.55 (t, *J*=2.20 Hz, 1 H), 8.09 (d, *J*=1,70 Hz, 1 H), 8.17 (d, *J*=2.37 Hz, 1 H); MS (DCI/NH₃) m/z 268 (M+1)⁺, 270 (M+1)⁺.

### Example 7B

### 5-{5-[(1S,5S)-6-methyl-3,6-diazabicyclo[3.2.0]heptan-3-yl]pyridin-3-yl}-2-(trifluoromethyl)-1H-indole bistrifluoroacetate

The product of Example 7A (100 mg, 0.37 mmol) was coupled with 5-(4,4,5,5-tetramethyl-1,3,2-dioxaborolan-2-yl)-2-(trifluoromethyl)-1H-indole (US 2005043347, 300 mg, 0.965 mmol) under the catalysis of bis(triphenylphosphine)palladium(II) chloride (Aldrich, 7.0 mg, 0.01 mmol,) and biphenyl-2-yl-dicyclohexyl-phosphane (Strem Chemicals, 10.5 mg, 0.03 mmol) in dioxane/EtOH/Na₂CO₃ (aq., 1 M) (v. 1/1/1 3 ml) at 130 °C (150 watts max.) for 15 min in an Emry^{™} Creator microwave. The inorganic solid was filtered off via a syringe filter, and the liquid mixture was purified by preparative HPLC (Gilson, column, Xterra^{®} 5 µm, 40 x 100 mm; eluting solvent, MeCN /H₂O (with 0.1% v. TFA) (v. 90/10 to 10/90 over 25 min.), flow rate, 40 mL/min., uv, 254 nm). Fractions of the desired product were collected and concentrated, and the residue was stirred in ether/ethanol (v. 10/1, 5 mL) at room temperature for 16 h to give the title compound (56.1 mg, yield, 22.8%). ¹H NMR (300 MHz, CD₃OD δ ppm 2.74 - 3.14 (m, 4 H), 3.41 (dd, *J*=13.2, 4.7 Hz, 1 H), 3.56 - 3.68 (m, 1 H), 4.02 - 4.27 (m, 3 H), 4.39 (d, *J*=12.9 Hz, 1 H), 5.92-5.05 (m, 1 H), 7.03 (s, 1 H), 7.60 - 7.73 (m, 2 H), 8.05 (s, 1 H), 8.11 (s, 1 H), 8.26 (s, 1 H), 8.51 (s, 1 H); MS (DCI/NH₃) m/z 373(M+1)⁺; Anal. calcd. for C₂₀H₁₉F₃N_{4·}2.5C₂F₃O₂H: C, 45.67; H, 3.30; N, 8.52. Found: C, 45.62; H, 3.31; N, 8.47.

### Example 8

### (1S,5S)-3-(5-(benzofuran-5-yl)pyridin-3-yl)-6-methyl-3,6-diazabicyclo[3.2.0]heptane bistrifluoroacetate

The product of Example 7A (100 mg, 0.37 mmol) was coupled with benzofuran-5-ylboronic acid (Maybridge, 150 mg, 0.93 mmol) according to the procedure of Example 7B to give the title compound (156.7 mg, yield, 76.7%). ¹H NMR (300 MHz, CD₃OD) δ ppm 2.74 - 3.13 (m, 4 H), 3.39 (dd, *J*=13.1, 4.9 Hz, 1 H), 3.55 - 3.66 (m, 1 H), 4.03 - 4.24 (m, 3 H), 4.40 (d, *J*=12.9 Hz, 1 H), 4.96 - 5.04 (m, 1 H), 6.97 (d, *J*=2.0 Hz, 1 H), 7.70 (s, 2 H), 7.88 (d, *J*=2.0 Hz, 1 H), 7.99 - 8.08 (m, 2 H), 8.29 (s, 1 H), 8.50 (s, 1 H); MS (DCI/NH₃) m/z 306 (M+1)⁺. Anal. calcd. for C₁₉H₁₉N₃O·2.1 CF₃CO₂H: C, 51.15; H, 3.90; N, 7.71. Found: C, 51.14; H, 3.47; N, 7.70.

### Example 9

### 5-{5-[1S,5S)-6-methyl-3,6-diazabicyclo[3.2.0]heptan-3-yl]pyridin-3-yl}-1H-indazole bistrifluoroacetate

### Examples 9A

### 5-(4,4,5,5-tetramethyl-1,3,2-dioxaborolan-2-yl)-1H-indazole

5-bromo-1*H*-indazole (US2003199511, 9.45 g, 48 mmol,) was coupled with bis(pinacolato)diboron (Combi-Blocks, 15.5g, 61 mmol) under the catalysis of Pd(dppf)₂Cl₂•CH₂Cl₂ (Aldrich, 985 mg, 1.2 mmol) in the presence of KOAc (Aldrich, 16.7 g, 170 mmol) in dry DMF (160 ml) at 90°C for 24 hours, After the reaction was completed, it was cooled to ambient temperature, diluted with EtOAc (250 mL) and washed with water (2 x 50 mL). The organic phase was concentrated under reduced pressure, and the residue was purified with chromatography (SiO₂, Hexane:EtOAc (v. 10:1), R_{f}=0.6) to give the title compound (9.8 g, yield, 84%). ¹H NMR (300 MHz, CD₃OD) δ ppm 1.36 (s, 12 H) 7.51 (dt, *J*=8.5, 1.0 Hz, 1 H) 7.73 (dd, *J*=8.5, 1.0 Hz, 1 H) 8.08 (d, *J*=1.0 Hz, 1 H) 8.23 (t, *J*=1.0 Hz, 1 H); MS (DCI/NH₃) m/z 245 (M+1)⁺.

### Example 9B

### 5-{5-[(1S,5S)-6-methyl-3,6-diazabicyclo[3.2.0]heptan-3-yl]pyridin-3-yl}-1H-indazole bistrifluoroacetate

The product of Example 7A (100 mg, 0.37 mmol) was coupled with the product of Example 9A (200 mg, 0.82 mmol) according to the procedure of Example 7B to give the title compound (128.2 mg, 0.235 mmol, yield, 62.7%). ¹H NMR (300 MHz, CD₃OD) δ ppm 2.75 - 3.12 (m, 4 H), 3.38 (dd, *J*=13.1, 4.9 Hz, 1 H), 3.55-3.66 (m, 1 H), 4.05 - 4.23 (m, 3 H), 4.38 (d, *J*=12.2 Hz, 1 H), 4.96 - 5.06 (m, 1 H), 7.69 - 7.83 (m, 2 H), 8.00 (s, 1 H), 8.18 (d, *J*=1.0 Hz, 1 H), 8.21 (s, 1 H), 8.29 (m, 1 H), 8.51 (s, 1 H); MS (DCI/NH₃) m/z 306 (M+1)⁺. Anal. calcd. for C₁₈H₁₉N₅ 2.10 CF₃CO₂H: C, 48.94; H, 3.90; N, 12.85. Found: C, 48.90; H, 3.73; N, 12.82.

### Example 10

### (1S,5S)-3-[5-(benzo[b]thiophen-5-yl)pyridin-3-yl]-3,6-diazabicyclo[3.2.0]heptane bistosylate

### Example 10A

### 5-[(1R,5S)-6-(tert-butoxycarbonyl)-3,6-diazabicyclo[3.2.0]heptan-3-yl]pyridin-3-ylboronic acid

The product of Example 1A (0.71 g, 2 mmol) was coupled with 4,4,4',4',5,5,5',5'-octamethyl-2,2'-bi(1,3,2-dioxaborolane) (Aldrich, 0.76 g, 3.0 mmol) under the catalysis of {1,1'-bis(diphenylphosphino)-ferrocene]dichloropalladium(II) dichloromethane complex PdCl₂(dppf)·CH₂Cl₂ (Aldrich, 33 mg, 0.04 mmol) with KOAc (Aldrich, 392 mg, 4.0 mmol) in dioxane (anhydrous, 20 mL) at 80 °C for 15h. The solution was then cooled to ambient temperature and diluted with EtOAc (100 mL). The mixture was then washed with brine (2 x 10 mL). The organic solution was concentrated to give the title compound (0.62 g, yield, 96.9%). ¹H NMR (300 MHz, CD₃OD) δ ppm 1.41 - 1.49 (m, 9 H), 2.98 (dd, *J*=10.85, 4.07 Hz, 1 H), 3.07 (dd, *J*=10.25, 6.40 Hz, , 1 H), 3.20 - 3.32 (m, 3 H), 3.52 - 3.67 (m, 1 H), 3.83 (d, .*J*=10.51 Hz, 1 H), 3.99 (d, *J*=10.85 Hz, 1 H), 4.10 (t, *J=*7.80 Hz, 1 H), 7.77 (d, *J*=2.03 Hz, 1 H), 7.90 (d, *J*=2.37 Hz, 1 H), 7.98 (s, 1 H); MS (DCI/NH₃) m/z 320 (M+1)⁺.

### Example 10B

### 5-[(1S,5S)-3,6-diazabicylo[3.2.0]heptan-3-yl]pyridin-3-yl boronic acid bistosylate

The product of example 10A (0.62 g, 1.94 mmol) was treated with p-TsOH·H₂O (0.95 g, 5.0 mmol) in EtOH (20 mL) at 80°C for 4 h and cooled to ambient temperature. EtOAc (20 mL) was added. The mixture was stirred at room temperature overnight to give the title compound (1.10 g, Yield, 97.5%). ¹H NMR (300 MHz, CD₃OD) δ ppm 2.36 (s, 6 H), 3.26 (dd, *J*=10.50, 6.10 Hz, 1 H), 3.37 (dd, *J*=12.72, 5.26 Hz, 1 H), 3.52 - 3.66 (m, 1 H), 3.78 (dd, *J*=11.19, 5.09 Hz, 1 H), 4.03 (d, *J*=10.85 Hz, 1 H), 4.24 - 4.37 (m, 2 H), 5.13 (dd, *J*=6.95, 5.26 Hz, 1 H), 7.21 (d, *J*=7.80 Hz, 4 H), 7.68 (d, *J*=8.48 Hz, 4 H), 8.13 (d, *J*=2.37 Hz, 1 H), 8.26 (s, 1 H), 8.30 (d, *J*=3.05 Hz, 1 H); MS (DCI/NH₃) m/z 220 (M+1)⁺.

### Example 10C

### (1S,5S)-3-[5-(benzo[b]thiophen-5-yl)pyridin-3-yl]-3,6-diazabicyclo[3.2.0]heptane bistosylate

The product of Example 10B (280 mg, 0.50 mmol) was coupled with 5-bromobenzo[b]thiophene (Alfa Aesar, 160 mg, 1.5 mmol) according to the procedure of Example 3A. The free base of the title compound was purified with preparative HPLC (Gilson, Xterra® column, 7 µm, 40 x 100 mm, eluting solvent, MeCN /H₂O (with 0.1 M NH₄HCO₃/NH₄OH, PH=10) (v. 90/10 to 10/90 over 25 minutes), flow rate, 40 mL/mm., uv, 254 nm) (40 mg, yield, 26%). The free base (40 mg, 0.13 mmol) was then treated with TsOH·H2O (57 mg, 0.3 mmol) in EtOAc (5.0 mL) at abient temperature overnight to give the title compound (50 mg, yield, 59.4%). ¹H NMR (400 MHz, CD₃OD) δ ppm 2.35 (s, 6 H), 3.31 - 3.36 (m, 1 H), 3.42 (dd, *J*=12.72, 5.26 Hz, 1 H), 3.54 - 3.69 (m, 1 H), 3.82 (dd, *J*=11.02, 5.26 Hz, 1 H), 4.12 (d, *J*=10.85 Hz, 1 H), 4.27 - 4.33 (m, 1 H), 4.37 (d, *J*=12.55 Hz, 1 H), 5.15 (dd, *J*=6.78, 5.43 Hz, 1 H),7.19 (d, *J*=7.80 Hz, 4 H), 7.51 (d, *J*=5.43 Hz, 1 H), 7.68 (d, *J*=8.14 Hz, 4 H), 7.71 - 7.77 (m, 2 H), 8.07 - 8.15 (m, 2 H), 8.28 (d, *J*=1.70 Hz, 1 H), 8.30 (d, *J*=2.71 Hz, 1 H), 8.52 (d, *J*=1.36 Hz, 1 H); MS (DCI/NH₃) m/z 308 (M+1)⁺. Anal. calcd. for C₁₈H₁₇N₃S 2.05TsOH·1.60H₂O: C, 56.37; H, 5.35; N, 6.10. Found: C, 56.03; H, 5.63; N, 6.10.

### Example 11

### (1S,5S)-3-[5-(benzo[b]thiophen-5-yl)pyridin-3-yl]-3,6-diazabicyclo[3.2.0]heptane bistosylate

### Example 11A

### 5-[(1S,5S)-6-methyl-3,6-diazabicyclo[3.2.0]heptan-3-yl]pyridin-3-ylboronic acid ylboronic acid

The product of Example 7A (2.50 g, 9.3 mmol) was coupled with 4,4,4',4',5,5,5',5'-octamethyl-2,2'-bi(1,3,2-dioxaborolane) (Aldrich, 2.53 g, 10.0 mmol) under the catalysis of {1,1'-bis(diphenylphosphino)-ferrocene]dichloropalladium(II) dichloromethane complex PdCl₂(dppf)•CH₂Cl₂ (Aldrich, 163 mg, 0.2 mmol) with KOAc (Aldrich, 1.96g, 20.0 mmol) in dioxane (anhydrous, 50 mL) at 80 °C for 15 h. The solution was then cooled to ambient temperature, concentrated and diluted with water (20 mL). The mixture was then extracted with dithyl ether (2 x 10 mL). The aquesous solution was concentrated to give the title compound. ¹H NMR (300 MHz, CD₃OD) δ ppm 2.95 (dd, *J*=10.85, 4.41 Hz, 1 H), 3.12 - 3.27 (m, 3 H), 3.31 - 3.39 (m, 1 H), 3.68 - 3.79 (m, 2 H), 4.00 (dd, *J*=6.44, 4.41 Hz, 1 H), 7.24 (d, *J*=4.75 Hz, 1 H), 7.87 (dd, *J*=4.41, 1.36 Hz, 1 H), 8.03 (d, *J*=2.71 Hz, 1 H); MS (DCI/NH₃) m/z 234 (M+1)⁺.

### Example 11B

### (1S,5S)-3-[5-(benzo[b]thiophen-5-yl)pyridin-3-yl]-3,6-diazabicyclo[3.2.0]heptane bistosylate

The product of Example 11A (117 mg, 0.50 mmol) was coupled with 5-bromobenzo[b]thiophene (Alfa Aesar, 160 mg, 0.75 mmol) according to the procedure of Example 10C to give the title compound (60 mg, yield, 18%). ¹H NMR (400 MHz, Pyridine-D₅) δ ppm 2.14 (s, 6 H), 2.95 (dd, *J*=10.13, 6.14 Hz, 1 H), 3.09 (s, 3 H), 3.13 (dd, *J*=12.89, 4.91 Hz, 1 H), 3.48 - 3.62 (m, 1 H), 3.86 (d, *J*=10.13 Hz, 1 H), 3.99 - 4.13 (m, 1 H), 4.41 - 4.38 (m, 1 H), 4.68 (d, *J*=12.89 Hz, 1 H), 5.32 - 5.49 (m, 1 H), 7.13 (d, *J*=7.98 Hz, 4 H), 7.50 (d, *J*=5.22 Hz, 1 H), 7.61 (t, *J*=2.15 Hz, 1 H), 7.69 (d, *J*=5.22 Hz, 1 H), 7.73 (dd, *J*=8.29, 1.53 Hz, 1 H), 8.05 (d, *J*=8.59 Hz, 1 H), 8.27 (d, *J*=1.53 Hz, 1 H), 8.33 (d, *J*=7.98 Hz, 4 H), 8.49 (d, *J*=2.45 Hz, 1 H), 8.74 (d, *J*=1.53 Hz, 1 H);

MS (DCI/NH₃) m/z 322 (M+1)⁺. Anal. calcd. for C₁₉H₁₉N₃S·2.00TsOH·1.20H₂O: C, 57.65; H, 5.48; N, 6.11. Found: C, 57.51; H, 5.24; N, 5.83.

### Example 12

### 7-{5-[(1S,5S)-6-methyl-3,6-diazabicyclo[3.2.0]heptan-3-yl]pyridin-3-yl}-1H-indole tritosylate

### Example 12A

### 7-{5-[(1S,5S)-6-methyl-3,6-diazabicyclo[3.2.0]heptan-3-yl]pyridin-3-yl}-1H-indole

The product of Example 11A (117 mg, 0.50 mmol) was coupled with 7-bromoindole (Aldrich, 147 mg, 0.75 mmol) according to the procedure of Example 3A. The title compound was purified by preparative HPLC (Gilson, Xterra® column, 7 µm, 40 x 100 mm, eluting solvent, MeCN /H₂O (with 0.1 M NH₄HCO₃/NH₄OH, PH=10) (v. 90/10 to 10/90 over 25 minutes), flow rate, 40 mL/min., uv, 254 nm) (40 mg, yield, 26%). ¹H NMR (300 MHz, CD₃OD) δ ppm 2.42 (s, 3 H), 3.06 (dd, *J*=11.02, 4.24 Hz, 1 H), 3.20 - 3.28 (m, 2 H), 3.33 - 3.41 (m, 2 H), 3.80 (t, *J*=10.51 Hz, 2 H), 4.03 (dd, *J*=6.27, 4.24 Hz, 1 H), 6.53 (d, *J*=3.05 Hz, 1 H), 7.06 - 7.18 (m, 2 H), 7.26 (d, *J*=3.39 Hz, 1 H), 7.34 - 7.42 (m, 1 H), 7.59 (dd, *J*=6.61, 2.20 Hz, 1 H), 8.07 (d, *J*=2.37 Hz, 1 H), 8.13 (d, *J*=1.36 Hz, 1 H); MS (DCI/NH₃) m/z 305 (M+1)⁺.

### Example 12B

### 7-{5-[(1S,5S)-6-methyl-3,6-diazabicyclo[3.2.0]heptan-3-yl]pyridin-3-yl}-1H-indole tritosylate

The product of Example 12A (40 mg, 0.13 mmol) was treated with *p*-TsOH·H₂O (74 mg, 0.39 mmol) in EtOAc (5 mL) at ambient temperature overnight to give the title compound (35 mg, yield, 32.8%). ¹H NMR (400 MHz, Pyridine-D₅) δ ppm 2.16 (s, 9 H), 2.85 (dd, *J*=10.13, 6.14 Hz, 1 H), 2.92 - 3.06 (m, 4 H), 3.37 - 3.51 (m, 1 H), 3.81 (d, *J*=10.13 Hz, 1 H), 3.97 - 4.05 (m, 1 H), 4.28 (t, *J*=9.36 Hz, 1 H), 4.67 (d, *J*=12.89 Hz, 1 H), 5.05 - 5.19 (m, 1 H), 6.61 - 6.87 (m, 2 H), 7.15 (d, *J*=7.67 Hz, 6 H), 7.32 (t, *J*=7.52 Hz, 1 H), 7.36 - 7.42 (m, 1 H), 7.64 - 7.77 (m, 2 H), 7.87 (d, *J*=7.98 Hz, 1 H), 8.30 (d, *J*=7.98 Hz, 6 H), 8.42 (d, *J*=2.46 Hz, 1 H), 12.00 (s, 1 H); MS (DCI/NH₃) m/z 305 (M+1)⁺. Anal. calcd. for C₁₉H₂₀N₄·3.00TsOH·1.00H₂O: C, 56.64; H, 5.61; N, 6.77. Found: C, 56.70; H, 5.34; N, 6.97.

### Example 13

### 5-{5-[(1S,5S)-6-methyl-3,6-diazabicyclo[3.2.0]heptan-3-yl]pyridin-3-yl}-1H-benzo[d]imidazole bistosylate

### Example 13A

### 5-{5-[(1S,5S)-6-methyl-3,6-diazabicyclo[3.2.0]heptan-3-yl]pyridin-3-yl}-1H-benzo[d]imidazole

The product of Example 11A (117 mg, 0.50 mmol) was coupled with 7-bromoindole (Aldrich, 147 mg, 0.75 mmol) according to the procedure of Example 12A to give the title compound (80 mg, yield, 52%). ¹H NMR (300 MHz, CD₃OD) δ ppm 2.41 (s, 3 H), 3.06 (dd, *J*=11.19, 4.41 Hz, 1 H), 3.22 - 3.28 (m, 2 H), 3.33 - 3.39 (m, 2 H), 4.03 - 4.06 (m, 1 H), 7.37 - 7.42 (m, 1 H), 7.58 (dd, *J*=8.48, 1.70 Hz, 1 H), 7.72 (d, *J*=8.48 Hz, 1 H), 7.87 (s, 1 H), 8.03 (d, *J*=2.71 Hz, 1 H), 8.18 (d, *J*=1.70 Hz, 1 H), 8.23 (s, 1 H); MS (DCI/NH₃) m/z 306 (M+1)⁺.

### Example 13B

### 5-{5-[(1S,5S)-6-methyl-3,6-diazabicyclo[3.2.0]heptan-3-yl]pyridin-3-yl}-1H-benzo[d]imidazole bistosylate

The product of Example 13A (80 mg, 0.26 mmol) was treated with *p*-TsOH·H₂O (114 mg, 0.60 mmol) in EtOAc (5 mL) at ambient temperature overnight to give the title compound (100 mg, yield, 47.5%). ¹H NMR (400 MHz, Pyridine-D₅) δ ppm 2.16 (s, 6 H), 2.91 (dd, *J*=10.43, 6.14 Hz, 1 H), 3.03 - 3.12 (m, 4 H), 3.42 - 3.57 (m, 1 H), 3.82 (d, *J*=10.13 Hz, 1 H), 4.01 (dd, *J*=10.43, 4.91 Hz, 1 H), 4.33 - 4.51 (m, *J*=9.21 Hz, 1 H), 4.62 (d, *J*=12.89 Hz, 1 H), 5.15 - 5.30 (m, 1 H), 7.15 (d, *J*=7.67 Hz, 4 H), 7.71 (dd, *J*=8.29, 1.53 Hz, 1 H), 7.84 - 7.95 (m, 1 H), 8.01 (d, *J*=8.29 Hz, 1 H), 8.29 - 8.37 (m, *J*=7.98 Hz, 5 H), 8.47 (d, *J*=2.45 Hz, 1 H), 8.67 (s, 1 H), 8.78 (d, *J*=1.53 Hz, 1 H); MS (DCI/NH₃) m/z 306 (M+1)⁺. Anal. calcd. for C₁₈H₁₉N₅·2.00TsOH·1.50H₂O: C, 56.79; H, 5.66; N, 10.35. Found: C, 56.58; H, 5.26; N,10.63.

### Example 14

### 3-methyl-5-{5-[(1S,5S)-6-methyl-3,6-diazabicyclo[3.2.0]heptan-3-yl]pyridin-3-yl}-1H-indole bistosylate

### Example 14A

### 3-methyl-5-{5-[(1S,5S)-6-methyl-3,6-diazabicyclo[3.2.0]heptan-3-yl]pyridin-3-yl}-1H-indole

The product of Example 11A (117 mg, 0.5 mmol) was coupled with 5-bromo-3-methyl-1H-indole (Aldrich, 210 mg, 1.0 mmol) according to the procedure of Example 7B. After the reaction was completed, the inorganic solid was filtered off via a syringe filter, and the filtrate was purified by preparative HPLC (Gilson, column, Xterra® 7 µm, 40 x 100 mm. eluting solvent, MeCN/H₂O (with 0.1M NH₄HCO₃/NH₄OH, pH=10) (v. 90/10 to 10/90 over 25 min.); flow rate, 40 mL/min., uv, 254 nm) to give the free base of the title compound (15 mg, yield, 9.4%), ¹H NMR (300 MHz, CD₃OD) δ ppm 2.35 (s, 3 H), 2.39 (s, 3 H), 3.03 (dd, *J*=11.19, 4.41 Hz, 1 H), 3.20 - 3.29 (m, 2 H), 3.31 - 3.39 (m, 2 H), 3.77 - 3.90 (m, 2 H), 4.03 (dd, *J*=6.27, 4.24 Hz, 2 H), 7.04 (s, 1 H), 7.34 - 7.46 (m, 3 H), 7.74 (s, 1 H), 7.97 (d, *J*=2.71 Hz, 2 H), 8.17 (d, *J*=1.70 Hz, 1 H); MS (DCI/NH₃) m/z 319 (M+1)⁺.

### Example 14B

### 3-methyl-5-{5-[(1S,5S)-6-methyl-3,6-diazabicyclo[3.2.0]heptan-3-yl]pyridin-3-yl}-1H-indole bistosylate

The product of Example 14A (15 mg, 0.047 mmol) was treated with *p*-TsOH (Aldrich, 19mg, 0.1 mmol) in EtOAc/EtOH(v. 5/1, 5 mL) at room temperaturefor 16 hours to give the title compound (20 mg,yield, 64.3%). ¹H NMR (300 MHz, CD₃OD) δ ppm 2.33 (s, 6 H), 2.38 (s, 3 H), 3.04 (s, 3 H), 3.35 - 3.49 (m, 2 H), 3.57 - 3.71 (m, 1 H), 4.05 - 4.24 (m, 3 H), 4.45 (d, *J*=12.9 Hz, 1 H), 5.02 (dd, *J*=7.1, 4.7 Hz, 1 H), 7.13 (d, *J*=1.02 Hz, 1 H), 7.18 (d, *J*=7.8 Hz, 4 H), 7.49 - 7.55 (m, 2 H), 7.67 (d, *J*=8.1 Hz, 4 H), 7.94 (t, *J*=1.36 Hz, 1H), 8.12 - 8.33 (m, 2 H), 8.51 (d, *J*=1.02 Hz, 1H); MS (DCI/NH₃) m/z 319 (M+1)⁺. Anal. calcd. for C₂₀H₂₂N₄·2.30C₇H₈O₃S·1.30H₂O: C, 58.76; H, 5.87; N, 7.59. Found: C, 58.97; H, 5.52; N, 7.71.

### Example 15

### 3-{5-[(1S,5S)-3,6-diazabicyclo[3.2.0]heptan-3-yl]pyridin-3-yl}-9H-carbazole hemifumarate

The product of Example 10B (109 mg, 0.5 mmol) was coupled with 3-bromo-9*H-*carbazole (Aldrich, 245 mg, 1.0 mmol) according to the procedure of Example 14A to give the free base of the title compound (68 mg, yield, 40%). The free base was then treated with fumaric acid (Aldrich, 23 mg, 0.2 mmol) in EtOAc/EtOH(v. 5/1, 5 mL) at room temperature for 16 hours to give the title compound (64 mg, yield, 72%). ¹H NMR (300 MHz, CD₃OD) δ ppm 3.12 (dd, *J*=10.3, 6.3 Hz, 1 H), 3.21 (dd, *J*=12.2, 5.1 Hz, 1 H), 3.48 - 3.60 (m, 1 H), 3.78 (dd, *J*=11.2, 5.1 Hz, 1 H), 4.05 (d, *J*=10.5 Hz, 1 H), 4.22 - 4.33 (m, 2 H), 5.06 (dd, *J*=7.1, 4.7 Hz, 1 H), 6.68 (s, 1.8 H), 7.19 (ddd, *J*=7.9, 6.9, 1.2 Hz, 1 H), 7.40 (td, *J*=7.5, 1.2 Hz, 1 H), 7.45 - 7.51 (m, 1 H), 7.54 - 7.60 (m, 1 H), 7.64 - 7.67 (m, 1 H), 7.67 - 7.72 (m, 1 H), 8.12 - 8.19 (m, 2 H), 8.38 (dd, *J*=9.7, 1.5 Hz, 2 H); MS (DCI/NH₃) m/z 341 (M+1)⁺. Anal. calcd. for C₂₂H₂₀N₄ 0.90C₄H₄O₄: C, 69.11; H, 5.35; N, 12.59. Found: C, 68.93; H, 5.40; N, 12.74.

### Example 16

### 5-{5-[(1S,5S)-3,6-diazabicyclo[3.2.0]heptan-3-yl]pyridin-3-yl}-3-methyl-1H-indole bistrifluoroacetate

The product of Example 10B (109 mg, 0.5 mmol) was coupled with 5-bromo-3-methyl-1H-indole (Aldrich, 210 mg, 1.0 mmol) according to the procedure described in Example 7B to give the title compound (90 mg, yield, 30.6%). ¹H NMR (300 MHz, CD₃OD) δ ppm 2.38 (d, *J=*1.4 Hz, 3 H), 3.28 - 3.36 (m, 1 H), 3.42 (dd, *J*=12.7, 5.3 Hz, 1 H), 3.57 - 3.66 (m, 1 H), 3.81 (dd, *J*=11.4, 5.3 Hz, 1 H), 4.13 (d, *J*=10.8 Hz, 1 H), 4.28 - 4.39 (m, 2 H), 5.15 (dd, *J*=7.1, 5.1 Hz, 1 H), 7.12 (q, *J*=1.0 Hz, 1 H), 7.50 (d, *J*=1.4 Hz, 2 H), 7.93 (t, *J*=1.4 Hz, 1 H), 8.11 (dd, *J*=2.4, 1.7 Hz, 1 H), 8.23 (d, *J*=2.7 Hz, 1 H), 8.52 (d, *J*=1.4 Hz, 1 H),; MS (DCI/NH₃) m/z 305 (M+1)⁺. Anal. calcd. for C₁₉H₂₀N₄ 2.50CF₃CO₂H: C, 48.90; H, 3.85; N, 9.50. Found: C, 49.13; H, 4.00; N, 9.50.

### Example 17

### 3-(5-((1S,5S)-6-methyl-3,6-diazabicyclo[3.2.0]heptan-3-yl)pyridin-3-yl)-9H-carbazole bistrifluoroacetate

The product of Example 11A (117 mg, 0.5 mmol) was coupled with 3-bromo-9*H-*carbazole (Aldrich, 245 mg, 1.0 mmol) according to the procedure of Example 7B to give the title compound (24.8 mg, yield, 7.3%). ¹H NMR (300 MHz, CD₃OD) δ ppm 2.77 - 3.12 (m, 4 H), 3.43 (dd, *J*=13.4, 4.9 Hz, 1 H), 3.56 - 3.66 (m, 1 H), 4.07 - 4.26 (m, 3 H) 4.43 (d, *J*=12.5 Hz, 1 H), 4.96 -5.05 (m, 1 H), 7.23 (ddd, *J*=8.0, 7.0, 1.4 Hz, 1 H), 7.44 (ddd, *J*=8.1, 7.0, 1.2 Hz, 1 H), 7.51 (dt, *J*=8.1, 1.0 Hz, 1 H), 7.63 (d, *J*=8.5 Hz, 1 H), 7.80 (dd, *J*=8.5, 1.7 Hz, 1 H), 8.12 - 8.21 (m, 2 H), 8.25 (s(br), 1 H), 8.53 (s, 1 H), 8.60 (s, 1 H); MS (DCI/NH₃) m/z 355 (M+1)⁺. Anal. calcd. for C₂₃H₂₂N₄ 2.70CF₃CO₂H·1.2H₂O: C, 49.87; H, 3.99; N, 8.19. Found: C, 49.67; H, 3.86; N, 8.43.

### Example 18

### 7-{5-[(1S,5S)-6-methyl-3,6-diazabicyclo[3.2.0]heptan-3-yl]pyridin-3-yl}-1H-pyrrolo[2,3-c]pyridine tritosylate

### Example 18A

### 7-{5-[(1S,5S)-6-methyl-3,6-diazabicyclo[3.2.0]heptan-3-yl]pyridin-3-yl}-1H-pyrrolo[2,3-c]pyridine

The product of Example 11A (117 mg, 0.50 mmol) was coupled with 7-bromo-1H-pyrrolo[2,3-c]pyridine(AstaTech, 197mg, 1.0 mmol) according to the procedure of Example 12A to give the title compound (40 mg, yield, 26.1%). ¹H NMR (300 MHz, CD₃OD) δ ppm 2.41 (s, 3 H), 3.10 (dd, *J*=11.19, 4.41 Hz, 1 H), 3.20 - 3.28 (m, 1 H), 3.33 - 3.42 (m, 3 H), 3.77 - 3.89 (m, 2 H), 4.04 (dd, *J*=6.78, 4.41 Hz, 1 H), 6.66 (d, *J*=3.05 Hz, 1 H), 7.52 - 7.55 (m, 1 H), 7.57 (d, *J*=3.05 Hz, 1 H), 7.63 (d, *J*=5.76 Hz, 1 H), 8.13 - 8.23 (m, 2 H), 8.33 (d, *J*=1.70 Hz, 1 H); MS (DCI/NH₃) m/z 306 (M+1)⁺.

### Example 18B

### 7-{5-[(1S,5S)-6-methyl-3,6-diazabicyclo[3.2.0]heptan-3-yl]pyridin-3-yl}-1H-pyrrolo[2,3-c]pyridine tritosylate

The product of Example 18A (80 mg, 0.13 mmol) was treated with *p*-TsOH·H₂O (76 mg, 0.40 mmol) in EtOAc (5 mL) at ambient temperature overnight to give the title compound (50 mg, yield, 46.8%). ¹H NMR (400 MHz, Pyridine-D₅) δ ppm 2.17 (s, 9 H), 2.94 (dd, *J*=10.43, 6.14 Hz, 1 H), 3.03 - 3.14 (m, 4 H), 3.40 - 3.57 (m, 1 H), 3.94 (d, *J*=10.13 Hz, 1 H), 4.10 -4.14 (m, 1 H), 4.28 - 4.38 (m, 1 H), 4.78 (d, *J*=12.89 Hz, 1 H), 5.18 (dd, *J*=7.06, 4.91 Hz, 1 H), 6.79 (d, *J*=2.76 Hz, 1 H), 7.16 (d, *J*=7.98 Hz, 6 H), 7.77 (d, *J*=5.52 Hz, 1 H), 8.01 (d, *J*=3.07 Hz, 1 H), 8.06 - 8.11 (m, 1 H), 8.31 (d, *J*=7.98 Hz, 6 H), 8.47 (d, *J*=2.46 Hz, 1 H), 8.60 (d, *J*=5.52 Hz, 1 H), 9.14 (d, *J*=1.53 Hz, 1 H); MS (DCI/NH₃) m/z 306 (M+1)⁺. Anal. calcd. for C₁₈H₁₉N₅·3.05TsOH·2.90H₂O: C, 53.53; H, 5.62; N, 7.93. Found: C, 53.17; H, 5.22; N, 7.87.

### Example 19

### 5-{5-[(1S,5S)-6-methyl-3,6-diazabicyclo[3.2.0]heptan-3-yl]pyridin-3-yl}-1H-pyrrolo[2,3-b]pyridine_tosylate

### Example 19A

### 5-(4,4,5,5-tetramethyl-1,3,2-dioxaborolan-2-yl)-1H-pyrrolo[2,3-b]pyridine

5-Bromo-1H-pyrrolo[2,3-b]pyridine (Alfa Aesar, 1.00 g, 5.0 mmol) was coupled with 4,4,4',4',5,5,5',5'-octamethyl-2,2'-bi(1,3,2-dioxaborolane) (Aldrich, 1.52 g, 6.0 mmol) under the catalysis of {1,1'-bis(diphenylphosphino)-ferrocene]dichloro-palladium(II) dichloromethane complex PdCl₂(dppf)·CH₂Cl₂ (Aldrich, 82 mg, 0.1 mmol) with KOAc (Aldrich, 0.98 g, 10.0 mmol) in dioxane (anhydrous, 20 mL) at 80 °C for 10 h. It was then cooled down to ambient temperature, concentrated and diluted with EtOAc (100 mL). The mixture was then washed with brine (2 x 10 mL). The organic solution was concentrated and the residue was purified using chromatography (SiO₂, EtOAc/hexane, v. 50/50, R_{f}=0.40) to give the title compound (1.15 g, yield, 94.2%). .¹H NMR (300 MHz, CD₃OD) δ ppm 1.38 (s, 12 H) 6.52 (d, *J*=3.39 Hz, 1 H) 7.38 (d, *J*=3.39 Hz, 1 H) 8.34 (d, *J*=1.70 Hz, 1 H) 8.49 (d, *J*=1.36 Hz, 1 H); MS (DCI/NH₃) m/z 245 (M+1)⁺.

### Example 19B

### 5-{5-[(1S,5S)-6-methyl-3,6-diazabicyclo[3.2.0]heptan-3-yl]pyridin-3-yl}-1H-pyrrolo[2,3-b]pyridine

The product of Example 19A (244 mg, 1.0 mmol) was coupled with the product of Example 7A (216 mg, 0.8 mmol) according to the procedure of Example 12A to give the title compound (110 mg, yield, 45.1%). ¹H NMR (300 MHz, CD₃OD) δ ppm 2.40 (s, 3 H), 3.06 (dd, *J*=11.19, 4.41 Hz, 1 H), 3.20 - 3.29 (m, 2 H), 3.33 - 3.38 (m, 2 H), 3.78 - 3.88 (m, 2 H), 4.03 (dd, *J*=6.44, 4.07 Hz, 1 H), 6.58 (d, *J*=3.39 Hz, 1 H), 7.39 (dd, *J*=2.71, 2.03 Hz, 1 H), 7.45 (d, *J*=3.39 Hz, 1 H), 8.04 (d, *J*=2.71 Hz, 1 H), 8.17 (d, *J*=1.70 Hz, 1 H), 8.27 (d, *J*=2.03 Hz, 1 H), 8.46 (d, *J*=2.03 Hz, 1 H); MS (DCI/NH₃) m/z 306 (M+1)⁺.

### Example 19C

### 5-{5-[(1S,5S)-6-methyl-3,6-diazabicyclo[3.2.0]heptan-3-yl]pyridin-3-yl}-1H-pyrrolo[2,3-b]pyridine tosylate

The product of Example 19B (110 mg, 0.36 mmol) was treated with *p*-TsOH-H₂O (76 mg, 0.40 mmol) in EtOAc (5 mL) at ambient temperature overnight to give the title compound (170 mg, yield, 99.0%). ¹H NMR (300 MHz, CD₃OD) δ ppm 2.34 (s, 4.5 H), 3.02 (s, 3 H), 3.10 - 3.26 (m, 2 H), 3.48 - 3.69 (m, 2 H), 4.00 - 4.21 (m, 2 H), 4.33 (d, *J*=13.90 Hz, 1 H), 4.89 - 5.07 (m, 1 H), 6.60 (d, *J*=3.73 Hz, 1 H), 7.20 (d, *J*=8.14 Hz, 3 H), 7.48 (d, *J*=3.73 Hz, 1 H), 7.65 - 7.76 (m, 4 H), 8.19 - 8.27 (m, 1 H), 8.32 (d, *J*=2.03 Hz, 1 H), 8.39 (d, *J*=1.70 Hz, 1 H), 8.50 (d, *J*=2.03 Hz, 1 H); MS (DCI/NH₃) m/z 306 (M+1)⁺, Anal. calcd. for C₁₈H₁₉N₅·1.45TsOH 0.75H₂O: C, 59.53; H, 5.70; N, 12.39. Found: C, 59.93; H, 5.81; N, 12.00.

### Example 20

### 3-{5-[(1S,5S)-6-methyl-3,6-diazabicyclo[3.2.0]heptan-3-yl]pyridin-3-yl}-1-(phenylsulfonyl)-1H-indole bistrifluoroacetate

The product of Example 7A (120 mg, 0.45 mmol) was coupled with 1-(phenylsulfonyl)-1*H*-indol-3-ylboronic acid (Aldrich, 350 mg, 1.16 mmol) according to the procedure of Example 7B to give the title compound (300 mg, yield, 92.8%). ¹H NMR (300 MHz, CD₃OD) δ ppm 2.74 - 2.88 (m, 1 H), 3.03 (s, 3 H), 3.33 - 3.40 (m, 1 H), 3.54 - 3.66 (m, 1 H), 4.02 - 4.24 (m, 3 H), 4.35 (d, *J*=12.5 He, 1 H), 4.95 - 5.04 (m, 1 H), 7.34 - 7.41 (m, 1 H), 7.46 (td, *J*=7.8, 1.4 Hz, 1 H), 7.51 - 7.59 (m, 2 H), 7.62 - 7.69 (m, 1 H), 7.82 (d, *J*=7.5 Hz, 1 H), 7.92 (s, 1 H), 8.01 - 8.07 (m, 2 H), 8.11 (d, *J*=8.1 Hz, 1 H), 8.20 (s, 1 H), 8.29 (s, 1 H), 8.47 (s, 1 H); MS (DCI/NH₃) m/z 445 (M+1)⁺. Anal. calcd. for C₂₅H₂₄N₄O₂S 2.40CF₃CO₂H: C, 49.84; H, 3.70; N, 7.80. Found: C, 49.85; H, 3.76; N, 7.71.

### Example 21

### 3-(5-((1S,5S)-6-methyl-3,6-diazabicyclo[3.2.0]heptan-3-yl)pyridin-3-yl)-1H-indole bistrifluoroacetate

The product of Example 20 (250 mg, 0.35 mmol) was treated with KOH (0.15 g, 2.68 mmol) in methanol (8 mL) at 50°C for 2 hours. After being concentrated, the mixture was purified by preparative HPLC (Gilson, column, Xterra^{®} 5 µm, 40 x 140 mm. Eluting Solvent, MEON /H₂O (with 0.1% v. TFA) (v. 90/10 to 10/90 over 25 min.), flow rate, 40 mL/min., uv, 254 nm). Fractions of the desired product were collected and concentrated. The residue was stirred in ether/ethanol (v. 10/1, 5 mL) at room temperature for 16 hours to give the title compound (128.4 mg, yield, 62.5%). ¹H NMR (300 MHz, CD₃OD) δ ppm 2.76 - 2.86 (m, 1 H), 3.04 (s, 3 H), 3.42 (dd, *J*=13.1, 4.9 Hz, 1 H), 3.56 - 3.65 (m, 1 H), 4.03 - 4.25 (m, 3 H), 4.39 (d, *J*=11.9 Hz, 1 H), 4.95 -5.03 (m, 1 H), 7.18 - 7.30 (m, 2 H), 7.52 (dd, *J*=7.0, 1.5 Hz, 1 H), 7.88 (s, 1 H), 7.90 - 7.96 (m, 1 H), 8.10 [s(br), 1 H], 8.15 [s(br), 1 H], 8.53 (s, 1 H); MS (DCI/NH₃) m/z 305 (M+1)⁺. Anal. calcd. for C₁₉H₂₀N₄·2.50CF₃CO₂H: C, 48.90; H, 3.85; N, 9.50. Found: C, 48.98; H, 3.62; N, 9.67.

### Example 22

### 4-{6-[(1S,5S)-3,6-diazabicyclo[3.2.0]heptan-3-yl]pyrazin-2-yl}-1H-indole bistosylate

### Example 22A

### (1R,5S)-tert-butyl 3-(6-chloropyrazin-2-yl)-3,6-diazabicyclo[3.2.0]heptane-6-carboxylate

(*1R,5S*)-*tert*-Butyl 3,6-diazabicyclo[3.2.0]heptane-6-carboxylate (US 2006035936, 0.99 g, 5.0 mmol) was coupled with 2,6-dichloropyrazine (Aldrich, 1.12 g, 7.5 mmol) with Na₂CO₃ (Aldrich, 1,06 g, 10 mmol) in DMSO (5 .0 mL) at 110 °C for 10 h. After the reaction was completed, it was then diluted with EtOAc (50 mL) and washed with brine (2 x 10 mL). The organic solution was then concentrated, and the residue was purified using chromatography (SiO₂, EtOAc/hexane, v. 50/50, R_{f}=0.10) to give the title compound (1.32 g, yield, 84.9%). ¹H NMR (300 MHz, CD₃OD) δ ppm 1.44 (s, 9 H), 3.13 - 3.29 (m, 2 H), 3.31-3.35 (m, 1 H), 3.46 - 3.63 (m, 1 H), 4.02 (d, *J*=10.85 Hz, 1 H), 4.06 - 4.15 (m, 2 H), 4.19 (d, *J*=12.55 Hz, 1 H), 4.83 - 4.86 (m, 1 H), 7.80 (s, 1 H), 7.97 (s, 1 H); MS (DCI/NH₃) m/z 311 (M+1)⁺, 313 (M+1)⁺.

### Example 22B

### 4-{6-[(1S,5S)-3,6-diazabicyclo[3.2.0]heptan-3-yl]pyrazin-2-yl}-1H-indole

The product of Example 22A (103 mg, 0.3 mmol) was coupled with *1H*-indol-4-ylboronic acid (Frontier, 80 mg, 0.5 mmol) according to the procedure of Example 12A. After the reaction was completed, it was concentrated, and the residue was treated with trifluroacetic acid (2 mL) at ambient temperature for 1.0 h. After being concentrated, the crude mixture was purified using preparative HPLC (Gilson, Xterra® column, 7 µm, 40 x 100 mm, eluting solvent, MeCN /H₂O (with 0.1 M NH₄HCO₃/NH₄OH, PH=10) (v. 90/10 to 10/90 over 25 minutes), flow rate, 40 mL/min., uv, 254 nm) to give the title compound (30 mg, yield, 32.8%). ¹H NMR (300 MHz, CD₃OD) δ ppm 3.32 - 3.50 (m, 4 H), 3.96 (t, *J*=8.31 Hz, 1 H), 4.08 - 4.26 (m, 2 H), 4.64 - 4.75 (m, 1 H), 7.07 (dd, *J*=3,22, 0.85 Hz, 1 H), 7.19 - 7.26 (m, 1 H), 7.35 (d, *J*=3,39 Hz, 1 H), 7.50 (dt, *J*=8.14, 0.85 Hz, 1 H), 7.56 (dd, *J*=7,46, 1.02 Hz, 1 H), 7.99 (s, 1 H), 8.38 (s, 1 H); MS (DCI/NH₃) m/z 292 (M+1)⁺.

### Example 22C

### 4-{6-[(1S,5S)-3,6-Diazabicyclo[3.2.0]heptan-3-yl]pyrazin-2-yl}-1H-indole bistosylate

The product of Example 22B (30 mg, 0.10 mmol) was treated with *p*-TsOH•H₂O (38 mg, 0.20 mmol) in EtOAc (5 mL) at ambient temperature overnight to give the title compound (65 mg, yield, 99.0%). ¹H NMR (500 MHz, Pyridine-D₅) δ ppm 2.17 (s, 6 H), 3.27 (dd, *J*=10.98, 6.41 Hz, 1 H), 3.39 (dd, *J*=13.12, 5,19 Hz, 1 H), 3.47-3.59 (m, 1 H), 3.96 (dd, *J*=10.98, 5.19 Hz, 1 H), 4.23 (d, *J=*11.29 Hz, 1 H), 4.67 (dd, *J*=10.83, 8.70 Hz, 1 H), 4.79 (d, *J*=13.43 Hz, 1 H), 5.53 (dd, *J=*6.71, 5.19 Hz, 1 H), 7.18 (d, *J*=7.93 Hz, 4 H), 7.40 - 7.49 (m, 2 H), 7.71 (t, *J*=2.75 Hz, 1 H), 7.79 (d, *J*=7.93 Hz, 1 H), 7.89 (d, *J*=7.02 Hz, 1 H), 8.22 (s, 1 H), 8.39 (d, *J*=8.24 Hz, 4 H), 8.94 (s, 1 H), 12.46 (s, 1 H); MS (DCI/NH₃) m/z 292 (M+1)⁺. Anal. calcd. for C₁₇H₁₇N₅·2.00TsOH 0.85H₂O: C, 57.19; H, 5.37; N, 10.76. Found: C, 57.38; H, 5.29; N, 11.04.

### Example 23

### 4-{6-[(1S,5S)-6-methyl-3,6-diazabicyclo[3.2.0]heptan-3-yl]pyrazin-2-yl}-1H-indole bistosylate

### Example 23A

### (1S,5S)-3-(6-chloropyrazin-2-yl)-3,6-diazabicyclo[3.2.0]hcptane

The product of Example 22A (1.30 g, 4.18 mmol) was treated with *p*-TsOH·H₂O (1.78 g, 9.36 mmol) in EtOAc (30 mL) at 80 °C for 2 h to give the title compound (1.50 g, yield, 93.4%). ¹H NMR (300 MHz, CD₃OD) δ ppm 2.38 (s, 3H), 3.33 (dd, *J*=11.60, 6.50 Hz, 1 H), 3.44 (dd, *J*=13.73*,* 5.26 Hz, 1 H), 3.50 - 3.63 (m, 1 H),3.72 (dd, *J*=11.02, 5.26 Hz, 1 H), 4.12 (d, *J*=11.87 Hz, 1 H), 4.26 (dd, *J*=11.02, 8.65 Hz, 1 H), 4,39 (d, *J*=13.56 Hz, 1 H), 5.03 - 5.15 (m, 1 H), 7.23 (d, *J*=7,80 Hz, 2 H), 7.69 (d, *J*=8.48 Hz, 2 H), 7.95 (s, 1 H), 8.11 (s, 1 H); MS (DCI/NH₃) m/z 211 (M+1)⁺, 213 (M+1)⁺.

### Example 23B

### (1S,5S)-3-(6-chloropyrazin-2-yl)-6-methyl-3,6-diazabicyclo[3.2.0]heptane

The product of Example 23A (1.30 g, 4.18 mmol) was treated with with formaldehyde in the presence of NaBH(OAc)₃ according to the procedure of Example 2A to give the title compound (1.50 g, yield, 93.4%). ¹H NMR (300 MHz, CD₃OD) δ ppm 2.66 (s, 3 H), 3.32 - 3.52 (m, 3 H), 3.53 - 3.64 (dd, *J*=9.50, 3.80 Hz, 1 H), 3.61 - 3.76 (m, 1 H), 3.99 (d, *J*=11.19 Hz, 1 H), 4.15 (d, *J*=13.22 Hz, 1 H), 4.44 (dd, *J*=6.78, 4.75 Hz, 1 H), 7.84(s, 1 H), 7.99 (s, 1 H); MS (DCI/NH₃) m/z 225 (M+1)⁺, 227 (M+1)⁺.

### Example 23C

### 4-{6-[(1S,5S)-6-methyl-3,6-diazabicyclo[3.2.0]heptan-3-yl]pyrazin-2-yl}-1H-indole

The product of Example 23B (112 mg, 0.5 mmol) was coupled with *1H-*indol-4-ylboronic acid (Frontier, 160 mg, 1.0 mmol) according to the procedure of Example 12A to give the title compound (140 mg, yield, 92.0%). ¹H NMR (300 MHz, CD₃OD) δ ppm 2.40 (s, 3 H), 3.18 - 3.38 (m, 4 H), 3.61 (dd, *J*=11.19, 8.14 Hz, 1 H), 4.00 - 4.08 (m, 3 H), 7.06 (dd, *J*=3.22, 0.85 Hz, 1 H), 7.18 - 7.26 (m, 1 H), 7.34 (d, *J*=3.39 Hz, 1 H), 7.47 - 7.52 (m, 1 H), 7.49 (d, *J*=8.14 Hz, 1 H), 7.91 (s, 1 H), 8.32 (s, 1 H); MS (DCI/NH₃) m/z 306(M+1)⁺.

### Example 23D

### 4-{6-[(1S,5S)-6-methyl-3,6-diazabicyclo[3.2.0]heptan-3-yl]-pyrazin-2-yl}-1H-indole bistosylate

The product of Example 23C (140 mg, 0.46 mmol) was treated with *p*-TsOH•H₂O (190 mg, 1.0 mmol) in EtOAc (10 mL) at ambient temperature overnight to give the title compound (170 mg, yield, 56.9%). ¹H NMR (300 MHz, CD₃OD) δ ppm 2.31 (s, 6 H), 3.04 (s, 3 H), 3.55 - 3.77 (m, 3 H), 4.15 - 4.22 (m, 2 H), 4.32 (d, *J*=11.19 Hz, 1 H), 4.72 (d, *J*=14.24 Hz, 1 H), 5.03 (dd, *J*=6.95, 4.92 Hz, 1 H), 6.57 (d, J=3.05 Hz, 1H), 7.17 (d, *J*=7.80 Hz, 4 H), 7.24 - 7.33 (m, 1 H), 7.44 - 7.48 (m, 1 H), 7.61 - 7.68 (m, 5 H), 7.72 (d, *J*=7.46 Hz, 1 H), 8.12 (s, 1 H), 8.53 (s, 1 H); MS (DCI/NH₃) m/z 306 (M+1)⁺. Anal. calcd. for C₁₈H₁₉N₅·2.08TsOH 1.70H₂O: C, 56.34; H, 5.67; N, 10.09. Found: C, 56.66; H, 5.39; N, 9.70.

### Example 24

### 5-{6-[(1S,5S)-6-methyl-3,6-diazabicyclo[3.2.0]heptan-3-yl]pyrazin-2-yl}-1H-indole tosylate

### Example 24A

### 5-{6-[(1S,5S)-6-methyl-3,6-diazabicyclo[3.2.0]heptan-3-yl]pyrazin-2-yl}-1H-indole

The product of Example 23B (112 mg, 0.5 mmol) was coupled with *1H*-indol-5-ylboronic acid (Frontier, 160 mg, 1.0 mmol) according to the procedure of Example 12A to give the title compound (120 mg, yield, 78.7%). ¹H NMR (300 MHz, CD₃OD) δ ppm 3.20 - 3.39 (m, 4 H), 3.58 (dd, *J*=11.53, 8.14 Hz, 1 H), 4.00 -4.08 (m, 3 H), 6.54 (d, *J*=2.37 Hz, 1 H), 7.28 (d, *J*=3.05 Hz, 1 H), 7.46 (d, *J*=8.82 Hz, 1 H), 7.81 - 7.88 (m, 2 H), 8.29 - 8.30 (m, 1 H), 8.31 (s, 1 H); MS (DCI/NH₃) m/z 306(M+1)⁺.

### Example 24B

### 4-{6-[(1S,5S)-6-methyl-3,6-diazabicyclo[3.2.0]heptan-3-yl]pyrazin-2-yl}-1H-indole tosylate

The product of Example 24A(120 mg, 0.39 mmol) was treated with *p*-TsOH•H₂O (950 mg, 0.5 mmol) in EtOAc (10 mL) at ambient temperature overnight to give the title compound (170 mg, yield, 91,4%). ¹H NMR (300 MHz, CD₃OD) δ ppm 2.34 (s, 4.5 H), 3.05 (s, 3 H), 3,41 - 3.72 (m, 3 H), 4.09-4.19 (m, 2 H), 4.27 (d, *J*=11.53 Hz, 1 H), 4.67 (d, *J*=13.90 Hz, 1 H), 4.99 (dd, *J*=7.29, 4.92 Hz, 1 H), 6.57 (d, *J*=3.05 Hz, 1 H), 7.19 (d, *J*=8.14 Hz, 3 H) 7.32 (d, *J*=3.05 Hz, 1 H), 7.50 (d, *J*=8.82 Hz, 1 H), 7.68 (d, *J*=8.14 Hz, 3 H), 7.91 (dd, *J*=8.48, 1.70 Hz, 1 H), 8.04 (s, 1 H), 8.39 (s, 1 H), 8.53 (s, 1 H); MS (DCT/NH₃) m/z 306 (M+1)⁺. Anal. calcd. for C₁₈H₁₉N₅·1.49TsOH 1.00H₂O: C, 58.88; H, 5.72; N, 12.08. Found: C, 58.85; H, 5.23; N, 11.68.

### Example 25

### 6-{6-[(1S,5S)-6-methyl-3,6-diazabicyclo[3.2.0]heptan-3-yl]pyrazin-2-yl}-1H-indole bistosylate

### Example 25A

### 6-{6-[(1S,5S)-6-methyl-3,6-diazabicyclo[3.2.0]heptan-3-yl]pyrazin-2-yl}-1H-indole

The product of Example 23B (112 mg, 0.5 mmol) was coupled with *1H*-indol-6-ylboronic acid (Frontier, 160 mg, 1.0 mmol) according to the procedure of Example 12A to give the title compound (80 mg, yield, 52.4%). ¹H NMR (300 MHz, CD₃OD) δ ppm 2.40 (s, 3 H), 3.20 - 3.28 (m, 2 H), 3.32 - 3.41 (m, 2 H), 3.59 (dd, *J*=11.53, 8.14 Hz, 1 H), 3.97 - 4.13 (m, 3 H), 6.48 (dd, *J*=3.22, 0.85 Hz, 1 H), 7.32 (d, *J*=3.05 Hz, 1 H), 7.62 (d, *J*=7.80 Hz, 1 H) 7.74 (dd, *J*=8.50, 1.70 Hz, 1 H), 7.85 (s, 1 H), 8.15 (s, 1 H,) 8.32 (s, 1 H); MS (DCI/NH₃) m/z 306(M+1)⁺.

### Example 25B

### 4-{6-[(1S,5S)-6-methyl-3,6-diazabicyclo[3.2.0]heptan-3-yl]pyrazin-2-yl}-1H-indole bistosylate

The product of Example 25A(80 mg, 0.26 mmol) was treated with *p*-TsOH·H₂O (114 mg, 0.6 mmol) in EtOAc (10 mL) at ambient temperature overnight to give the title compound (120 mg, yield, 70.6%). ¹H NMR (300 MHz, CD₃OD) δ ppm 2.30 s, 6 H), 3.04 (s, 3 H), 3.55 - 3.67 (m, 3 H), 4.14 - 4.21 (m, 2 H), 4.31 (d, *J*=11.53 Hz, 1 H), 4.72 (d, *J*=14.24 Hz, 1 H), 5.03 (dd, *J*=7.12, 5.09 Hz, 1 H), 6.55 (d, *J*=3.05 Hz, 1 H) 7,17 (d, *J*=8.48 Hz, 4 H), 7.41 - 7.47 (m, 1 H), 7.66 (d, *J*=8.14 Hz, 4 H), 7.70 (d, *J*=8.48 Hz, 1 H), 7.82 (dd, J=8.40, 1.40 Hz, 1 H), 8.06 (s, 1 H), 8.24 (s, 1 H), 8.51 (s, 1 H); MS (DCI/NH₃) m/z 306 (M+1)⁺. Anal. calcd. for C₁₈H₁₉N₅·2.00TsOH·0.70H₂O: C, 58.02; H, 5.54; N, 10.57. Found: C, 57.71; H, 5.24; N,10.35.

### Example 26

### 5-(6-((1S,5S)-6-methyl-3,6-diazabicyclo[3.2.0]heptan-3-yl)pyrazin-2-yl)-2-(trifluoromethyl)-1H-indole bisfumarate

The product of Example 23B (105 mg, 0.469 mmol) was coupled with 5-(4,4,5,5-tetramethyl-1,3,2-dioxaborolan-2-yl)-2-(trifluoromethyl)-1H-indole (US 2005043347, 225 mg, 0.723 mmol) according to the procedure of Example 14A to give the free base of title compound (15 mg, yield, 10%). The free base (15 mg, 0.04 mmol) was treated with fumaric acid (12 mg, 0.1 mmol) in ether/ethanol (v. 10/1,5 mL) at room temperature for 16 hours give the title compound (17.8 mg, yield, 53.3%). ¹H NMR (300 MHz, CD₃OD) δ ppm 2.96 (s, 3 H), 3.37 (dd, *J=*11.2*,* 6.4 Hz, 1 H), 3.46 (dd, *J*=13.9, 4.7 Hz, 1 H), 3.59 (dt, *J*=13.6, 6.9 Hz, 1 H), 3.99 (dd, *J*=11.2, 5.1 Hz, 1 H), 4.19 - 4.28 (m, 2 H), 4.64 (d, *J*=13.9 Hz, 1 H), 4.97 (dd, *J*=7.0, 4.9 Hz, 1 H), 6,98 - 7.01 (m, 1 H), 7.56 (d, *J*=8.8 Hz, 1 H), 8.03 - 8.10 (m, 2 H), 8.45 (d, *J*=1.0 Hz, 1 H), 8.54 (s, 1 H),; MS (DCI/NH₃) m/z 374 (M+1)⁺; Anal. calcd. for C₁₉H₁₈F₃N₅·2.33C₄O₄H₄ 1.20H₂O·: C, 51.12; H, 4.50; N, 10.52. Found: C, 51.51; H, 4.13; N, 10.14,

### Example 27

### 5-{5-[(1R,5R)-6-methyl-3,6-diazabicyclo[3.2.0]heptan-3-yl]pyridin-3-yl}-1H-indole bistosylate

### Example 27A

### (1S,5R)-tert-butyl 3-(5-bromopyridin-3-yl)-3,6-diazabicyclo[3.2.0]heptane-6-carboxylate

3,5-Dibromopyridine (Aldrich, 1.00 g, 4.5 mmol) was coupled with (*1S,5R*)*-tert-*butyl 3,6-diazabicyclo[3.2.0]heptane-6-carboxylate (WO 2001081347, 0.60 g, 3 mmol) accoding to the procedure of Example 1A to give the title compound (0.55 g, yield, 50%). ¹H NMR (300 MHz, CD₃OD) δ ppm 1.45 (s, 9 H), 2.95 (dd, *J*=11.19, 4.41 Hz, 1 H), 3.05 (dd, *J*=10.51, 6.78 Hz, 1 H), 3.18 - 3.29 (m, 1 H), 3.51 - 3.67 (m, 1 H), 3.79 (d, *J*=10.51 Hz, 1 H), 3.93 (d, *J*=11.53 Hz, 1 H), 4.02 - 4.19 (m, 1 H), 4.83 - 4,91 (m, Hz, 1 H), 7.39 (t, *J*=2.20 Hz, 1 H), 7.97 (d, *J*=1.70 Hz, 1 H), 8.05 (d, *J*=2.37 Hz, 1 H); MS (DCI/NH₃) m/z 354 (M+1)⁺, 356 (M+1)⁺.

### Example 27B

### (1R,5R)-3(5-bromopyridin-3-yl)-6-methyl-3,6-diazabicyclo[3.2.0]heptane

The product of Example 27A (0.55 g, 12.7 mmol) was treated with HCHO (Aldrich, 37%, 5 mL) in HCO₂H (Aldrich, 88%, 10 mL) according to the procedure of Example 7A to give the title compound (0.33 g, yield, 79%). ¹H NMR (300 MHz, CD₃OD) δ ppm 2.37 (s, 3 H), 3.02 (dd, *J*=11.19, 4.41 Hz, 1 H), 3.15 - 3.38 (m, 4 H), 3.60 - 3.79 (m, 2 H), 3.99 (dd, *J*=6.44, 4.41 Hz, 1 H), 7.27 - 7.35 (t, *J*=2.00 Hz, 1 H), 7.92 (d, *J*=2.03 Hz, 1 H), 7.99 (d, *J*=2.37 Hz, 1 H); MS (DCI/NH₃) m/z 268 (M+1)⁺, 270 (M+1)⁺.

### Example 27C

### 5-{5-[(1R,5R)-6-methyl-3,6-diazabicyclo[3.2.0]heptan-3-yl]pyridin-3-yl}-1H-indole

The product of Example 27B (100 mg, 0.37 mmol) was coupled with the indol-5-ylboronic acid (Frontier, 81.6 mg, 0.51 mmol) according to the procedure of Example 12A to give the title compound (40 mg, yield, 41.0%). ¹H NMR (300 MHz, CD₃OD) δ ppm 2.44 (s, 3 H), 3.04 (dd, *J*=11.02, 4.24 Hz, 1 H), 3.18 - 3.28 (m, 2 H), 3.34 - 3.47 (m, 2 H), 3.78 - 3.91 (m, 2 H), 4.09 (dd, *J*=6.10, 4.75 Hz, 1 H), 6.52 (d, *J*=3.05 Hz, 1 H), 7.28 (d, *J*=3.05 Hz, 1 H), 7.36 - 7.43 (m, 2 H), 7,45 - 7.52 (m, 1 H), 7.83 (s, 1 H), 7.98 (d, *J*=2.71 Hz, 1 H), 8.18 (s, 1 H); MS (DCI/NH₃) m/z 305 (M+1)⁺.

### Example 27D

### 5-{5-[(1R,5R)-6-methyl-3,6-diazabicyclo[3.2.0]heptan-3-yl]pyridin-3-yl}-1H-indole bistosylate

The product of Example 27C (40 mg, 0.13 mmol) was treated with *p*-TsOH•H₂O (49 mg, 0.26 mmol) in EtOAc (5 mL) at ambient temperature overnight to give the title compound (50 mg, yield, 59.4%). ¹H NMR (500 MHz, Pyriditie-D₅) δ ppm 2.16 (s, 6 H), 2.90 (dd, *J*=10.07, 6.10 Hz, 1 H), 2.98 - 3.12 (m, 4 H), 3.41 - 3.57 (m, 1 H), 3.82 (d, *J*=10.07 Hz, 1 H), 3.92 - 4.02 (m, 1 H), 4.44 (t, *J*=9.46 Hz, 1 H), 4.59 (d, *J*=12.82 Hz, 1 H), 5.16 - 5.29 (m, 1 H), 6.82 (s, 1 H), 7.15 (d, *J*=7.93 Hz, 4 H), 7.59 - 7.61 (m, 1 H), 7.62 - 7.69 (m, 2 H), 7.77 (d, *J*=8.54 Hz, 1 H), 8.18 (s, 1 H), 8.35 (d, *J*=7.93 Hz, 4 H), 8.47 (d, *J*=2.44 Hz, 1 H), 8.84 (d, *J*=1.53 Hz, 1 H), 12.34 (s, 1 H), MS (DCI/NH₃) m/z 305 (M+1)⁺. Anal. calcd. for C₁₉H₂₀N₄·2.25TsOH 0.40H₂O: C, 59.71; H, 5.59; N, 8.01. Found: C, 59.77; H, 5.19; N, 8.12.

### Example_28

### 4-{5-[(1R,5R)-6-methyl-3,6-diazabicyclo[3.2.0]heptan-3-yl]pyridin-3-yl}-1H-indole tritosylate

### Example 28A

### 4-{5-[(1R,5R)-6-methyl-3,6-diazabicyclo[3.2.0]heptan-3-yl]pyridin-3-yl}-1H-indole

The product of Example 27B (100 mg, 0.37 mmol) was coupled with the indol-4-ylboronic acid (Frontier, 81.6 mg, 0.51 mmol) according to the procedure of Example 12A to give the title compound (15 mg, yield, 13 %). ¹H NMR (300 MHz, CD₃OD) δ ppm 3.02 (s, 3 H), 3.17 - 3.27 (m, 2 H), 3.46 - 3.67 (m, 1 H), 3.98 - 4.22 (m, 3 H), 4.30 (dd, dd, *J*=11.87, 2.37 Hz , 1 H), 4.93 - 5.04 (m, 1 H), 6.62 (d, *J*=2.37 Hz, 1 H), 7.15 - 7.30 (m, 2 H), 7.35 - 7.40 (m, 1 H), 7.51 (d, *J*=8.14 Hz, 1 H), 7.85 (s, 1 H), 8.25 (d, *J*=3.05 Hz, 1 H), 8.42 (s, 1 H); MS (DCI/NH₃) m/z 305 (M+1)⁺.

### Example 28B

### 5-{5-[(1R,5R)-6-methyl-3,6-diazabicyclo[3.2.0]heptan-3-yl]pyridin-3-yl}-1H-indole tritosylate

The product of Example 28A (15 mg, 0.05 mmol) was treated with *p*-TsOH•H₂O(27 mg, 0.15 mmol) in EtOAc (5 mL) at ambient temperature overnight to give the title compound (15 mg, yield, 36.5%). ¹H NMR (500 MHz, Pyridine-D₅) δ ppm 2.16 (s, 9 H), 2.93 (dd, *J*=10.07, 6.10 Hz, 1 H), 3.01 (s, 3 H), 3.07 (dd, *J*=12.51, 4.88 Hz, 1 H), 3.41- 3.54 (m, 1 H), 3.79 (d, *J*=10.07 Hz, 1 H), 3.87 - 3.98 (m, 1 H), 4.40 (t, *J*=9.92 Hz, 1 H), 4.49 (d, *J*=12.82 Hz, 1 H), 5.14 - 5.20 (m, 1 H), 6.99 (s, 1 H), 7.14 (d, *J*=7.93 Hz, 6 H), 7.36 - 7.45 (m, 2 H), 7.60 - 7.62 (m, 1 H), 7.63 - 7.67 (m, 1 H), 7.71 (dd, *J*=6.10, 2.75 Hz, 1 H), 8.37 (d, *J*=7.93 Hz, 6 H), 8.53 (d, *J*=2.75 Hz, 1 H), 8.91 (d, *J*=1.83 Hz, 1 H), 12.44 (s, 1 H); MS (DCI/NH₃) m/z 305 (M+1)⁺. Anal. calcd. for C₁₉H₂₀N₄ 3.00TsOH·1.20H₂O: C, 57.02; H, 5.55; N, 6.65. Found: C, 57.06; H, 5.23; N, 6.26.

### Example 29

### 6-{5-[(1R,5R)-6-methyl-3,6-diazabicyclo[3.2.0]heptan-3-yl]pyridin-3-yl}-1H-indole bistosylate

### Example 29A

### 6-{5-[(1R,5R)-6-methyl-3,6-diazabicyclo[3.2.0]heptan-3-yl]pyridin-3-yl}-1H-indole

The product of Example 27B (100 mg, 0.37 mmol) was coupled with the indol-6-ylboronic acid (Frontier, 81.6 mg, 0.51 mmol) according to the procedure of Example 12A to give the title compound (50 mg, yield, 44 %). ¹H NMR (300 MHz, CD₃OD) δ ppm 2.40 (s, 3 H), 3.04 (dd, *J*=11.19, 4.41 Hz, 1 H), 3.20 - 3.28 (m, 2 H), 3.32 - 3.44 (m, 2 H), 3.74 - 3.90 (m, 2 H), 4.04 (dd, *J*=6.10*,* 4.41 Hz, 1 H), 6.48 (d, *J*=3.05 Hz, 1 H), 7.28 - 7.33 (m, 2 H), 7.38 (t, *J*=2.40 Hz, 1 H), 7.59 - 7.70 (m, 2 H), 7.98 (d, *J*=2.71 Hz, 1 H), 8.17 (d, *J*=1.70 Hz, 1 H); MS (DCI/NH₃) m/z 305 (M+1)⁺.

### Example 29B

### 6-{5-[(1R,5R)-6-methyl-3,6-diazabicyclo[3.2.0]heptan-3-yl]pyridin-3-yl}-1H-indole tritosylate

The product of Example 29A (50 mg, 0.16 mmol) was treated with *p*-TsOH•H₂O (38 mg, 0.20 mmol) in EtOAc (5 mL) at ambient temperature overnight to give the title compound (55 mg, yield, 53.0%). ¹H NMR (500 MHz, Pyridine-D₅) δ ppm 2.15 (s, 6 H), 2.87 (dd, *J*=10.07, 6.10 Hz, 1 H), 2.98 - 3.12 (m, 4 H), 3.40 - 3.53 (m, 1 H), 3.78 (d, *J*=10.37 Hz, 1 H), 3.89 - 4.00 (m, 1 H), 4.41 (t, *J*=9.76 Hz, 1 H), 4.57 (d, *J*=12.82 Hz, 1 H) 5.09 - 5.36 (m, 1 H), 6.79 (s, 1 H), 7.14 (d, *J*=7.93 Hz, 4 H), 7.58 - 7.65 (m, 2 H), 7.93 (d, *J*=8.24 Hz, 1 H), 8.12 (s, 1 H), 8.34 (d, *J*=8.24 Hz, 4 H), 8,44 (d, *J*=2.44 Hz, 1 H), 8.81 (d, *J*=1.83 Hz, 1 H), 12.30 (s, 1 H); MS (DCI/NH₃) m/z 305 (M+1)⁺. Anal, calcd. for C₁₉H₂₀N₄ 2.00TsOH·0.80H₂O: C, 59.76; H, 5.71; N, 8.45. Found: C, 59.37; H, 5.64; N, 8.19.

### Example 30

### 5-{5-[(1R,5R)-3,6-diazabicyclo[3.2.0]heptan-6-yl]pyridin-3-yl}-1H-indole bistrifluoroacetate

### Example 30A

### (1S,5SR)-benzyl 6-(5-bromopyridin-3-yl)-3,6-diazabicyclo[3.2.0]heptan-3-carboxylate

3,5-Dibromopyridine (Aldrich, 2.60 g, 11 mmol) was coupled with (*1S,5S*)-benzyl 3,6-diazabicyclo[3.2.0]heptane-3-carboxylate tosylate (US 2006035936, 4.05 g, 10 mmol) under the catalysis of Pd₂(dba)₃ (Aldrich, 45 mg, 0.05 mmol) and 4,5-bis(diphenylphosphino)-9,9-dimethylxanthene (Aldrich, 80 mg, 0.138 mmol) in the presence of sodium *t*-butoxide (Aldrich, 2.88 g, 30.0 mmol) in toluene (anhydrous, Aldrich, 40 mL) at 100 °C for 16 h. After the completion of the reaction, it was cooled down to ambient temperature and diluted with EtOAc (100 mL), washed with brine (2 x 20 mL), and concentrated under reduced pressure. The residue was purified using chromatography (SiO₂, EtOAc/hexane, v. 50/50, R_{f}=0.40) to give the title compound (2.57 g, yield, 66%). ¹H NMR (300 MHz, CD₃OD) δ ppm 3.21 (dd, *J=*12.9, 4.1 Hz, 1 H), 3.62 (dd, *J*=7.8, 3.4 Hz, 1 H), 3.93 - 4.12 (m, 4 H), 4.73 (dd, *J*=6.1, 4.1 Hz, 1 H), 5.12 (s, 2 H), 7.01 - 7.07 (m, 1 H), 7.18 - 7.41 (m, 5 H), 7.69 (d, *J*=2.4 Hz, 1 H), 7.90 (d, *J*=1.7 Hz, 1 H); MS (DCI/NH₃) m/z 388 (M+1)⁺, 390 (M+1)⁺.

### Example 30B

### (1R,5S)-6-(5-bromopyridin-3-yl)-3,6-diazabicyclo[3.2.0]heptane

The product of Example 30A (2.57 g, 6.6 mmol) was treated with trifluoroacetic acid (Aldrich, 15 mL) at 75°C for 1.5 h. The mixture was then cooled to ambient temperature and concentrated. The residue was diluted with CHCl₃ (100 mL), washed with saturated Na₂CO₃( 2 x 10 mL) and then concentrated under reduced pressure. The residue was purified using chromatography (SiO₂, EtOAc/MeOH-NH₄OH, v. 70/30) to give the title compound (1.6 g, yield, 95%). ¹H NMR (300 MHz, CD₃OD) δ ppm 3.03 (dd, *J*=12.7, 3.6 Hz, 1 H), 3.19 (dd, *J*=12.4, 7.3 Hz, 1 H), 3.36 - 3.43 (m, 1 H), 3.60 (t, *J*=13.1 Hz, 2 H), 3.74 (dd, *J*=8.1, 3.4 Hz, 1 H), 4.02 (t, *J*=8.0 Hz, 1 H), 4.89 (dd, *J*=6.3,3.6 Hz, 1 H), 7.16 - 7.20 (m, 1 H) 7.80 (d, *J*=2.7 Hz, 1 H) 7.98 (d, *J*=2.0 Hz, 1 H); MS (DCI/NH₃) m/z 254 (M+1)⁺, 256 (M+1)⁺.

### Example 30C

### 5-{5-[(1R,5R)-3,6-diazabicyclo[3.2.0]heptan-6-yl]pyridin-3-yl}-1H-indole bistrifluoroacetate

The product of Example 30B (120 mg, 0.47 mmol) was coupled with *1H*-indol-5-ylboronic acid (Frontier, 160 mg, 1.0 mmol) according to the procedure described in Example 7B to give the title compound (135.5 mg, yield, 52%). ¹H NMR (300 MHz, CD₃OD) δ ppm 3.22 - 3.29 (m, 1 H), 3.39 (dd, *J*=12.9, 7.8 Hz, 1 H), 3.52 - 3.63 (m, 1 H), 3.78 (d, *J*=12.5 Hz, 1 H), 3.85 - 3.96 (m, 2 H), 4.24 (t, *J*=8.3 Hz, 1 H), 5.16 (dd, *J*=6.4, 3.7 Hz, 1 H), 6.56 - 6.59 (m, 1 H), 7.33 - 7.36 (m, 1 H), 7.45 - 7.51 (m, 1 H), 7.53 - 7.57 (m, 1 H), 7.72 - 7.77 (m, 1 H), 7.96 (d, *J*=2.4 Hz, 1 H), 7.98 (d, *J*=1.7 Hz, 1 H), 8.42 (d, *J*=1.7 Hz, 1 H); MS (DCT/NH₃) m/z 291(M+1)⁺, Anal. calcd. for C₁₈H₁₈N₄·2.20CF₃CO₂H·0.70H₂O: C, 48.58; H, 3.93; N, 10.12. Found: C, 48.61; H, 3.99; N, 9.75.

### Example 31

### 5-{5-[(1R,5S)-3-methyl-3,6-diazabicyclo[3.2.0]heptan-6-yl]pyridin-3-yl}-1H-indole tosylate

### Example 31A

### (1R,5S)-6-(5-bromopyridin-3-yl)-3-methyl-3,6-diazabicyclo[3.2.0]heptane

The product of Example 30B (1.2 g, 4.7 mmol) was treated with fomaldhyde (Aldrich, aq., wt. 37%, 5 mL, 62 mmol) and NaBH(OAc)₃ (Aldrich, 600 mg, 2.83 mmol) in MeCN (20 mL) at room temperature for 16 h. The mixture was then extracted with CHCl₃ (3 x 20 mL). The combined extracts were concentrated, and the residue was purified with chromatography (SiO₂, EtOAc/MeOH-N₄OH, v. 70/30, R_{f}=0.20) to give the title compound (1.1 g, yield, 87.7%). ¹H NMR (300 MHz, CD₃OD) δ ppm 2.33 (dd, *J*=11.5, 3.7 Hz, 1 H), 2.43 (dd, *J*=10.9, 6.4 Hz, 1 H), 2.57 (s, 3 H), 3.30-3.38 (m, 2H), 3.44 (d, *J*=11.5 Hz, 1 H), 3.75 (dd, *J*=8.0, 3.9 Hz, 1 H), 3.98 (t, *J*=8.0 Hz, 1 H), 4.73 - 4.78 (m, 1 H), 7.05 (t, *J*=2.2 Hz, 1 H) 7.70 (d, *J*=2.4 Hz, 1 H) 7.88 (d, *J*=1.7 Hz, 1 H); MS (DCI/NH₃) m/z 268 (M+1)⁺, 270 (M+1)⁺.

### Example 31B

### 5-(5-((1R,5S)-3-methyl-3,6-diazabicyclo[3.2.0]heptan-6-yl)pyridin-3-yl)-1H-indole bistosylate

The product of Example 31A (120 mg, 0.47 mmol) was coupled with *1H*-indol-5-ylboronic acid (Frontier, 160 mg, 1.0 mmol) according to the procedure of Example 14 to give the title compound (204.4 mg, 0.291 mmol, yield, 62%). ¹H NMR (300 MHz, CD₃OD) δ ppm 2.31 (s, 6 H), 3.10 (s, 3 H), 3.25 (dd, *J*=12.7, 3.6 Hz, 1 H), 3.30 - 3.38 (m, 1 H), 3.56 - 3.65 (m, 1 H), 3.98 - 4.06 (m, 2 H), 4.12 - 4.19 (m, 1 H), 4.24 (t, *J*=8.5 Hz, 1 H), 5.20 (dd, *J*=6.8, 3.4 Hz, 1 H), 6.58 (dd, *J*=3.2, 0.8 Hz, 1 H), 7.17 (d, *J*=8.1 Hz, 4 H), 7.36 (d, *J*=3.1 Hz, 1 H), 7.45 - 7.52 (m, 1 H), 7.53 - 7.59 (m, 1 H), 7.66 (d, *J*=8.5 Hz, 4 H), 7.80 (dd, *J*=2.4, 1.7 Hz, 1 H), 7.94 - 7.98 (m, 1 H), 7.99 (dd, *J*=2.0, 0.7 Hz, 1 H), 8.41 (d, *J*=0.7 Hz, 1 H); MS (DCI/NH₃) m/z 305(M+1)⁺. Anal. calcd. for C₁₉H₂₀N₄·2.10C₇H₈O₃S 1.30H₂O: C, 57.66; H, 5.86; N, 7.98. Found: C, 57.81; H, 5.72; N, 7.76.

### Example 32

### 6-{5-[(1R,5S)-3,6-diazabicyclo[3.2.0]heptan-6-yl]pyridin-3-yl}-1H-indole bistrifluoroacetate

The product of Example 30B (130 mg, 0.514 mmol) was coupled with *1H*-indol-6-ylboronic acid (Frontier, 158 mg, 1.0 mmol) according to the procedure described in Example 7B to give the title compound (139.2 mg, yield, 50.6%). ¹H NMR (300 MHz, CD₃OD) δ ppm 3.22 - 3.30 (m, 1 H), 3.39 (dd, *J*=12.4, 7.6 Hz, 1 H), 3.53 -3.62 (m, 1 H), 3.78 (d, *J*=12.2 Hz, 1 H), 3.86 - 3.97 (m, 2 H), 4.24 (t, *J*=8.3 Hz, 1 H), 5.16 (dd, *J*=6.4, 3.4 Hz, 1 H), 6.53 (dd, *J*=3.2, 0.8 Hz, 1 H), 7.35 - 7.41 (m, 2 H), 7.69 - 7.74 (m, 2 H), 7.75 - 7.78 (m, 1 H), 7.98 (d, *J*=2.4 Hz, 1 H), 8.42 (d, *J*=1.4 Hz, 1 H); MS (DCI/NH₃) m/z 291(M+1)⁺. Anal. calcd. for C₁₈H₁₈N₄ 2.15CF₃CO₂H: C, 50.02; H, 3.79; N, 10.46. Found: C, 50.02; H, 3.75; N, 10.50.

### Example 33

### 6-{5-[(1R,5S)-3-methyl-3,6-diazabicyclo[3.2.0]heptan-6-yl]pyridin-3-yl}-1H-indole bistosylate

The product of Example 31A (130 mg, 0.487 mmol) was coupled with *1H*-indol-6-ylboronic acid (Frontier, 160 mg, 1.0 mmol) according to the procedure of Example 14 to give the title compound (189.8 mg, 0.296 mmol, yield, 60.9%). ¹H NMR (300 MHz, CD₃OD) δ ppm 2.30 (s, 6 H), 3.10 (s, 3 H), 3.24 (dd, *J*=12.5, 3.4 Hz, 1 H), 3.30 - 3.37 (m, 1 H), 3.55 - 3.65 (m, 1 H), 3.97 - 4.04 (m, 2 H), 4.13 (d, *J*=12.5 Hz, 1 H), 4.21 (t, *J*=8.3 Hz, 1 H), 5.16 (dd, *J*=6.8, 3.4 Hz, 1 H), 6.51 - 6.54 (m, 1 H), 7.16 (d, *J*=7.8 Hz, 4 H), 7.35 - 7.40 (m, 2 H), 7.65 (d, *J*=8.1 Hz, 4 H), 7.69 - 7.74 (m, 2 H), 7.76 - 7.79 (m, 1 H), 7.95 (d, *J*=2.4 Hz, 1 H), 8.39 (d, *J*=1.7 Hz, 1 H); MS (DCI/NH₃) m/z 305(M+1)⁺. Anal. calcd. for C₁₉H₂₀N₄·1.70C₇H₈SO₃ 2.40H₂O: C, 57.96; H, 6.04; N, 8.75. Found: C, 57.82; H, 5.74; N, 8.71.

### Examples 34

### 4-(5-((1R,5S)-3-methyl-3,6-diazabicyclo[3.2.0]heptan-6-yl)pyridin-3-yl)-1H-indole tosylate

The product of Example 31A (140 mg, 0.52 mmol) was coupled with *1H*-indol-4-ylboronic acid (Frontier, 160 mg, 1.0 mmol) according to the procedure of Example 14 to give the title compound (132.3 mg, yield, 42.1%). ¹H NMR (300 MHz, CD₃OD) δ ppm 2.32 (s, 4.2 H), 3.10 (s, 3 H), 3.23 (dd, *J*=12.4, 3.6 Hz, 1 H), 3.31 - 3.37 (m, 1 H), 3.54 - 3.65 (m, 1 H), 3.94 - 4.04 (m, 2 H), 4.05 - 4.12 (m, 1 H), 4.21 (t, *J*=8.1 Hz, 1 H), 5.13 (dd, *J*=6.8, 3.4 Hz, 1 H), 6.60 (d, *J*=1.7 Hz, 1 H), 7.18 (d, *J*=7.8 Hz, 2.8 H), 7.20 - 7.29 (m, 2 H), 7.37 - 7.41 (m, 1 H), 7.53 (d, *J*=8.1 Hz, 1 H), 7.61 - 7.64 (m, 1 H), 7.67 (d, *J*=8.1 Hz, 2.8 H) 8.01 (d, *J*=2.7 Hz, 1 H), 8.37 (d, *J*=1.4 Hz, 1 H); MS (DCI/NH₃) m/z 305(M+1)⁺. Anal. calcd. for C₁₉H₂₀N₄ 1.39C₇H₈O₃S·2.62H₂O: C, 58.39; H, 6.20; N, 9.48. Found: C, 58.71; H, 5.98; N, 9.08.

### Example 35

### 6-{5-[(3aS,6aS)-5-methylhexahydropyrrolo[3,4-b]pyrrol-1(2H)-yl]pyridin-3-yl}-1H-indole bistrifluoroacetate

### Example 35A

### (3aS,6aS)-tert-butyl 1-(5-bromopyridin-3-yl)hexahydropyrrolo[3,4-b]pyrrole-5(1H)-carboxylate

3,5-Dibromopyridine (Aldrich, 2.82 g, 12 mmol) was coupled with (*3aS,6aS*)*-*tert-butyl hexahydropyrrolo[3,4-b]pyrrole-5(1H)-carboxylate (WO 2001081347, 2.12 g, 10 mmol) according to the procedure of Example 30A to give the title compound (2.75 g, yield, 74.5%). ¹H NMR (300 MHz, CD₃OD) δ ppm 1.42 (s, 9 H), 1.93 (td, *J*=12.7, 6.1 Hz, 1 H), 2.21 (td, *J*=13.2, 7.5 Hz, 1 H), 3.02 - 3.16 (m, 1 H), 3.33 - 3.42 (m, 3 H), 3.50 - 3.63 (m, 2 H), 3.67 (dd, *J*=11.9, 6.1 Hz, 1 H), 4.27 (td, *J*=6.7, 2.9 Hz, 1 H), 7.14 - 7.19 (m, 1 H), 7.84 (d, *J*=2.7 Hz, 1 H), 7.89 (d, *J*=2.0 Hz, 1 H); MS (DCI/NH₃) m/z 368 (M+1)⁺, 370 (M+1)⁺.

### Example 35B

### (3aS,6aS)-1-(5-bromopyridin-3-yl)-5-methyloctahydropyrrolo[3,4-b]pyrrole

The product of Example 35A (2.1 g, 5.7 mmol) was treated with formaldehyde (Aldrich, aq.37%, 4 mL) in formic acid (Aldrich, 10 mL) at 100°C for 2 h according to the procedure of Example 7A to give the title compound (1.4 g, yield, 87%). ¹H NMR (300 MHz, CD₃OD) δ ppm 1.92 - 2.05 (m, 1 H), 2.18 - 2.35 (m, 1 H), 2.57 (s, 3 H), 2.94 - 3.00 (m, 2 H), 3.00 - 3.10 (m, 2 H), 3.13 - 3.26 (m, 1 H), 3.35 (dd, *J*=7.8, 6.1 Hz, 1 H), 3.60 (ddd, *J*=9.3, 7.1, 7.0 Hz, 1 H), 4.32 (ddd, *J*=8.2, 5.3, 3.1 Hz, 1 H), 7.18 - 7,24 (m, 1 H), 7.88 (d, *J*=2.7 Hz, 1 H), 7.93 (d, *J*=1.7 Hz, 1 H); MS (DCI/NH₃) m/z 282 (M+1)⁺, 284 (M+1)⁺.

### Examples 35C

### 6-{5-[(3aS,6aS)-5-methylhexahydropyrrolo[3,4-b]pyrrol-1(2H)-yl]pyridin-3-yl}-1H-indole bistrifluoroacetate

The product of Example 35B (140 mg, 0.50 mmol) was coupled with *1H*-indol-6-ylboronic acid (Frontier, 165 mg, 1.04 mmol) according to the procedure described in Example 7B to give the title compound (173.7 mg, yield, 61.1%). ¹H NMR (300 MHz, CD₃OD) δ ppm 2.06 - 2.19 (m, 1 H), 2.31 - 2.49 (m, 1 H), 2.97 (s, 3 H), 3.37 - 3.97 (m, 7 H), 4.67 - 4.76 (m, 1 H), 6.54 (d, *J*=3.1 Hz, 1 H), 7.36 - 7.43 (m, 2 H), 7.73 (d, *J*=8.1 Hz, 1 H), 7.78 (s, 1 H), 7.81 (s, 1 H), 8.05 (d, *J*=2.7 Hz, 1 H), 8.41 (s, 1 H); MS (DCI/NH₃) m/z 319(M+1)⁺. Anal. calcd. for C₂₀H₂₂N₄·2.20CF₃CO₂H: C, 51.48; H, 4.28; N, 9.84. Found: C, 51.30; H, 4.35; N, 9.93.

### Example 36

### 5-{5-[(3aS,6aS)-5-methylhexahydropyrrolo[3,4-b]pyrrol-1(2H)-yl]pyridin-3-yl}-1H-indole bisfumarate

The product of Example 35B (140 mg, 0.50 mmol) was coupled with *1H*-indol-5-ylboronic acid (Frontier, 158 mg, 1.0 mmol) according to the procedure of Example 26 to give the title compound (124.2 mg, 0.214 mmol, yield, 42.8%). ¹H NMR (300 MHz, CD₃OD) δ ppm 1.98 - 2.14 (m, 1 H), 2.32 - 2.38 (m, 1 H), 2.91 (s, 3 H), 3.35 - 3.66 (m, 6 H), 3.75 - 3.86 (m, 1 H), 4.50 -4.58 (m, 1 H), 6.53 (dd, *J*=3.2, 0.8 Hz, 1 H), 6.71 (s, 4 H), 7.27 - 7.32 (m, 2 H), 7.36 - 7.43 (m, 1 H), 7.46 - 7.53 (m, 1 H), 7.83 (d, *J*=1.0 Hz, 1 H), 7.89 (d, *J*=2.7 Hz, 1 H), 8.23 (d, *J*=1.7 Hz, 1 H); MS (DCI/NH₃) m/z 319 (M+1)⁺. Anal. calcd. for C₂₀H₂₂N₄·2.10C₄O₄H₄·H₂O: C, 58.79; H, 5.63; N, 9.66. Found: C, 58.87; H, 5.76; N, 9.30.

### Compositions and Use of Compositions of the Invention

The invention also provides pharmaceutical compositions comprising a therapeutically effective amount of a compound of formula (I) in combination with a pharmaceutically acceptable carrier. The compositions comprise compounds of the invention formulated together with one or more pharmaceutically acceptable carriers. The compositions can be formulated for oral administration in solid or liquid form, for parenteral injection or for rectal administration.

A pharmaceutically acceptable carrier means a non-toxic, inert solid, semi-solid or liquid filler, diluent, encapsulating material or formulation auxiliary of any type. Some examples of materials that can serve as pharmaceutically acceptable carriers are: sugars, such as lactose, glucose and sucrose; starches, such as corn starch and potato starch; cellulose and its derivatives, such as sodium carboxymethyl cellulose, ethyl cellulose and cellulose acetate; powdered tragacanth; malt; gelatin; talc; cocoa butter and suppository waxes; oils, such as peanut oil, cottonseed oil, safflower oil, sesame oil, olive oil, corn oil and soybean oil; glycols, such a propylene glycol; esters, such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions. Other components, such as non-toxic compatible lubricants, such as sodium lauryl sulfate and magnesium stearate; coloring agents, releasing agents, coating agents, sweetening, flavoring and perfuming agents, preservatives and antioxidants can also be present in the composition.

The pharmaceutical compositions of the invention can be administered to humans and other mammals orally, rectally, parenterally, intracisternally, intravaginally, intraperitoneally, topically (as by powders, ointments or drops), bucally or as an oral or nasal spray. The term "parenterally," as used herein, refers to modes of administration, including intravenous, intramuscular, intraperitoneal, intrasternal, subcutaneous, intraarticular injection and infusion.

Pharmaceutical compositions for parenteral injection include pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like, and suitable mixtures thereof), vegetable oils (such as olive oil) and injectable organic esters, such as ethyl oleate, or suitable mixtures thereof. Suitable fluidity of the composition can be maintained, for example, by the use of a coating, such as lecithin; by the maintenance of the required particle size in the case of dispersions, and by the use of surfactants.

These compositions can also contain adjuvants, such as preservative agents, wetting agents, emulsifying agents, and dispersing agents. Prevention of the action of microorganisms can be ensured by various antibacterial and antifungal agents; for example, parabens, chlorobutanol, phenol, sorbic acid, and the like. It also can be desirable to include isotonic agents; for example, sugars, sodium chloride and the like. Prolonged absorption of the injectable pharmaceutical form can be brought about by the use of agents delaying absorption, for example, aluminum monostearate and gelatin.

In some cases, in order to prolong the effect of a drug, absorption can be slowed. This can be accomplished by the use of a liquid suspension of crystalline or amorphous material with poor water solubility. The rate of absorption of the drug can depend upon its rate of dissolution, which, in turn, can depend upon crystal size and crystalline form. Alternatively, dissolving or suspending the drug in an oil vehicle can administer a parenterally administered drug form.

Suspensions can contain the active compounds and suspending agents, such as ethoxylated isostearyl alcohols, polyoxyethylene sorbitol and sorbitan esters, microcrystalline cellulose, aluminum metahydroxide, bentonite, agar-agar, tragacanth, and mixtures thereof.

If desired, and for more effective distribution, the compounds of the invention can be incorporated into slow-release or targeted-delivery systems, such as polymer matrices, liposomes, and microspheres. They can be sterilized, for example, by filtration through a bacteria-retaining filter or by incorporation of sterilizing agents in the form of sterile solid compositions, which may be dissolved in sterile water or some other sterile injectable medium immediately before use.

Injectable depot forms are made by forming microencapsulated matrices of the drug in biodegradable polymers, such as polylactide-polyglycolide. Depending upon the ratio of drug to polymer and the nature of the particular polymer used, the rate of drug release can be controlled. Examples of other biodegradable polymers include poly(orthoesters) and poly(anhydrides). Depot injectable formulations also are prepared by entrapping the drug in liposomes or microemulsions that are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Injectable preparations, for example, sterile injectable aqueous or oleaginous suspensions, can be formulated using suitable dispersing or wetting agents and suspending agents. Sterile injectable preparations can be sterile injectable solutions, suspensions or emulsions in a nontoxic, parenterally acceptable diluent or solvent such as a solution in 1,3-butanediol. Among the acceptable vehicles and solvents that can be used are water, Ringer's solution, U.S.P. and isotonic sodium chloride solution. In addition, sterile, fixed oils are conventionally used as a solvent or suspending medium. For this purpose any bland fixed oil can be used including synthetic mono- or diglycerides. In addition, fatty acids, such as oleic acid can be used.

Solid dosage forms for oral administration include capsules, tablets, pills, powders, and granules. In such solid dosage forms, one or more compounds of the invention is mixed with at least one inert pharmaceutically acceptable carrier, such as sodium citrate or dicalcium phosphate and/or fillers or extenders, such as starches, lactose, sucrose, glucose, mannitol, and salicylic acid; binders, such as carboxymethylcellulose, alginates, gelatin, polyvinylpyrrolidinone, sucrose, and acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as cetyl alcohol and glycerol monostearate; absorbents, such as kaolin and bentonite clay; and lubricants, such as talc, calcium stearate, magnesium stearate, solid polyethylene glycols, sodium lauryl sulfate, and mixtures thereof. In the case of capsules, tablets and pills, the dosage form can also include buffering agents.

Solid compositions of a similar type can also be used as fillers in soft and hard-filled gelatin capsules using lactose or milk sugar, as well as high-molecular weight polyethylene glycols.

The solid dosage forms of tablets, dragees, capsules, pills, and granules can be prepared with coatings and shells, such as enteric coatings. They can optionally contain opacifying agents and can also be of a composition that they release the active ingredient(s) only, or preferentially, in a certain part of the intestinal tract in a delayed manner. Examples of materials useful for delaying release of the active agent include polymeric substances and waxes.

Compositions for rectal or vaginal administration are preferably suppositories that can be prepared by mixing the compounds of the invention with suitable non-irritating carriers, such as cocoa butter, polyethylene glycol or a suppository wax which are solid at ambient temperature but liquid at body temperature, and therefore melt in the rectum or vaginal cavity to release the active compound.

Liquid dosage forms for oral administration include pharmaceutically acceptable emulsions, microemulsions, solutions, suspensions, syrups and elixirs. In addition to the active compounds, the liquid dosage forms can contain inert diluents such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, dimethylformamide; oils, such as cottonseed, groundnut, corn, germ, olive, castor, and sesame oils; glycerol, tetrahydrofurfuryl alcohol, polyethylene glycols and fatty acid esters of sorbitan, and mixtures thereof.

Besides inert diluents, the oral compositions can also include adjuvants, such as wetting agents, emulsifying and suspending agents, sweetening, flavoring, and perfuming agents.

Dosage forms for topical or transdermal administration of a compound of the invention include ointments, pastes, creams, lotions, gels, powders, solutions, sprays, inhalants or patches. Ophthalmic formulation, eardrops, eye ointments, powders and solutions are also contemplated.

Ointments, pastes, creams and gels can contain, in addition to an active compound of the invention, animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of the invention, lactose, talc, silicic acid, aluminum hydroxide, calcium silicates and polyamide powder, or mixtures of these substances. Sprays can additionally contain customary propellant, such as chlorofluorohydrocarbons.

Compounds of the invention can be administered as liposomes. Liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to the compounds of the invention, stabilizers, preservatives, and the like. The preferred lipids are the natural and synthetic phospholipids and phosphatidylcholines (lecithins) used separately or together. Methods to form liposomes are known in the art. See, for example, Prescott, Ed., Methods in Cell Biology, Volume XIV, Academic Press, New York, N. Y., (1976), p 33 *et seq.*

### Determination of Biological Activity

To determine the effectiveness of representative compounds of the invention as α7 nAChR, modulators, the compounds were evaluated according to the [³H]-methyllycaconitine (MLA) binding assay the [³H]-DPPB binding assay, and/or the [³H]-cytisine binding assay, which were performed as described below.

### [³H]-Cytisine binding

Compounds of the invention were analyzed for their ability to compete for α4β2 nAChRs by co-incubating a test compound with a known α4β2 nAChR ligand, cytisine. Binding conditions were modified from the procedures described in Pabreza LA, Dhawan, S, Kellar KJ, [3H]-Cytisine Binding to Nicotinic Cholinergic Receptors in Brain, Mol. Pharm. 39: 9-12, 1991. Membrane enriched fractions from rat brain minus cerebellum (ABS Inc., Wilmington, DE) were slowly thawed at 4 °C, washed and resuspended in 30 volumes of BSS-Tris buffer (120 mM NaCl/5 mM KCl/2 mM CaCl₂/2 mM MgCl₂/50 mM Tris-Cl, pH 7.4, 4 °C). Samples containing 100-200 µg of protein and 0.75 nM [³H]-cytisine (30 Cᵢ/mmol; Perkin Elmer/NEN Life Science Products, Boston, MA) were incubated in a final volume of 500 µL for 75 minutes at 4 °C. Seven log-dilution concentrations of each compound were tested in duplicate. Non-specific binding was determined in the presence of 10 µM (-)-nicotine. Bound radioactivity was isolated by vacuum filtration onto prewetted glass fiber filter plates (Millipore, Bedford, MA) using a 96-well filtration apparatus (Packard Instruments, Meriden, CT) and were then rapidly rinsed with 2 mL of ice-cold BSS buffer (120 mM NaCl/5 mM KCl/2 mM CaCl₂/2 mM MgCl₂). PACKARD MICROSCINT-20^{®} scintillation cocktail (40 µL) was added to each well and radioactivity determined using a PACKARD TOPCOUNT^{®} instrument. The IC₅₀ values were determined by nonlinear regression in MICROSOFT EXCEL^{®} software. Kᵢ values were calculated from the IC₅₀s using the Cheng-Prusoff equation, where Kᵢ = IC₅₀/(1+[Ligand]/K_{D}).

### [³H]-methyllycaconitine (MLA) binding

Compounds of the invention were analyzed for their ability to compete for α7 nAChRs by co-incubating a test compound with a known α7 nAChR ligand, MLA. Binding conditions were similar to those for [³H]-cytisine binding. Membrane enriched fractions from rat brain minus cerebellum (ABS Inc., Wilmington, DE) were slowly thawed at 4 °C, washed and resuspended in 30 volumes of BSS-Tris buffer (120 mM NaCl, 5 mM KCl, 2 mM CaCl₂, 2 mM MgCl₂, and 50 mM Tris-Cl, pH 7.4, 22 °C). Samples containing 100-200 µg of protein, 5 nM [³H]-MLA (25 Cᵢ/mmol; Perkin Elmer/NEN Life Science Products, Boston, MA) and 0.1% bovine serum albumin (BSA, Millipore, Bedford, MA) were incubated in a final volume of 500 µL for 60 minutes at 22 °C. Seven log-dilution concentrations of each compound were tested in duplicate. Non-specific binding was determined in the presence of 10 µM MLA. Bound radioactivity was isolated by vacuum filtration onto glass fiber filter plates prowetted with 2% BSA using a 96-well filtration apparatus (Packard Instrument, Meriden, CT) and were then rapidly rinsed with 2 mL of ice-cold BSS. Packard MICROSCINT-20^{®} scintillation cocktail (40 µL) was added to each well and radioactivity was determined using a Packard TOPCOUNT^{®} instrument. The IC₅₀ values were determined by nonlinear regression in Microsoft EXCEL^{®} software. Kᵢ values were calculated from the IC₅₀s using the Cheng-Prusoff equation, where Kᵢ = TC₅₀/(1+[Ligand]/K_{D}).

### [³H]-DPPB binding

Compounds of the invention were analyzed for their ability to compete for α7 nAChRs by co-incubating a test compound with the known α7 nAChR ligand, DPPB, which is (S,S)-2,2-dimethyl-5-(6-phenyl-pyridazin-3-yl)-5-aza-2-azonia-bicyclo[2.2.1]heptane iodide. Procedures for preparing radiolabeled DPPB, [³H]-DPPB, are described below. Binding to the α7 nAChR subtype was determined using membrane enriched fractions from rat brain minus cerebellum or human cortex (ABS Inc., Wilmington, DE). Pellets were thawed at 4°C, washed and resuspended with a Polytron at a setting of 7 in 30 volumes of BSS-Tris buffer (120 mM NaCl, 5 mM KCl, 2 mM CaCl₂, 2 mM MgCl₂, and 50 mM Tris-Cl, pH 7.4, 4°C). Seven log-dilution concentrations of test compounds containing 100-200 µg of protein, and 0.5 nM [³H]-DPPB (62.8 Ci/mmol; R46V, Abbott Labs) were incubated in a final volume of 500 µl for 75 minutes at 4°C in duplicate. Non-specific binding was determined in the presence of 10 µM methyllycaconitine. Bound radioactivity was collected on Millipore MULTISCREEN^{®} harvest plates FB presoaked with 0.3% PEI using a Packard cell harvester, washed with 2.5 ml ice-cold buffer, and radioactivity determined using a Packard TOPCOHNT^{®} Microplate beta counter. IC₅₀ values were determined by nonlinear regression in Microsoft^{®} Excel or Assay Explorer. Kᵢ values were calculated from the IC₅₀s using the Cheng-Prusoff equation, where Kᵢ = IC₅₀/(1+[Ligand]/K_{D}). [³H]-DPPB was obtained according to the following preparation procedure.

### Preparation of [Methyl-³H]2,2-Dimethyl-5-(6-phenyl-pyridazin-3-yl)-5-aza-2-azonia-bicyclo[2.2.1]heptane iodide

[Methyl-³H]2,2-dimethyl-5-(6-phenyl-pyridazin-3-yl)-5-aza-2-azonia-bicyclo[2.2.1]heptane iodide used in the [³H]-DPPB binding assay above was prepared according to the following procedures.

### Step 1: Preparation of t-butyl (S,S)-5-(6-Phenyl-pyridazin-3-yl)-2,5-diaza-bicyclo[2.2.1]heptane-2-carboxylate

Triethylamine (20 mL) was added to a suspension of t-butyl (S,S)-2,5-diazabicyclo[2.2.1]heptane-2-carboxylate (3.43 g, 17.3 mmol, Aldrich Chemical Company) and 3-chloro-6-phenylpyridazine (3.30 g, 17.3 mmol, Aldrich Chemical Company) in toluene (50 mL), and the mixture was heated under nitrogen at 100°C for 7 days. The dark mixture was cooled to room temperature, and the resulting precipitate was isolated by filtration, washed with toluene (15 mL) and dried under vacuum to provide the title compound as an off-white solid (3.00 g). The filtrate was concentrated, and the residue was purified by column chromatography on silica gel, eluting with ethyl acetate, to provide additional product (0.41 g, total yield 3.41 g, 56%): MS (DCI/NH₃) m/z 353 (M+H)⁺.

### Step 2: Preparation of (S,S)-2-methyl 5-(6-phenyl-pyridazin-3-yl)-2,5-diaza-bicyclo[2.2.1]heptane

The product obtained from Step 1 (3.41 g, 9.7 mmol) was dissolved in formic acid (20 mL) and treated with formalin (37% by weight, 1.0 g, 12.3 mmol). The mixture was heated at 100°C for 1 hour, and the brown solution was cooled to room temperature and concentrated under vacuum. The residue was purified by column chromatography on silica gel, eluting with CH₂Cl₂ - CH₃OH - NH₄OH (95:5:1) to provide the title compound as an off-white solid (2.50 g, 96%): MS (DCI/NH₃) m/z 267 (M+H)⁺.

### Step 3: Preparation of [³H]-(S,S)-2,2-Dimethyl-5-(6-phenyl-pyridazin-3-yl)-5-aza-2-azonia-bicyclo[2.2.1]heptane iodide ([³H]-DPPB)

[³H]Methyl iodide in toluene (250 mCi in 0.1 mL, 85Ci/mmol, American Radiolabeled Chemicals, Inc.) was combined with a solution of the product obtained from Step 2 in dichloromethane (0.788 mg, 2.96 µ mole in 0.45 mL). The vial was capped and the mixture was allowed to react overnight at room temperature. Methanol was added, and the solvents were evaporated to give 42 mCi. The product was taken up in methanol for HPLC purification.

### Step 4: Purification by High Performance Liquid Chromatography (HPLC)

About 7 mCi of [³H]-DPPB was evaporated to dryness, and the residue was dissolved in total about 4.5 ml acetonitrile:water:TFA (15:85:0.1). Approximately 0.9 mL per injection was made onto a PhenomenexLuna C18(2) column (5 µm, 250 mm x 4.6 mm ID) using an Agilent HPLC system. [³H]-DPPB was eluted by a gradient mobile phase from 10% B to 20% B in 20 min where Mobile Phase A= 0.1% trifluoroacetic acid in water and Mobile Phase B= 0.1% trifluoroacetic acid in acetonitrile at a flow rate of approximately 1 mL/min. Peak detection and chromatograms were obtained with an Agilent variable wavelength UV detector set at 275 nm. The fractions containing [³H]-DPPB were collected at approximately 14 minutes using an Agilent fraction collector. The fractions were combined and the solvents were evaporated in vacuo. The residue was dissolved in 200 proof ethanol (2 mL) to give 0.7 mCi.

### Step 5: Determination of Purity and Specific Activity

[³H]-DPPB was assayed using an Agilent 1100 series HPLC system consisting of a quaternary pump, an autosampler, and a photodiode array UV detector. A Packard Radiomatic A 500 radioactivity detector was connected to the HPLC system. For radiodetection, a 500 µL flow cell and a 3:1 ratio of Ultima-Flo M scintillation cocktail to HPLC mobile phase were used. The analyses were performed using a Phenomenex Luna C18(2) column (5 µm, 250 mm x 4.6 mm ID). The mobile phase consisted of a gradient starting with 10% B and ramping to 20% B in 20 minutes followed by ramping to 90% B in 1 minute and hold at 90% B for 9 minutes, where Mobile Phase A = 0.1% trifluoroacetic acid in water and Mobile Phase B= 0.1% trifluoroacetic acid in acetonitrile. The flow rate was set at approximately 1 mL/min and the UV detection was set at 275 nm.

Compounds of the invention had Kᵢ values of from about 1 nanomolar to about 10 micromolar when tested by the [³H]-MLA assay, many having a Kᵢ of less than 1 micromolar. [³H]-cytisine binding values of compounds of the invention ranged from about 50 nanomolar to at least 100 micromolar. Preferred compounds typically exhibited greater potency at α7 receptors compared to α4β2 receptors. The determination of preferred compounds typically considered the Kᵢ value as measured by MLA assay in view of the Kᵢ value as measured by [³H]-cytisine binding, such that in the formula D = Kᵢ ³_{H-cytisine} / K_{i MLA}, D is greater than about 50. Alternatively, the Kᵢ value as measured by [³H]-DPPB assay can be used in place of the Kᵢ MLA such that in the formula D' = Kᵢ ³_{H-cytisine} / K_{i [3H]}-_{DPPB}, D' is greater than about 50.

Compounds of the invention are α7 nAChRs ligands that modulate function of α7 nAChRs by altering the activity of the receptor or signaling. The compounds can be inverse agonists that inhibit the basal activity of the receptor or antagonists that completely block the action of receptor-activating agonists. The compounds also can be partial agonists that partially block or partially activate the α7 nAChR receptor or agonists that activate the receptor. Binding to α7 receptor also trigger key signaling processes involving various kinases and phosphatases and protein-protein interactions that are important to effects on memory, cytoprotection, gene transcription and disease modification.

### Methods of the invention

Compounds and compositions of the invention are useful for modulating the effects of nAChRs, and more particularly α7 nAChRs and α4β2 nAChRs. In particular, the compounds and compositions of the invention can be used for treating and preventing disorders modulated by α7 nAChRs. Typically, such disorders can be ameliorated by selectively modulating the α7 nAChRs in an animal, such as a human, preferably by administering a compound or composition of the invention, either alone or in combination with another active agent, for example, as part of a therapeutic regimen. Also, some compounds of the invention possess affinity at the α4β2 nAChRs in addition to α7 nAChRs, and selective compounds with dual affinities at both receptor subtypes have beneficial effects.

### Conditions, diseases and disorders

Because α7-containing nAChRs have been shown to be involved in the neuroprotective effects of nicotine both *in vitro* and *in vivo,* the compounds of the invention can be used to treat ncurodegeneration that underlies several progressive CNS disorders, such as Alzheimer's disease, Parkinson's disease, amyotrophic lateral sclerosis, Huntington's disease, dementia with Lewy bodies, as well as diminished CNS function resulting from traumatic brain injury. Compounds that activate α7 nAChRs can be used to counter the deficits of Alzheimer's and other neurodegenerative diseases.

Thus, α7 ligands can be used in the treatment schizophrenia. Activators of α7 receptors are useful for enhancing cognitive function in schizophrenic patients who are being treated with atypical antipsychotics. Accordingly, the combination of a α7 nAChR ligand and an atypical antipsychotic offer improved therapeutic utility. Specific examples of suitable atypical antipsychotics include, but are not limited to, clozapine, risperidone, olanzapine, quietapine, ziprasidone, zotepine, iloperidone, and the like.

Because improved angiogenesis has been shown to involve activation of the α7 nAChR, nAChR ligands that are selective for the α7 subtype can be used for stimulating angiogenesis with an improved side effect profile.

α7 nAChR ligands can be used to treat pain, including acute pain, post-surgical pain, as well as chronic pain states including inflammatory pain and neuropathic pain. They can also be used for treating conditions involving TNF-mediated diseases; for example, rheumatoid arthritis, Crohn's disease, ulcerative colitis, inflammatory bowel disease, organ transplant rejection, acute immune disease associated with organ transplantation, chronic immune disease associated with organ transplantation, septic shock, toxic shock syndrome, sepsis syndrome, depression, and rheumatoid spondylitis.

Because activation of a α7 nAChR on the sperm cell has been shown to be essential for the acrosome reaction, selective α7 agents of the invention can be used to treat fertility disorders.

Compounds of the invention are α7 nAChRs ligands that modulate function of α7 nAChRs by altering the activity of the receptor or signaling. The compounds can be inverse agonists that inhibit the basal activity of the receptor or antagonists that completely block the action of receptor-activating agonists. The compounds also can be partial agonists that partially block or partially activate the α7 nAChR receptor or agonists that activate the receptor. Binding to an α7 receptor also triggers key signaling processes involving various kinases and phosphatases and protein-protein interactions that are important to effects on memory, cytoprotection, gene transcription and disease modification. Therefore, the administration of a therapeutically effective amount of a compound of formula (I) to a mammal provides a method of selectively modulating the effects of α4β2, α7, or both α4β2 and α7 nicotinic acetylcholine receptors.

Nicotinic receptor modulation of dopine transmission has been identified as an important mechanism underlying various forms of substance abuse, including for example, smoking cessation, alcohol addition, cannibis addiction, and other forms of substance abuse. (Rose, J.E., Biochem Pharmacol., 74(8): 1263-1270, 2007; Rollema H., Coe J.W., Chambers L.K., Hurst R.S., Stahl S.M., Williams K.E., Trends Pharmacol Sci., 28(7): 316-25, 2007; Steensland P., Simms J.A., Holgate J., Richards J.K., Bartlett S.E., Proc Nat'l Acad Sci U.S.A., 104(30):12518-23, 2007; and Scherma M., Fattor Le., Stoik J., Wertheim C., Tanda G., Fratta W., Goldberg S.R., 27(21):5615-20, 2007). For example, nicotinic receptors including α4β2 and α7 nAChRs are present in brain pathways implicated in addiction. Accordingly, a method of selectively modulating the effects of α4β2, α7, or both α4β2 and α7 nicotinic acetylcholine receptors would be useful in treating or preventing substance abuse.

Therefore, the administration of a therapeutically effective amount of a compound of formula (I) to a mammal provides a method of treating or preventing a condition or disorder selected from the group consisting of attention deficit disorder, attention deficit hyperactivity disorder (ADHD), Alzheimer's disease (AD), mild cognitive impairment, senile dementia, AIDS dementia, Pick's Disease, dementia associated with Lewy bodies, dementia associated with Down's syndrome, amyotrophic lateral sclerosis, Huntington's disease, diminished CNS function associated with traumatic brain injury, acute pain, post-surgical pain, chronic pain, inflammatory pain, neuropathic pain, infertility, need for new blood vessel growth associated with wound healing, need for new blood vessel growth associated with vascularization of skin grafts, and lack of circulation, more particularly circulation around a vascular occlusion, rheumatoid arthritis, Crohn's disease, ulcerative colitis, inflammatory bowel disease, organ transplant rejection, acute immune disease associated with organ transplantation, chronic immune disease associated with organ transplantation, septic shock, toxic shock syndrome, sepsis syndrome, depression, rheumatoid spondylitis, and substance abuse, More prefered, the administration of a therapeutically effective amount of a compound of formula (I) to a mammal provides a method of treating cognitive disorders, neurodegeneration, and schizophrenia.

The compounds of the invention can be administered with other medications, either simultaneously, in combined formulations, or in a regimen where the compounds are administered separately. In addition to the atypical psychotics listed previously, the compounds of the invention can be administered in combination with other compounds that treat attention deficit hyperactivity disorder, such as dextroamphetamine, levoamphetamine, dextrothreomethylphenidate, levothreomethylphenidate, amantadine, amineptine, benzphetamine, bupropion, clonidine, modafinil, pemoline, selegiline, and milnacipran; with compounds that treat Alzheimer's disease, such as acetylcholinesterase inhibitors (e.g., tacrine, donepezil, galanthamine and rivastigmine) and memantine and other NMDA antagonists.

### Administration - dosage

Actual dosage levels of active ingredients in the pharmaceutical compositions of the invention can be varied so as to obtain an amount of the active compound(s) that is effective to achieve the desired therapeutic response for a particular patient, compositions and mode of administration. The selected dosage level depends upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

When used in the above or other treatments, a therapeutically effective amount of one of the compounds of the invention can be used in pure form or, where such forms exist, in pharmaceutically acceptable salt or prodrug form. Alternatively, the compound can be administered as a pharmaceutical composition containing the compound of interest in combination with one or more pharmaceutically acceptable carriers. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the invention decided by a treating physician within the scope of sound medical judgment.

The total daily dose of the compounds of the invention administered to a human or lower animal range from about 0.010 mg/kg body weight to about 1 g/kg body weight. More preferable doses can be in the range of from about 0.010 mg/kg body weight to about 100 mg/kg body weight. If desired, the effective daily dose can be divided into multiple doses for purposes of administration. Single dose compositions can contain such amounts or submultiples thereof to make the daily dose.

It is understood that this detailed description and accompanying examples are merely illustrative and not limitations on the scope of the invention, which is defined solely by the appended claims and their equivalents. Various changes and modifications to the disclosed embodiments will be apparent to those skilled in the art. Such changes and modifications, including without limitation those relating to the chemical structures, substituents, derivatives, intermediates, syntheses, formulations and/or methods of use of the invention, can be made without departing from the spirit and scope thereof.

In addition, the following items are included in the present invention:
1. A compound of formula (I) 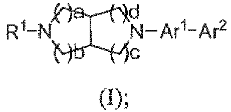 or a pharmaceutically acceptable salt or prodrug thereof, wherein
   R¹ is selected from group consisting of hydrogen, alkyl, cyclic alkyl, haloalkyl, aiyl, and heteroaryl;
   a and c are each independently selected from 0, 1, 2; b and d are each independently selected from 1, 2, or 3; provided that when both b and d are 1, a and c can not be I simultaneously;
   Ar¹ is selected from 5- or 6-membered aromatic group of formula: 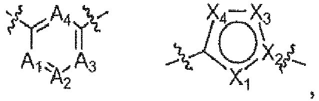 wherein
   A₁, A₂, A₃ and A₄ are each -N- or -CR^{a};
   X₁, X₃, X₄ are independently selected from group consisting of -CR^{a}, - NR^{a}, -O-, and -S-;
   X₂ is -C- or -N-, provided that when X₂ is -C-, at least one of X₁, X₃, X₄ is other than -C-;
   R^{a} is selected from group consisting of hydrogen, alkyl, cyclic alkyl, haloalkyl, aryl, heteroaryl, halogen, -CO₂R¹, -COR¹, -CONR¹, -OR¹, and -NR¹;
   Ar² is a fused bicyclic aromatic group of formula 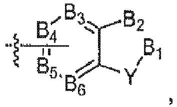 wherein
   B₁, B₂, B₃, B₄, B₅, B₆ are each independently -N- or -CR^{a}-;
   Y is selected from group consisting of -NR^{d}-, -O- and -S-;
   R^{a} is selected from group consisting of hydrogen, alkyl, cyclic alkyl, haloalkyl, aryl, heteroaryl, halogen, -CO₂R¹, -COR¹, -CONR¹, -OR¹, and -NR¹; and
   R^{d} is selected from group consisting of hydrogen, alkyl, and cyclic alkyl.
2. The compound of item 1, wherein the fused diazabicycloalkane moiety is selected from the group consisting of 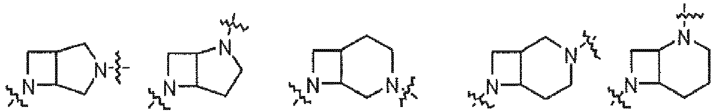 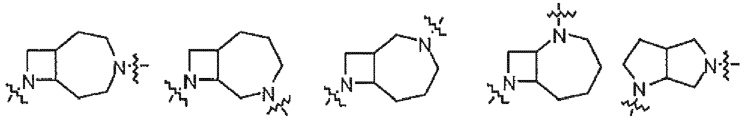 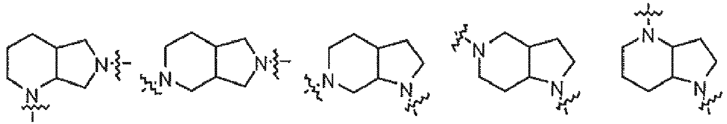 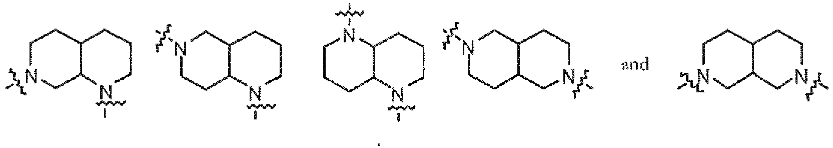
3. The compound of item 1, wherein Ar¹ is selected from the group consisting of midazolyl, isoxazolyl, isothiazolyl, furyl, oxazolyl, 1,2,4-oxadiazolyl, 1,3,4-oxadiazolyl, phenyl, pyrazolyl, pyridinyl, pyrimidinyl, pyridazinyl, pyrazinyl, thiophenyl, thiazolyl, 1,2,4-thiadiazolyt, and 1,3,4-thiadiazolyl.
4. The compound of item 3, wherein Ar¹ is selected from the group consisting of pyridazinyl, pyridinyl, thiazolyl, 1,3,4-thiadiazolyl, and 1,3,4-oxadiazolyl, wherein Ar¹ is substituted with 0, 1, or 2 substitutents selected from alkoxy, alkyl, cyano, haloalkyl, hydroxy, halogen and NR¹.
5. The compound of item 1, wherein Ar² is selected from the group consisting of benzofuranyl, benzo[*d*]imidazolyl, benzo[*d*]isoxazolyl, benzo[*d*]isothiazolyl, benzo[*d*]oxazolyl, benzo[*d*]thiazolyl, benzo[*b*]thiophenyl, furo[3,2-*b*]pyridinyl, furo[3,2-*c*]pyridinyl, imidazo[4,5-*b*]pyridinyl, imidazo[4,5-*c*]pyridine, indolyl, indazolyl, isoxazolo[4,5-*b*]pyridinyl, isoxazolo[4,5-*c*]pyridinyl, isoxazolo[5,4-*b*]pyridinyl, isoxazolo[5,4-*c*]pyridinyl, isothiazolo[4,5-*c*]pyridinyl, isothiazolo[4,5-*c*]pyridinyl, isothiazolo[5,4-*b*]pyridinyl, isothiazolo[5,4-*c*]pyridinyl, oxazolo[4,5-*b*]pyridinyl, oxazolo[4,5-*c*]pyridinyl, oxazolo[5,4-*b*]pyridinyl, oxazolo[5,4-*c*]pyridinyl, pyrazolo[3,4-*b*]pyridinyl, pyrazolo[3,4-*c*]pyridinyl, pyrazolo[4,3-*b*]pyridinyl, pyrazolo[4,3-*c*]pyridinyl, pyrrolo[2,3-*b*]pyridinyl, pyrrolo[2,3-*c*]pyridinyl, pyrrolo[3,2-*b*]pyridinyl, pyrrolo[3,2-*c*]pyridinyl, thiazolo[4,5-*b*]pyridinyl, thiazolo[4,5-*c*]pyridinyl, thiazolo[5,4-*b*]pyridinyl, thiazolo[5,4-*c*]pyridinyl, thieno[2,3-*b*]pyridinyl, thieno[2,3-*c*]pyridinyl, thieno[3,2-*b*]pyridinyl, and thieno[3,2-*c*]pyridinyl.
6. The compound of item 2, wherein the fused diazabicycloalkane moiety is 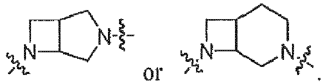
7. The compound of item 3, wherein Ar¹ is selected from the group consisting of 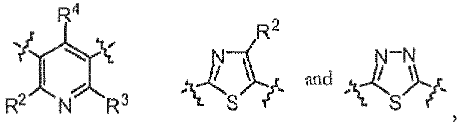 wherein
   R², R³ and R⁴ are independently selected from the group consisting of alkoxy, alkyl, cyano, haloalkyl, hydroxy, halogen and NR¹.
8. The compound of item 4, wherein Ar² is selected from the group consisting of 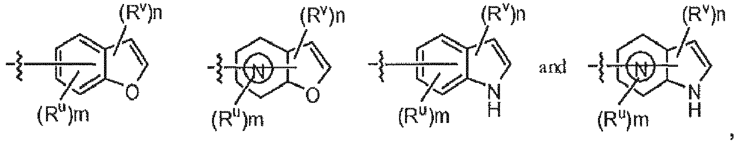 wherein
   R^{u} and R^{v} are each independently selected from the group consisting of alkoxy, alkyl, cyano, haloalkyl, hydroxy, halogen and NR¹; and
   m and n arc arc each independently selected from the group consisting of 0, 1 and 2.
9. The compound of item I, wherein the compound is one selected from the group consisting of
   5-{5-[(*1S,5S*)-3,6-diazabicyclo[3.2.0]heptan-3-yl]pyridin-3-yl}-1H-indole;
   5-{5-[(*1S,5S*)-6-methyl-3,6-diazabicyclo[3.2.0]heptan-3-yl]pyridin-3-yl}-*1H*-indole;
   4-{5-[(*1S,5S*)-3,6-diazabicyclo[3.2.0]heptan-3-yl]pyridin-3-yl}-*1H*-indole;
   4-{5-[(*1S,5S*)-6-methyl-3,6-diazabicyclo[3.2.0]heptan-3-yl]pyridin-3-yl}-*1H*-indole;
   6-{5-[(*1S,5S*)-3,6-diazabicyclo[3.2.0]heptan-3-yl]pyridin-3-yl}-*1H*-indole;
   6-{5-[(*1S,5S*)-6-methyl-3,6-diazabicyclo[3.2.0]heptan-3-yl]pyridin-3-yl}-*1H*-indole;
   5-{5-[(*1S,5S*)-6-methyl-3,6-diazabicyclo[3.2.0]heptan-3-yl]pyridin-3-yl}-2-(trifluoromethyl)-1*H*-indole;
   (*1S,5S*)-3-(5-(benzofuran-5-yl)pyridin-3-yl)-6-methyl-3,6-diazabicyclo[3.2.0]heptane;
   5-{5-[(*1S,5S*)-6-methyl-3,6-diazabicyclo[3.2.0]heptan-3-yl]pyridin-3-yl}-*1H-*indazole;
   (*1S,5S*)-3-[5-(benzo[b]thiophen-5-yl)pyridin-3-yl]-3,6-diazabicyclo[3.2.0]heptane;
   (*1S,5S*)-3-[5-(benzo[b]thiophen-5-yl)pyridin-3-yl]-3,6-diazabicyclo[3.2.0]heptane;
   7-{5-[(1*S,*5*S*)-6-methyl-3,6-diazabicyclo[3.2.0]heptan-3-yl]pyridin-3-yl}-1H-indole;
   5-{5-[(*1S,5S*)-6-methyl-3,6-diazabicyclo[3.2.0]heptan-3-yl]pyridin-3-yl}-1H-benzo[d]imidazole;
   3-methyl-5-{5-[(*1S,5S*)-6-methyl-3,6-diazabicyclo[3.2.0]heptan-3-yl]pyridin-3-yl}-1*H*-indole;
   3-{5-[(*1S,5S*)-3,6-diazabicyclo[3.2.0]heptan-3-yl]pyridin-3-yl}-9*H*-carbazole;
   5-{5-[(*1S,5S*)-3,6-diazabicyclo[3.2.0]heptan-3-yl]pyridin-3-yl}-3-methyl-1*H*-indole;
   3-(5-((*1S,5S*)-6-methyl-3,6-diazabicyclo[3.2.0]heptan-3-yl)pyridin-3-yl)-9*H-*carbazole;
   7-{5-[(*1S,5S*)-6-methyl-3,6-diazabicyclo[3.2.0]heptan-3-yl]pyridin-3-yl}-*1H-*pyrrolo[2,3-c]pyridine;
   5-{5-[(*1S,5S*)-6-methyl-3,6-diazabicyclo[3.2.0]heptan-3-yl]pyridin-3-yl}-1H-pyrrolo[2,3-b]pyridine;
   3-{5-[(*1S,5S*)-6-methyl-3,6-diazabicyclo[3.2.0]heptan-3-yl]pyridin-3-yl}-1-(phenylsulfonyl)-1*H*-indole;
   3-(5-((*1S*,*5S*)-6-methyl-3,6-diazabicyclo[3.2.0]heptan-3-yl)pyridin-3-yl)-1*H*-indole;
   4-{6-[(*1S,5S*)-3,6-diazabicyclo[3.2.0]heptan-3-yl]pyrazin-2-yl}-*1H*-indole;
   4-{6-[(*1S,5S*)-6-methyl-3,6-diazabicyclo[3.2.0]heptan-3-yl]pyrazin-2-yl}-*1H*-indole;
   5-{6-[(*1S,5S*)-6-methyl-3,6-diazabicyclo[3.2.0]heptan-3-yl]pyrazin-2-yl}-*1H*-indole;
   6-{6-[(*1S,5S*)-6-methyl-3,6-diazabicyclo[3.2.0]heptan-3-yl]pyrazin-2-yl}-1*H*-indole;
   5-(6-((*1S,5S*)-6-methyl-3,6-diazabicyclo[3.2.0]heptan-3-yl)pyrazin-2-yl)-2-(trifluoromethyl)-1*H*-indole
   5-{5-[(*1R,5R*)-6-methyl-3,6-diazabicyclo[3.2.0]heptan-3-yl]pyridin-3-yl}-1H-indole;
   4- {5-[(*1R,5R*)-6-methyl-3,6-diazabicyclo[3.2.0]heptan-3-yl]pyridin-3-yl}-1H-indole;
   6-{5-[(*1R,5R*)-6-methyl-3,6-diazabicyclo[3.2.0]heptan-3-yl]pyridin-3-yl}-1H-indole;
   5-{5-[(*1R,5S*)-3,6-diazabicyclo[3.2.0]heptan-6-yl]pyridin-3-yl}-1*H*-indole;
   5-{5-[(*1R,5S*)-3-methyl-3,6-diazabicyclo[3.2.0]heptan-6-yl]pyridin-3-yl}-1*H*-indole;
   6-{5-[(*1R,5S*)-3,6-diazabicyclo[3.2.0]heptan-6-yl]pyridin-3-yl}-1*H*-indole;
   6-{5-[(*1R,5S*)-3-methyl-3,6-diazabicyclo[3.2.0]heptan-6-yl]pyridin-3-yl}-1*H*-indole;
   6-(5-((*1R,5S*)-3-methyl-3,6-diazabicyclo[3.2.0]heptan-6-yl]pyridin-3-yl)-1*H*-indole;
   6- {5-[(*3aS*,*6aS*)-5-methylhexahydropyrrolo[3,4-b]pyrrol-1(2H)-yl]pyridin-3-yl}-1H-indole; and
   5-{5-[(*3aS,6aS*)-5-methylhexahydropyrrolo[3,4-b]pyrrol-1(2H)-yl]pyridin-3-yl}-1*H-*indole.
10. A pharmaceutical composition comprising a therapeutically effective amount of a compound of item 1 and a pharmaceutically acceptable carrier.
11. A method of selectively modulating an α7 nicotinic acetylcholine receptors, α4β2 nicotinic acetylcholine receptors, or both α7 and α4β2 nicotinic acetylcholine receptors in a mammal comprising administering an effective amount of a compound of item 1.
12. The method of item 11, wherein the compound is an agonist of at least one α7 or α4β2 nicotinic acetylcholine receptor.
13. A method of treating an α7 and α4β2 nicotinic acetylcholine receptor-mediated condition or disorder of a subject comprising administering a compound of item 1 to the subject.
14. The method of item 13, wherein the α7 and α4β2 nicotinic acetylcholine receptor-mediated condition or disorder is selected from the group consisting of attention deficit disorder, attention deficit hyperactivity disorder, Alzheimer's disease, mild cognitive impairment, senile dementia, AIDS dementia, Pick's Disease, dementia associated with Lcwy bodies, dementia associated with Down's syndrome, amyotrophic lateral sclerosis, Huntington's disease, diminished CNS function associated with traumatic brain injury, acute pain, post-surgical pain, chronic pain, inflammation, inflammatory pain, neuropathic pain, infertility, need for new blood vessel growth associated with wound healing, need for new blood vessel growth associated with vascularization of skin grafts, and lack of circulation, rheumatoid arthritis, Crohn's disease, ulcerative colitis, inflammatory bowel disease, organ transplant rejection, acute immune disease associated with organ transplantation, chronic immune disease associated with organ transplantation, septic shock, toxic shock syndrome, sepsis syndrome, depression, rheumatoid spondylitis, and substance abuse.
15. The method according to item 14, wherein the α7 and α4β2 nicotinic acetylcholine receptor-mediated condition or disorder is an α7 nicotinic acetylcholine receptor-mediated condition or disorder, and is selected from the group consisting of a cognitive disorder, neurodegeneration, and schizophrenia.
16. The method according to item 15, wherein the compound is an agonist of at least one α7 nicotinic acetylcholine receptor, and wherein the method further comprises administering an atypical antipsychotic.
17. The method of item 16, wherein the atypical antipsychotic is at least one selected from the group consisting of clozapine, risperidone, olanzapine, quietapine, ziprasidone, zotepine, and iloperidone.
18. The method of item 11, further comprising administering a compound of item 1 with a second composition to treat a cognitive disorder.
19. The method of item 18, wherein the cognitive disorder is attention deficit disorder, and the second composition comprises at least one compound selected from the group consisting of dextroamphetamine, levoamphetamine, dextrothreomethylphenidate, levothreomethylphenidate, amantadine, amineptine, benzphetamine, bupropion, clonidine, modafinil, pemoline, selegiline, and milnacipran.
20. The method of item 18, wherein the cognitive disorder is Alzheimer's disease, and the second composition comprises at least one selected from the group consisting of an acetylcholinesterase inhibitor, a NMDA antagonist, vitamin C, and vitamin E.

## Claims

1. A compound of formula (I) 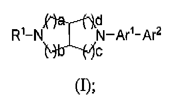 or a pharmaceutically acceptable salt or prodrug thereof, wherein
R¹ is selected from group consisting of hydrogen, alkyl, cyclic alkyl, haloalkyl, aryl, and heteroaryl;
a and c are each independently selected from 0, 1, 2; b and d are each independently selected from 1, 2, or 3; provided that when both b and d are 1, a and c can not be 1 simultaneously;
Ar¹ is selected from 5- or 6-membered aromatic group of formula: 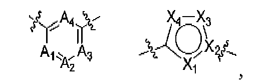 wherein
A₁, A₂, A₃ and A₄ are each -N- or -CR^{a};
X₁, X₃, X₄ are independently selected from group consisting of-CR^{a}, -NR^{a}, -O-, and -S-;
X₂ is -C- or -N-, provided that when X₂ is -C, at least one of X₁, X₃, X₄ is other than -C-;
R^{a} is selected from group consisting of hydrogen, alkyl, cyclic alkyl, haloalkyl, aryl, heteroaryl, halogen, -CO₂R¹, -COR¹, -CONR¹, -OR¹, and -NR¹;
Ar² is a fused bicyclic aromatic group of formula 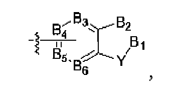 wherein
B₁, B₂, B₃, B₄, B₅, B₆ are each independently -N- or -CR^{a}-;
Y is selected from group consisting of -NR^{d}-, -O- and -S-;
R^{a} is selected from group consisting of hydrogen, alkyl, cyclic alkyl, haloalkyl, aryl, heteroaryl, halogen, -CO₂R¹, -COR¹, -CONR¹, -OR¹, and -NR¹; and
R^{d} is selected from group consisting of hydrogen, alkyl, and cyclic alkyl.

2. The compound of claim 1, wherein the fused diazabicycloalkane moiety is selected from the group consisting of

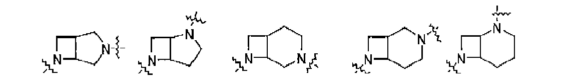

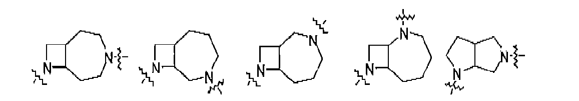

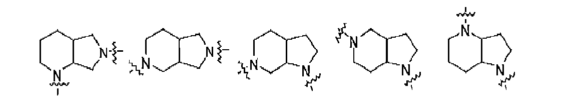

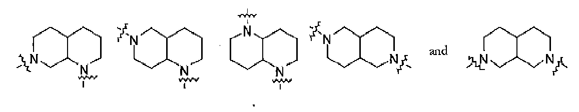

3. The compound of claim 1, wherein Ar¹ is selected from the group consisting of midazolyl, isoxazolyl, isothiazolyl, furyl, oxazolyl, 1,2,4-oxadiazolyl, 1,3,4-oxadiazolyl, phenyl, pyrazolyl, pyridinyl, pyrimidinyl, pyridazinyl, pyrazinyl, thiophenyl, thiazolyl, 1,2,4-thiadiazolyl, and 1,3,4-thiadiazolyl.

4. The compound of claim 3, wherein Ar¹ is selected from the group consisting of pyridazinyl, pyridinyl, thiazolyl, 1,3,4-thiadiazolyl, and 1,3,4-oxadiazolyl, wherein Ar¹ is substituted with 0, 1, or 2 substitutents selected from alkoxy, alkyl, cyano, haloalkyl, hydroxy, halogen and NR¹.

5. The compound of claim 1, wherein Ar² is selected from the group consisting of benzofuranyl, benzo[*d*]imidazolyl, benzo[*d*]isoxazolyl, benzo[*d*]isothiazolyl, benzo[*d*]oxazolyl, benzo[*d*]thiazolyl, benzo[*b*]thiophenyl, furo[3,2-*b*]pyridinyl, furo[3,2-*c*]pyridinyl, imidazo[4,5-*b*]pyridinyl, imidazo[4,5-*c*]pyridine, indolyl, indazolyl, isoxazolo[4,5-*b*]pyridinyl, isoxazolo[4,5-*c*]pyridinyl, isoxazolo[5,4-*b*]pyridinyl, isoxazolo[5,4-*c*]pyridinyl, isothiazolo[4,5-*c*]pyridinyl, isothiazolo[4,5-*c*]pyridinyl, isothiazolo[5,4-*b*]pyridinyl, isothiazolo[5,4-*c*]pyridinyl, oxazolo[4,5-*b*]pyridinyl, oxazolo[4,5-*c*]pyridinyl, oxazolo[5,4-*b*]pyridinyl, oxazolo[5,4-*c*]pyridinyl, pyrazolo[3,4-*b*]pyridinyl, pyrazolo[3,4-*c*]pyridinyl, pyrazolo[4,3-*b*]pyridinyl, pyrazolo[4,3-*c*]pyridinyl, pyrrolo[2,3-*b*]pyridinyl, pyrrolo[2,3-*c*]pyridinyl, pyrrolo[3,2-*b*]pyridinyl, pyrrolo[3,2-*c*]pyridinyl, thiazolo[4,5-*b*]pyridinyl, thiazolo[4,5-*c*]pyridinyl, thiazolo[5,4-*b*]pyridinyl, thiazolo[5,4-*c*]pyridinyl, thieno[2,3-*b*]pyridinyl, thieno[2,3-*c*]pyridinyl, thieno[3,2-*b*]pyridinyl, and thieno[3,2-*c*]pyridinyl.

6. The compound of claim 2, wherein the fused diazabicycloalkane moiety is

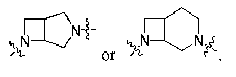

7. The compound of claim 3, wherein Ar¹ is selected from the group consisting of 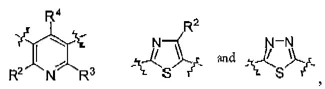 wherein
R², R³ and R⁴ are independently selected from the group consisting of alkoxy, alkyl, cyano, haloalkyl, hydroxy, halogen and NR¹.

8. The compound of claim 4, wherein Ar² is selected from the group consisting of 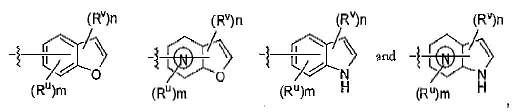 wherein
R^{u} and R^{v} are each independently selected from the group consisting of alkoxy, alkyl, cyano, haloalkyl, hydroxy, halogen and NR¹; and
m and n are are each independently selected from the group consisting of 0, 1 and 2.

9. The compound of claim 1, wherein the compound is one selected from the group consisting of
5-{5-[(*1S,5S*)-3,6-diazabicyclo[3.2.0]heptan-3-yl]pyridin-3-yl}-1H-indole;
5-{5-[(*1S,5S*)-6-methyl-3,6-diazabicyclo[3.2.0]heptan-3-yl]pyridin-3-yl}-*1H-*indole;
4-{5-[(*1S,5S*)-3,6-diazabicyclo[3.2.0]heptan-3-yl]pyridin-3-yl}-*1H*-indole;
4-{5-[(*1S,5S*)-6-methyl-3,6-diazabicyclo[3.2.0]heptan-3-yl]pyridin-3-yl}-*1H-*indole;
6-{5-[(*1S,5S*)-3,6-diazabicyclo[3.2.0]heptan-3-yl]pyridin-3-yl}-*1H*-indole;
6-{5-[(*1S,5S*)-6-methyl-3,6-diazabicyclo[3.2.0]heptan-3-yl]pyridin-3-yl}-*1H-*indole;
5-{5-[(*1S,5S*)-6-methyl-3,6-diazabicyclo[3.2.0]heptan-3-yl]pyridin-3-yl}-2-(trifluoromethyl)-1*H*-indole;
(*1S,5S*)-3-(5-(benzofuran-5-yl)pyridin-3-yl)-6-methyl-3,6-diazabicyclo[3.2.0]heptane;
5-{5-[(*1S,5S*)-6-methyl-3,6-diazabicyclo[3.2.0]heptan-3-yl]pyridin-3-yl}-1*H-*indazole;
(1*S*,*5S*)-3-[5-(benzo[b]thiophen-5-yl)pyridin-3-yl]-3,6-diazabicyclo[3.2.0]heptane;
(*1S,5S*)-3-[5-(benzo[b]thiophen-5-yl)pyridin-3-yl]-3,6-diazabicyclo[3,2.0]heptane;
7-{5-[(1S,5S)-6-methyl-3,6-diazabicyolo[3.2.0]heptan-3-yl]pyridin-3-yl}-1H-indole;
5-{5-[(*1S,5S*)-6-methyl-3,6-diazabicyclo[3.2.0]heptan-3-yl]pyridin-3-yl}-1H-benzo[d]imidazole;
3-methyl-5-{5-[(*1S,5S*)-6-methyl-3,6-diazabicyclo[3.2.0]heptan-3-yl]pyridin-3-yl}-1*H*-indole;
3-{5-[(*1S,5S*)-3,6-diazabicyclo[3.2.0]heptan-3-yl]pyridin-3-yl}-9*H*-carbazole;
5-{5-[(*1S,5S*)-3,6-diazabicyclo[3.2.0]heptan-3-yl]pyridin-3-yl}-3-methyul-1*H-*indole ;
3-{5-[(*1S,5S*)-6-methyl-3,6-diazabicyclo[3.2.0]heptan-3-yl)pyridin-3-yl)-9H-carbazole;
7-{5-[(*1S,5S*)-6-methyl-3,6-diazabicyclo[3.2.0]heptan-3-yl]pyridin-3-yl}-*1H-*pyrrolo[2,3-c]pyridine;
5-{5-[(*1S,5S*)-6-methyl-3,6-diazabicyclo[3.2.0]heptan-3-yl]pyridin-3-yl}-1H-pyrrolo[2,3-b]pyridine;
3-{5-[(*1S,5S*)-6-methyl-3,6-diazabicyclo[3.2.0]heptan-3-yl]pyridin-3-yl}-1-(phenylsulfonyl)-1*H*-indole;
3-(5-((*1S,5S*)-6-methyl-3,6-diazabicyclo[3.2.0]heptan-3-yl)pyridin-3-yl)-1*H-*indole;
4-{6-[(*1S,5S*)-3,6-diazabicyclo[3.2.0]heptan-3-yl]pyrazin-2-yl}-*1H*-indole; 4-{6-[(*1S,5S*)-6-methyl-3,6-diazabicyclo[3.2.0]heptan-3-yl]pyrazin-2-yl}-*1H-*indole;
5-{6-[(*1S,5S*)-6-methyl-3,6-diazabicyclo[3.2.0]heptan-3-yl]pyrazin-2-yl}-*1H-*indole;
6-{6-[(*1S,5S*)-6-methyl-3,6-diazabicyclo[3.2.0]heptan-3-yl]pyrazin-2-yl}-*1H-*indole;
5-(6-((*1S,5S*)-6-methyl-3,6-diazabicyclo[3.2.0]heptan-3-yl)pyrazin-2-yl)-2-(trifluoromethyl)-1*H*-indole
5-{5-[(*1R,5R*)-6-methyl-3,6-diazabicyclo[3.2.0]heptan-3-yl]pyridin-3-yl}-1H-indole ;
4-{5-[(*1R,5R*)-6-methyl-3,6-diazabicyclo[3.2.0]heptan-3-yl]pyridin-3-yl}-1H-indole;
6-{5-[(*1R,5R*)-6-methyl-3,6-diazabicyclo[3.2.0]heptan-3-yl]pyridin-3-yl}-1H-indole;
5-{5-[(*1R,5S*)-3,6-diazabicyclo[3.2.0]heptan-6-yl]pyridin-3-yl}-1*H*-indole;
5-{5-[(*1R,5S*)-3-methyl-3,6-diazabicyclo[3.2.0]heptan-6-yl]pyridin-3-yl}-1*H-*indole;
6-{5-[(*1R,5S*)-3,6-diazabicyclo[3.2.0]heptan-6-yl]pyridin-3-yl}-1*H*-indole;
6-{5-[(*1R,5S*)-3-methyl-3,6-diazabicyclo[3.2.0]heptan-6-yl]pyridin-3-yl}-1*H-*indole;
4-(5-((*1R,5S*)-3-methyl-3,6-diazabicyclo[3.2.0]heptan-6-yl)pyridin-3-yl)-1*H-*indole;
6-{5-[(*3aS*,*6aS*)-5-methylhexahydropyrrolo[3,4-b]pyrrol-1(2H)-yl]pyridin-3-yl}-1H-indole; and
5-{5-[(*3aS,6aS*)-5-methylhexahydropyrrolo[3,4-b]pyrrol-1(2H)-yl]pyridin-3-yl}-1*H*-indole.

10. A pharmaceutical composition comprising a therapeutically effective amount of a compound of claim 1 and a pharmaceutically acceptable carrier.

11. The compound of claim 1 for use in treating an α7 and α4β2 nicotinic acetylcholine receptor-mediated condition or disorder in a subject in need thereof by administering said compound to the subject, wherein the α7 and α4β2 nicotinic acetylcholine receptor-mediated condition or disorder is selected from the group consisting of cognitive disorder, neurodegeneration, schizophrenia, attention deficit disorder, attention deficit hyperactivity disorder, Alzheimer's disease, mild cognitive impairment, senile dementia, AIDS dementia, Pick's Disease, dementia associated with Lewy bodies, dementia associated with Down's syndrome, amyotrophic lateral sclerosis, Huntington's disease, diminished CNS function associated with traumatic brain injury, acute pain, post-surgical pain, chronic pain, inflammation, inflammatory pain, neuropathic pain, infertility, need for new blood vessel growth associated with wound healing, need for new blood vessel growth associated with vascularization of skin grafts, and lack of circulation, rheumatoid arthritis, Crohn's disease, ulcerative colitis, inflammatory bowel disease, organ transplant rejection, acute immune disease associated with organ transplantation, chronic immune disease associated with organ transplantation, septic shock, toxic shock syndrome, sepsis syndrome, depression, rheumatoid spondylitis, and substance abuse.

12. The compound for use according to claim 11, wherein the compound is an agonist of at least one α7 nicotinic acetylcholine receptor, and is further administered with an atypical antipsychotic.

13. The compound for use of claim 12, wherein the atypical antipsychotic is at least one selected from the group consisting of clozapine, risperidone, olanzapine, quietapine, ziprasidone, zotepine, and iloperidone.

14. The compound for use of claim 11 wherein said compound is further administered with a second composition to treat a cognitive disorder which is attention deficit disorder, and the second composition comprises at least one compound selected from the group consisting of dextroamphetamine, levoamphetamine, dextrothreomethylphenidate, levothreomethylphenidate, amantadine, amineptine, benzphetamine, bupropion, clonidine, modafinil, pemoline, selegiline, and milnacipran.

15. The compound for use of claim 11 wherein said compound is further administered with a second composition to treat a cognitive disorder which is Alzheimer's disease, and the second composition comprises at least one selected from the group consisting of an acetylcholinesterase inhibitor, a NMDA antagonist, vitamin C, and vitamin E.
